# EUROPEAN PATENT APPLICATION

(11) **EP 1 876 241 A2**
(43) Date of publication of application: **09.01.2008**
(21) Application number: 07011366.7
(22) Date of filing: 10.01.1997
(51) Int. Cl.: C12N 15/12, C07K 14/47, C07K 14/82, C07K 14/15, C12Q 1/68, G01N 33/53, A61K 38/17, A61K 39/00

(54) **Compositions and methods for the treatment and diagnosis of breast cancer**

(30) Priority: 10.01.1996 US 585392; 20.08.1996 US 700014
(62) Divisional of application: 97902911.3
(71) Applicant: CORIXA CORPORATION, Hamilton, Montana 59840-3131 (US)
(72) Inventor: Frudakis, Tony N., Seattle, WA 98232-0232 (US); Smith, John M., Everett, WA 98208 (US); Reed, Steven G., Bellevue, WA 98005 (US)
(74) Representative: Thornton, Neil

(57) **Abstract**

Compositions and methods for the detection and therapy of breast cancer are disclosed. The compounds provided include nucleotide sequences that are preferentially expressed in breast tumor tissue, as well as polypeptides encoded by such nucleotide sequences. Vaccines and pharmaceutical compositions comprising such compounds are also provided and may be used, for example, for the prevention and treatment of breast cancer. The polypeptides may also be used for the production of antibodies, which are useful for diagnosing and monitoring the progression of breast cancer in a patient.

## Description

### Technical Field

The present invention relates generally to the detection and therapy of breast cancer. The invention is more specifically related to nucleotide sequences that are preferentially expressed in breast tumor tissue and to polypeptides encoded by such nucleotide sequences. The nucleotide sequences and polypeptides may be used in vaccines and pharmaceutical compositions for the prevention and treatment of breast cancer. The polypeptides may also be used for the production of compounds, such as antibodies, useful for diagnosing and monitoring the progression of breast cancer in a patient.

### Background of the Invention

Breast cancer is a significant health problem for women in the United States and throughout the world. Although advances have been made in detection and treatment of the disease, breast cancer remains the second leading cause of cancer-related deaths in women, affecting more than 180,000 women in the United States each year. For women in North America, the life-time odds of getting breast cancer are now one in eight.

No vaccine or other universally successful method for the prevention or treatment of breast cancer is currently available. Management of the disease currently relies on a combination of early diagnosis (through routine breast screening procedures) and aggressive treatment, which may include one or more of a variety of treatments such as surgery, radiotherapy, chemotherapy and hormone therapy. The course of treatment for a particular breast cancer is often selected based on a variety of prognostic parameters, including an analysis of specific tumor markers. *See, e.g.,* Porter-Jordan and Lippman, Breast Cancer 8:73-100 (1994). However, the use of established markers often leads to a result that is difficult to interpret, and the high mortality observed in breast cancer patients indicates that improvements are needed in the treatment, diagnosis and prevention of the disease.

Accordingly, there is a need in the art for improved methods for therapy and diagnosis of breast cancer. The present invention fulfills these needs and further provides other related advantages.

### Summary of the Invention

Briefly stated, the subject invention provides compositions and methods for the diagnosis and therapy of breast cancer. In one aspect, isolated DNA molecules are provided, comprising (a) a nucleotide sequence preferentially expressed in breast cancer tissue, relative to normal tissue; (b) a variant of such a sequence that contains one or more nucleotide substitutions, deletions, insertions and/or modifications at no more than 20% (preferably no more than 5%) of the nucleotide positions, such that the antigenic and/or immunogenic properties of the polypeptide encoded by the nucleotide sequence are retained; or (c) a nucleotide sequence encoding an epitope of a polypeptide encoded by at least one of the above sequences. In one embodiment, the isolated DNA molecule comprises a human endogenous retroviral sequence recited in SEQ ID NO:1. In other embodiments, the isolated DNA molecule comprises a nucleotide sequence recited in any one of SEQ ID NO:3 - SEQ ID NO:77 or SEQ ID NOS: 142, 143, 146-152, 154-166, 168-176, 178-192, 194-198, 200-204, 206, 207, 209-214, 216, 218, 219, 221-227.

In related embodiments, the isolated DNA molecule encodes an epitope of a polypeptide, wherein the polypeptide is encoded by a nucleotide sequence that: (a) hybridizes to a sequence recited in any one of SEQ ID NO: 1 or SEQ ID NO:3 - SEQ ID NO:77 or SEQ ID NOS: 142, 143, 146-152, 154-166, 168-176, 178-192, 194-198, 200-204, 206, 207, 209-214, 216, 218, 219, 221-227 under stringent conditions; and (b) is at least 80% identical to a sequence recited in any one of SEQ ID NO: 1 or SEQ ID NO:3 - SEQ ID NO:77 or SEQ ID NOS: 142, 143, 146-152, 154-166, 168-176, 178-192, 194-198, 200-204, 206, 207, 209-214, 216, 218, 219, 221-227; and wherein RNA corresponding to said nucleotide sequence is expressed at a greater level in human breast tumor tissue than in normal breast tissue.

In another embodiment, the present invention provides an isolated DNA molecule encoding an epitope of a polypeptide, the polypeptide being encoded by: (a) a nucleotide sequence transcribed from the sequence of SEQ ID NO: 141; or (b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained. Isolated DNA and RNA molecules comprising a nucleotide sequence complementary to a DNA molecule as described above are also provided.

In related aspects, the present invention provides recombinant expression vectors comprising a DNA molecule as described above and host cells transformed or transfected with such expression vectors.

In further aspects, polypeptides, comprising an amino acid sequence encoded by a DNA molecule as described above, and monoclonal antibodies that bind to such polypeptides are provided.

In yet another aspect, methods are provided for determining the presence of breast cancer in a patient. In one embodiment, the method comprises detecting, within a biological sample, a polypeptide as described above. In another embodiment, the method comprises detecting, within a biological sample, an RNA molecule encoding a polypeptide as described above. In yet another embodiment, the method comprises (a) intradermally injecting a patient with a polypeptide as described above; and (b) detecting an immune response on the patient's skin and therefrom detecting the presence of breast cancer in the patient. In further embodiments, the present invention provides methods for determining the presence of breast cancer in a patient as described above wherein the polypeptide is encoded by a nucleotide sequence selected from the group consisting of SEQ ID NO.: 78-86, SEQ ID NOS: 144, 145, 153, 167, 177, 193, 199, 205, 208, 215, 217, 220 and sequences that hybridize thereto under stringent conditions.

In a related aspect, diagnostic kits useful in the determination of breast cancer are provided. The diagnostic kits generally comprise either one or more monoclonal antibodies as described above, or one or more monoclonal antibodies that bind to a polypeptide encoded by a nucleotide sequence selected from the group consisting of sequences provided in SEQ ID NOS: 78-86 and SEQ ID NOS: 144, 145, 153, 167, 177, 193, 199, 205, 208, 215, 217, 220, and a detection reagent.

Within a related aspect, the diagnostic kit comprises a first polymerase chain reaction primer and a second polymerase chain reaction primer, the first and second primers each comprising at least about 10 contiguous nucleotides of an RNA molecule as described above, or an RNA molecule encoding a polypeptide encoded by a nucleotide sequence selected from the group consisting of SEQ ID NOS: 78-86 and SEQ ID NOS: 144, 145, 153, 167, 177, 193, 199, 205, 208, 215, 217, 220.

Within another related aspect, the diagnostic kit comprises at least one oligonucleotide probe, the probe comprising at least about 15 contiguous nucleotides of a DNA molecule as described above, or a DNA molecule selected from the group consisting of SEQ ID NOS: 78-86 and SEQ ID NOS: 144, 145, 153, 167, 177, 193, 199,205,208,215,217,220.

In another related aspect, the present invention provides methods for monitoring the progression of breast cancer in a patient. In one embodiment, the method comprises: (a) detecting an amount, in a biological sample, of a polypeptide as described above at a first point in time; (b) repeating step (a) at a subsequent point in time; and (c) comparing the amounts of polypeptide detected in steps (a) and (b), and therefrom monitoring the progression of breast cancer in the patient. In another embodiment, the method comprises (a) detecting an amount, within a biological sample, of an RNA molecule encoding a polypeptide as described above at a first point in time; (b) repeating step (a) at a subsequent point in time; and (c) comparing the amounts of RNA molecules detected in steps (a) and (b), and therefrom monitoring the progression of breast cancer in the patient. In yet other embodiments, the present invention provides methods for monitoring the progression of breast cancer in a patient as described above wherein the polypeptide is encoded by a nucleotide sequence selected form the group consisting of SEQ ID NO.: 78-86, SEQ ID NOS:144, 145, 153, 167, 177, 193, 199, 205, 208, 215, 217, 220 and sequences that hybridize thereto under stringent conditions.

In still other aspects, pharmaceutical compositions, which comprise a polypeptide as described above in combination with a physiologically acceptable carrier, and vaccines, which comprise a polypeptide as described above in combination with an immune response enhancer or adjuvant are provided. In yet other aspects, the present invention provides pharmaceutical compositions and vaccines comprising a polypeptide encoded by a nucleotide sequence selected form the group consisting of SEQ ID NO.: 78-86, SEQ ID NOS:144, 145, 153, 167, 177, 193, 199, 205, 208, 215, 217, 220 and sequences that hybridize thereto under stringent conditions.

In related aspects, the present invention provides methods for inhibiting the development of breast cancer in a patient, comprising administering to a patient a pharmaceutical composition or vaccine as described above.

These and other aspects of the present invention will become apparent upon reference to the following detailed description and attached drawings. All references disclosed herein are hereby incorporated by reference in their entirety as if each was incorporated individually.

### Brief Description of the Drawings

Figure 1 shows the differential display PCR products, separated by gel electrophoresis, obtained from cDNA prepared from normal breast tissue (lanes 1 and 2) and from cDNA prepared from breast tumor tissue from the same patient (lanes 3 and 4). The arrow indicates the band corresponding to B 18Ag1.
Figure 2 is a northern blot comparing the level of B18Ag1 mRNA in breast tumor tissue (lane 1) with the level in normal breast tissue.
Figure 3 shows the level of B18Ag1 mRNA in breast tumor tissue compared to that in various normal and non-breast tumor tissues as determined by RNase protection assays.
Figure 4 is a genomic clone map showing the location of additional retroviral sequences obtained from ends of XbaI restriction digests (provided in SEQ ID NO:3 - SEQ ID NO:10) relative to B18Ag1.
Figures 5A and 5B show the sequencing strategy, genomic organization and predicted open reading frame for the retroviral element containing B18Ag1.
Figure 6 shows the nucleotide sequence of the representative breast tumor-specific cDNA B18Ag1.
Figure 7 shows the nucleotide sequence of the representative breast tumor-specific cDNA B17Ag1.
Figure 8 shows the nucleotide sequence of the representative breast tumor-specific cDNA B17Ag2.
Figure 9 shows the nucleotide sequence of the representative breast tumor-specific cDNA B13Ag2a.
Figure 10 shows the nucleotide sequence of the representative breast tumor-specific cDNA B13Aglb.
Figure 11 shows the nucleotide sequence of the representative breast tumor-specific cDNA B13Agla.
Figure 12 shows the nucleotide sequence of the representative breast tumor-specific cDNA B11Ag1.
Figure 13 shows the nucleotide sequence of the representative breast tumor-specific cDNA B3CA3c.
Figure 14 shows the nucleotide sequence of the representative breast tumor-specific cDNA B9CG1.
Figure 15 shows the nucleotide sequence of the representative breast tumor-specific cDNA B9CG3.
Figure 16 shows the nucleotide sequence of the representative breast tumor-specific cDNA B2CA2.
Figure 17 shows the nucleotide sequence of the representative breast tumor-specific cDNA B3CA1.
Figure 18 shows the nucleotide sequence of the representative breast tumor-specific cDNA B3CA2.
Figure 19 shows the nucleotide sequence of the representative breast tumor-specific cDNA B3CA3.
Figure 20 shows the nucleotide sequence of the representative breast tumor-specific cDNA B4CA1.
Figure 21A depicts RT-PCR analysis of breast tumor genes in breast tumor tissues (lanes 1-8) and normal breast tissues (lanes 9-13) and H₂O (lane 14).
Figure 21B depicts RT-PCR analysis of breast tumor genes in prostate tumors (lane 1,2), colon tumors (lane 3), lung tumor (lane 4), normal prostate (lane 5), normal colon (lane 6), normal kidney (lane 7), normal liver (lane 8), normal lung (lane 9), normal ovary (lanes 10, 18), normal pancreases (lanes 11, 12), normal skeletal muscle (lane 13), normal skin (lane 14), normal stomach (lane 15), normal testes (lane 16), normal small intestine (lane 17), HBL-100 (lane 19), MCF-12A (lane 20), breast tumors (lanes 21-23), H₂O (lane 24), and colon tumor (lane 25).

### Detailed Description of the Invention

As noted above, the present invention is generally directed to compositions and methods for the diagnosis, monitoring and therapy of breast cancer. The compositions described herein include polypeptides, nucleic acid sequences and antibodies. Polypeptides of the present invention generally comprise at least a portion of a protein that is expressed at a greater level in human breast tumor tissue than in normal breast tissue (i.e., the level of RNA encoding the polypeptide is at least 2-fold higher in tumor tissue). Such polypeptides are referred to herein as breast tumor-specific polypeptides, and cDNA molecules encoding such polypeptides are referred to as breast tumor-specific cDNAs. Nucleic acid sequences of the subject invention generally comprise a DNA or RNA sequence that encodes all or a portion of a polypeptide as described above, or that is complementary to such a sequence. Antibodies are generally immune system proteins, or fragments thereof, that are capable of binding to a portion of a polypeptide as described above. Antibodies can be produced by cell culture techniques, including the generation of monoclonal antibodies as described herein, or via transfection of antibody genes into suitable bacterial or mammalian cell hosts, in order to allow for the production of recombinant antibodies.

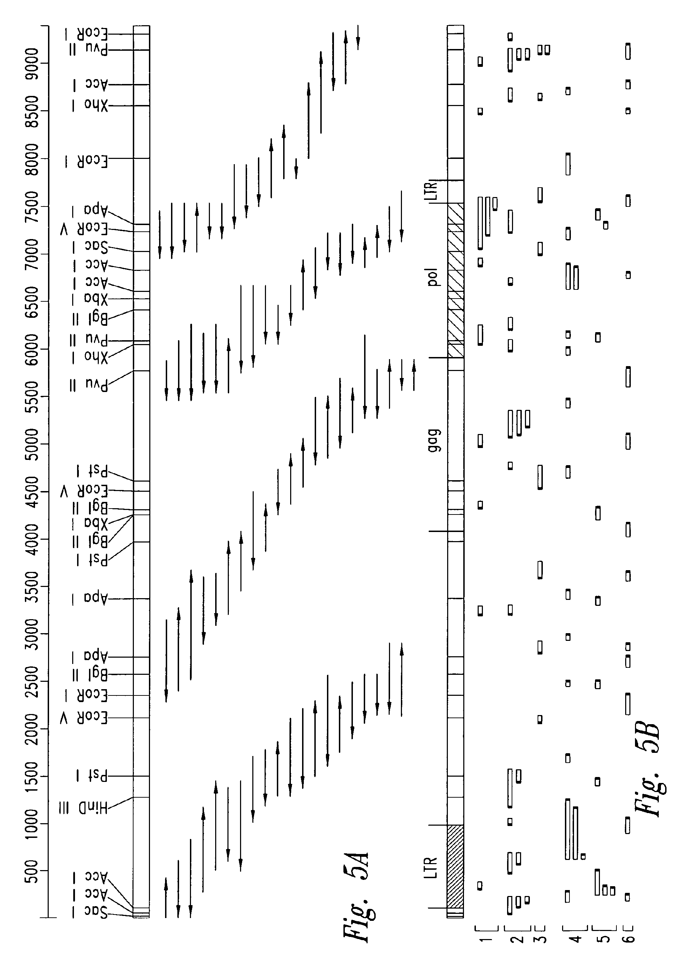

Polypeptides within the scope of this invention include, but are not limited to, polypeptides (and epitopes thereof) encoded by a human endogenous retroviral sequence, such as the sequence designated B18Ag1 (Figure 5 and SEQ ID NO:1). Also within the scope of the present invention are polypeptides encoded by other sequences within the retroviral genome containing B18Ag1 (SEQ ID NO: 141). Such sequences include, but are not limited to, the sequences recited in SEQ ID NO:3 - SEQ ID NO:10. B18Ag1 has homology to the *gag* p30 gene of the endogenous human retroviral element S71, as described in Werner et al., Virology 174:225-238 (1990) and also shows homology to about thirty other retroviral gag genes. As discussed in more detail below, the present invention also includes a number of additional breast tumor-specific polypeptides, such as those encoded by the nucleotide sequences recited in SEQ ID NO:11 - SEQ ID NO: 77 and SEQ ID NOS:142, 143, 146-152, 154-166, 168-176, 178-192, 194-198, 200-204, 206, 207, 209-214, 216, 218, 219, 221-227. As used herein, the term "polypeptide" encompasses amino acid chains of any length, including full length proteins containing the sequences recited herein. A polypeptide comprising an epitope of a protein containing a sequence as described herein may consist entirely of the epitope, or may contain additional sequences. The additional sequences may be derived from the native protein or may be heterologous, and such sequences may (but need not) possess immunogenic or antigenic properties.

An "epitope," as used herein is a portion of a polypeptide that is recognized (i.e., specifically bound) by a B-cell and/or T-cell surface antigen receptor. Epitopes may generally be identified using well known techniques, such as those summarized in Paul, Fundamental Immunology, 3rd ed., 243-247 (Raven Press, 1993) and references cited therein. Such techniques include screening polypeptides derived from the native polypeptide for the ability to react with antigen-specific antisera and/or T-cell lines or clones. An epitope of a polypeptide is a portion that reacts with such antisera and/or T-cells at a level that is similar to the reactivity of the full length polypeptide (*e.g.*, in an ELISA and/or T-cell reactivity assay). Such screens may generally be performed using methods well known to those of ordinary skill in the art, such as those described in Harlow and Lane, Antibodies: A Laboratory Manual, Cold Spring Harbor Laboratory, 1988. B-cell and T-cell epitopes may also be predicted via computer analysis. Polypeptides comprising an epitope of a polypeptide that is preferentially expressed in a tumor tissue (with or without additional amino acid sequence) are within the scope of the present invention.

The compositions and methods of the present invention also encompass variants of the above polypeptides and nucleic acid sequences encoding such polypeptides. A polypeptide "variant," as used herein, is a polypeptide that differs from the native polypeptide in substitutions and/or modifications, such that the antigenic and/or immunogenic properties of the polypeptide are retained. Such variants may generally be identified by modifying one of the above polypeptide sequences and evaluating the reactivity of the modified polypeptide with antisera and/or T-cells as described above. Nucleic acid variants may contain one or more substitutions, deletions, insertions and/or modifications such that the antigenic and/or immunogenic properties of the encoded polypeptide are retained. One preferred variant of the polypeptides described herein is a variant that contains nucleotide substitutions, deletions, insertions and/or modifications at no more than 20% of the nucleotide positions.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also (or alternatively) be modified by, for example, the deletion or addition of amino acids that have minimal influence on the immunogenic or antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.,* poly-His), or to enhance binding of the polypeptide to a solid support. For example, a polypeptide may be conjugated to an immunoglobulin Fc region.

In general, nucleotide sequences encoding all or a portion of the polypeptides described herein may be prepared using any of several techniques. For example, cDNA molecules encoding such polypeptides may be cloned on the basis of the breast tumor-specific expression of the corresponding mRNAs, using differential display PCR. This technique compares the amplified products from RNA template prepared from normal and breast tumor tissue. cDNA may be prepared by reverse transcription of RNA using a (dT)₁₂AG primer. Following amplification of the cDNA using a random primer, a band corresponding to an amplified product specific to the tumor RNA may be cut out from a silver stained gel and subcloned into a suitable vector (*e.g.*, the T-vector, Novagen, Madison, WI). Nucleotide sequences encoding all or a portion of the breast tumor-specific polypeptides disclosed herein may be amplified from cDNA prepared as described above using the random primers shown in SEQ ID NO.:87-125.

Alternatively, a gene encoding a polypeptide as described herein (or a portion thereof) may be amplified from human genomic DNA, or from breast tumor cDNA, via polymerase chain reaction. For this approach, B18Ag1 sequence-specific primers may be designed based on the sequence provided in SEQ ID NO: 1, and may be purchased or synthesized. One suitable primer pair for amplification from breast tumor cDNA is (5'ATG GCT ATT TTC GGG GGC TGA CA) (SEQ ID NO.:126) and (5'CCG GTA TCT CCT CGT GGG TAT T) (SEQ ID NO.:127). An amplified portion of B18Ag1 may then be used to isolate the full length gene from a human genomic DNA library or from a breast tumor cDNA library, using well known techniques, such as those described in Sambrook et al., Molecular Cloning: A Laboratory Manual, Cold Spring Harbor Laboratories, Cold Spring Harbor, NY (1989). Other sequences within the retroviral genome of which B18Ag1 is a part may be similarly prepared by screening human genomic libraries using B18Ag1-specific sequences as probes. Nucleotides translated into protein from the retroviral genome shown in SEQ ID NO: 141 may then be determined by cloning the corresponding cDNAs, predicting the open reading frames and cloning the appropriate cDNAs into a vector containing a viral promoter, such as T7. The resulting constructs can be employed in a translation reaction, using techniques known to those of skill in the art, to identify nucleotide sequences which result in expressed protein. Similarly, primers specific for the remaining breast tumor-specific polypeptides described herein may be designed based on the nucleotide sequences provided in SEQ ID NO:11 - SEQ ID NO:86 and SEQ ID NO:142-SEQ ID NO:226.

Recombinant polypeptides encoded by the DNA sequences described above may be readily prepared from the DNA sequences. For example, supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

In general, any of a variety of expression vectors known to those of ordinary skill in the art may be employed to express recombinant polypeptides of this invention. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO.

Such techniques may also be used to prepare polypeptides comprising epitopes or variants of the native polypeptides. For example, variants of a native polypeptide may generally be prepared using standard mutagenesis techniques, such as oligonucleotide-directed site-specific mutagenesis, and sections of the DNA sequence may be removed to permit preparation of truncated polypeptides. Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146 (1963). Equipment for automated synthesis of polypeptides is commercially available from suppliers such as Applied BioSystems, Inc., Foster City, CA, and may be operated according to the manufacturer's instructions.

In specific embodiments, polypeptides of the present invention encompass amino acid sequences encoded by a DNA molecule having a sequence recited in any one of SEQ ID NO: or SEQ ID NO:3 - SEQ ID NO:77 or SEQ ID NOS:142, 143, 146-152, 154-166, 168-176, 178-192, 194-198, 200-204, 206, 207, 209-214, 216, 218, 219, 221-227, variants of such polypeptides that are encoded by DNA molecules containing one or more nucleotide substitutions, deletions, insertions and/or modifications at no more than 20% of the nucleotide positions, and epitopes of the above polypeptides. Polypeptides within the scope of the present invention also include polypeptides (and epitopes thereof) encoded by DNA sequences that hybridize to a DNA molecule having a sequence recited in any one of SEQ ID NO:1 or SEQ ID NO:3 - SEQ ID NO:77 or SEQ ID NOS:142, 143, 146-152, 154-166, 168-176, 178-192, 194-198, 200-204, 206, 207, 209-214, 216, 218, 219, 221-227 under stringent conditions, wherein the DNA sequences are at least 80% identical in overall sequence to a recited sequence and wherein RNA corresponding to the nucleotide sequence is expressed at a greater level in human breast tumor tissue than in normal breast tissue. As used herein, "stringent conditions" refers to prewashing in a solution of 6X SSC, 0.2% SDS; hybridizing at 65°C, 6X SSC, 0.2% SDS overnight; followed by two washes of 30 minutes each in 1X SSC, 0.1% SDS at 65°C and two washes of 30 minutes each in 1 X SSC, 0.1% SDS at 65°C. DNA molecules according to the present invention include molecules that encode any of the above polypeptides.

In another aspect of the present invention, antibodies are provided. Such antibodies may be prepared by any of a variety of techniques known to those of ordinary skill in the art. *See, e.g.,* Harlow and Lane, Antibodies: A Laboratory Manual, Cold Spring Harbor Laboratory, 1988. In one such technique, an immunogen comprising the polypeptide is initially injected into any of a wide variety of mammals (*e.g.*, mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519 (1976), and improvements thereto. Briefly, these methods involve the preparation of immortal cell lines capable of producing antibodies having the desired specificity (i.e., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Antibodies may be used, for example, in methods for detecting breast cancer in a patient. Such methods involve using an antibody to detect the presence or absence of a breast tumor-specific polypeptide as described herein in a suitable biological sample. As used herein, suitable biological samples include tumor or normal tissue biopsy, mastectomy, blood, lymph node, serum or urine samples, or other tissue, homogenate, or extract thereof obtained from a patient.

There are a variety of assay formats known to those of ordinary skill in the art for using an antibody to detect polypeptide markers in a sample. *See, e.g.,* Harlow and Lane, Antibodies: A Laboratory Manual, Cold Spring Harbor Laboratory, 1988. For example, the assay may be performed in a Western blot format, wherein a protein preparation from the biological sample is submitted to gel electrophoresis, transferred to a suitable membrane and allowed to react with the antibody. The presence of the antibody on the membrane may then be detected using a suitable detection reagent, as described below.

In another embodiment, the assay involves the use of antibody immobilized on a solid support to bind to the polypeptide and remove it from the remainder of the sample. The bound polypeptide may then be detected using a second antibody or reagent that contains a reporter group. Alternatively, a competitive assay may be utilized, in which a polypeptide is labeled with a reporter group and allowed to bind to the immobilized antibody after incubation of the antibody with the sample. The extent to which components of the sample inhibit the binding of the labeled polypeptide to the antibody is indicative of the reactivity of the sample with the immobilized antibody, and as a result, indicative of the concentration of polypeptide in the sample.

The solid support may be any material known to those of ordinary skill in the art to which the antibody may be attached. For example, the solid support may be a test well in a microtiter plate or a nitrocellulose filter or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The antibody may be immobilized on the solid support using a variety of techniques known to those in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the antibody, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of antibody ranging from about 10 ng to about 1 µg, and preferably about 100-200 ng, is sufficient to immobilize an adequate amount of polypeptide.

Covalent attachment of antibody to a solid support may also generally be achieved by first reacting the support with a bifunctional reagent that will react with both the support and a functional group, such as a hydroxyl or amino group, on the antibody. For example, the antibody may be covalently attached to supports having an appropriate polymer coating using benzoquinone or by condensation of an aldehyde group on the support with an amine and an active hydrogen on the binding partner *(see, e.g.*, Pierce Immunotechnology Catalog and Handbook (1991) at A12-A13).

In certain embodiments, the assay for detection of polypeptide in a sample is a two-antibody sandwich assay. This assay may be performed by first contacting an antibody that has been immobilized on a solid support, commonly the well of a microtiter plate, with the biological sample, such that the polypeptide within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (i.e., incubation time) is that period of time that is sufficient to detect the presence of polypeptide within a sample obtained from an individual with breast cancer. Preferably, the contact time is sufficient to achieve a level of binding that is at least 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

The second antibody is then incubated with the immobilized antibody-polypeptide complex for an amount of time sufficient to detect the bound polypeptide. An appropriate amount of time may generally be determined by assaying the level of binding that occurs over a period of time. Unbound second antibody is then removed and bound second antibody is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of breast cancer, the signal detected from the reporter group that remains bound to the solid support is generally compared to a signal that corresponds to a predetermined cut-off value established from non-tumor tissue. In one preferred embodiment, the cut-off value is the average mean signal obtained when the immobilized antibody is incubated with samples from patients without breast cancer. In general, a sample generating a signal that is three standard deviations above the predetermined cut-off value may be considered positive for breast cancer. In an alternate preferred embodiment, the cut-off value is determined using a Receiver Operator Curve, according to the method of Sackett et al., Clinical Epidemiology: A Basic Science for Clinical Medicine, p. 106-7 (Little Brown and Co., 1985). Briefly, in this embodiment, the cut-off value may be determined from a plot of pairs of true positive rates (i.e., sensitivity) and false positive rates (100%-specificity) that correspond to each possible cut-off value for the diagnostic test result. The cut-off value on the plot that is the closest to the upper left-hand corner (i.e., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for breast cancer.

In a related embodiment, the assay is performed in a flow-through or strip test format, wherein the antibody is immobilized on a membrane, such as nitrocellulose. In the flow-through test, the polypeptide within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of breast cancer. Typically, the concentration of second antibody at that site generates a pattern, such as a line, that can be read visually. The absence of such a pattern indicates a negative result. In general, the amount of antibody immobilized on the membrane is selected to generate a visually discernible pattern when the biological sample contains a level of polypeptide that would be sufficient to generate a positive signal in the two-antibody sandwich assay, in the format discussed above. Preferably, the amount of antibody immobilized on the membrane ranges from about 25 ng to about 1 µg, and more preferably from about 50 ng to about 1 µg. Such tests can typically be performed with a very small amount of biological sample.

The presence or absence of breast cancer in a patient may also be determined by evaluating the level of mRNA encoding a breast tumor-specific polypeptide as described herein within the biological sample (*e.g.*, a biopsy, mastectomy and/or blood sample from a patient) relative to a predetermined cut-off value. Such an evaluation may be achieved using any of a variety of methods known to those of ordinary skill in the art such as, for example, *in situ* hybridization and amplification by polymerase chain reaction. For example, polymerase chain reaction may be used to amplify sequences from cDNA prepared from RNA that is isolated from one of the above biological samples. Sequence-specific primers for use in such amplification may be designed based on the sequences provided in any one of SEQ ID NO: 1 or SEQ ID NO:11 - SEQ ID NO:86 and SEQ ID NO:142-SEQ ID NO:226, and may be purchased or synthesized. In the case of B18Ag1, as noted herein, one suitable primer pair is B18Ag1-2 (5'ATG GCT ATT TTC GGG GGC TGA CA) (SEQ ID NO.:126) and B18Ag1-3 (5'CCG GTA TCT CCT CGT GGG TAT T) (SEQ ID NO.:127). The PCR reaction products may then be separated by gel electrophoresis and visualized according to methods well known to those of ordinary skill in the art. Amplification is typically performed on samples obtained from matched pairs of tissue (tumor and non-tumor tissue from the same individual) or from unmatched pairs of tissue (tumor and non-tumor tissue from different individuals). The amplification reaction is preferably performed on several dilutions of cDNA spanning two orders of magnitude. A two-fold or greater increase in expression in several dilutions of the tumor sample as compared to the same dilution of the non-tumor sample is considered positive.

Conventional RT-PCR protocols using agarose and ethidium bromide staining while important in defining gene specificity do not lend themselves to diagnostic kit development because of the time and effort required in making them quantitative (i.e., construction of saturation and/or titration curves), and their sample throughput. This problem is overcome by the development of procedures such as real time RT-PCR which allows for assays to be performed in single tubes, and in turn can be modified for use in 96 well plate formats. Instrumentation to perform such methodologies are available from ABI/Perkin Elmer. Alternatively, other high throughput assays using labelled probes (e.g., digoxygenin) in combination with labelled (e.g., enzyme fluorescent, radioactive) antibodies to such probes can also be used in the development of 96 well plate assays.

In yet another method for determining the presence or absence of breast cancer in a patient, one or more of the breast tumor-specific polypeptides described may be used in a skin test. As used herein, a "skin test" is any assay performed directly on a patient in which a delayed-type hypersensitivity (DTH) reaction (such as swelling, reddening or dermatitis) is measured following intradermal injection of one or more polypeptides as described above. Such injection may be achieved using any suitable device sufficient to contact the polypeptide or polypeptides with dermal cells of the patient, such as a tuberculin syringe or 1 mL syringe. Preferably, the reaction is measured at least 48 hours after injection, more preferably 48-72 hours.

The DTH reaction is a cell-mediated immune response, which is greater in patients that have been exposed previously to a test antigen (i.e., an immunogenic portion of a polypeptide employed, or a variant thereof). The response may measured visually, using a ruler. In general, a response that is greater than about 0.5 cm in diameter, preferably greater than about 5.0 cm in diameter, is a positive response, indicative of breast cancer.

The breast tumor-specific polypeptides described herein are preferably formulated, for use in a skin test, as pharmaceutical compositions containing at least one polypeptide and a physiologically acceptable carrier, such as water, saline, alcohol, or a buffer. Such compositions typically contain one or more of the above polypeptides in an amount ranging from about 1 µg to 100 µg, preferably from about 10 µg to 50 µg in a volume of 0.1 mL. Preferably, the carrier employed in such pharmaceutical compositions is a saline solution with appropriate preservatives, such as phenol and/or Tween 80^{™}.

In other aspects of the present invention, the progression and/or response to treatment of a breast cancer may be monitored by performing any of the above assays over a period of time, and evaluating the change in the level of the response (i.e., the amount of polypeptide or mRNA detected or, in the case of a skin test, the extent of the immune response detected). For example, the assays may be performed every month to every other month for a period of 1 to 2 years. In general, breast cancer is progressing in those patients in whom the level of the response increases over time. In contrast, breast cancer is not progressing when the signal detected either remains constant or decreases with time.

In further aspects of the present invention, the compounds described herein may be used for the immunotherapy of breast cancer. In these aspects, the compounds (which may be polypeptides, antibodies or nucleic acid molecules) are preferably incorporated into pharmaceutical compositions or vaccines. Pharmaceutical compositions comprise one or more such compounds and a physiologically acceptable carrier. Vaccines may comprise one or more polypeptides and an immune response enhancer, such as an adjuvant or a liposome (into which the compound is incorporated). Pharmaceutical compositions and vaccines may additionally contain a delivery system, such as biodegradable microspheres which are disclosed, for example, in U.S. Patent Nos. 4,897,268 and 5,075,109. Pharmaceutical compositions and vaccines within the scope of the present invention may also contain other compounds, including one or more separate polypeptides.

Alternatively, a vaccine may contain DNA encoding one or more of the polypeptides as described above, such that the polypeptide is generated *in situ.* In such vaccines, the DNA may be present within any of a variety of delivery systems known to those of ordinary skill in the art, including nucleic acid expression systems, bacteria and viral expression systems. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface. In a preferred embodiment, the DNA may be introduced using a viral expression system (*e.g.*, vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749 (1993), and reviewed by Cohen, Science 259:1691-1692 (1993). The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.*, polylactate polyglycolate) may also be employed as carriers for the pharmaceutical compositions of this invention.

Any of a variety of adjuvants may be employed in the vaccines of this invention to nonspecifically enhance the immune response. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI), Merck Adjuvant 65 (Merck and Company, Inc., Rahway, NJ), alum, biodegradable microspheres, monophosphoryl lipid A and quil A. Cytokines, such as GM-CSF or interleukin-2, -7, or -12, may also be used as adjuvants.

The above pharmaceutical compositions and vaccines may be used, for example, for the therapy of breast cancer in a patient. As used herein, a "patient" refers to any warm-blooded animal, preferably a human. A patient may or may not be afflicted with breast cancer. Accordingly, the above pharmaceutical compositions and vaccines may be used to prevent the development of breast cancer or to treat a patient afflicted with breast cancer. To prevent the development of breast cancer, a pharmaceutical composition or vaccine comprising one or more polypeptides as described herein may be administered to a patient. Alternatively, naked DNA or plasmid or viral vector encoding the polypeptide may be administered. For treating a patient with breast cancer, the pharmaceutical composition or vaccine may comprise one or more polypeptides, antibodies or nucleotide sequences complementary to DNA encoding a polypeptide as described herein (*e.g.*, antisense RNA or antisense deoxyribonucleotide oligonucleotides).

Routes and frequency of administration, as well as dosage, will vary from individual to individual. In general, the pharmaceutical compositions and vaccines may be administered by injection (*e.g.*, intracutaneous, intramuscular, intravenous or subcutaneous), intranasally (*e.g.*, by aspiration) or orally. Between 1 and 10 doses may be administered for a 52-week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g.*, more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 100 µg to 5 mg. Suitable dose sizes will vary with the size of the patient, but will typically range from about 0.1 mL to about 5 mL.

The following Examples are offered by way of illustration and not by way of limitation.

### EXAMPLES

### Example 1

### Preparation of Breast Tumor-Specific cDNAs Using Differential Display RT-PCR

This Example illustrates the preparation of cDNA molecules encoding breast tumor-specific polypeptides using a differential display screen.

### A. Preparation of B18Ag1 cDNA and Characterization of mRNA Expression

Tissue samples were prepared from breast tumor and normal tissue of a patient with breast cancer that was confirmed by pathology after removal from the patient. Normal RNA and tumor RNA was extracted from the samples and mRNA was isolated and converted into cDNA using a (dT)₁₂AG (SEQ ID NO.:130) anchored 3' primer. Differential display PCR was then executed using a randomly chosen primer (CTTCAACCTC) (SEQ ID NO.:103). Amplification conditions were standard buffer containing 1.5 mM MgCl₂, 20 pmol of primer, 500 pmol dNTP, and 1 unit of *Taq* DNA polymerase (Perkin-Elmer, Branchburg, NJ). Forty cycles of amplification were performed using 94°C denaturation for 30 seconds, 42°C annealing for 1 minute, and 72°C extension for 30 seconds. An RNA fingerprint containing 76 amplified products was obtained. Although the RNA fingerprint of breast tumor tissue was over 98% identical to that of the normal breast tissue, a band was repeatedly observed to be specific to the RNA fingerprint pattern of the tumor. This band was cut out of a silver stained gel, subcloned into the T-vector (Novagen, Madison, WI) and sequenced.

The sequence of the cDNA, referred to as B18Ag1, is provided in SEQ ID NO:1. A database search of GENBANK and EMBL revealed that the B18Ag1 fragment initially cloned is 77% identical to the endogenous human retroviral element S71, which is a truncated retroviral element homologous to the Simian Sarcoma Virus (SSV). S71 contains an incomplete *gag* gene, a portion of the *pol* gene and an LTR-like structure at the 3' terminus *(see* Werner et al., Virology 174:225-238 (1990)). B18Ag1 is also 64% identical to SSV in the region corresponding to the P30 (gag) locus. B18Agl contains three separate and incomplete reading frames covering a region which shares considerable homology to a wide variety of gag proteins of retroviruses which infect mammals. In addition, the homology to S71 is not just within the *gag* gene, but spans several kb of sequence including an LTR.

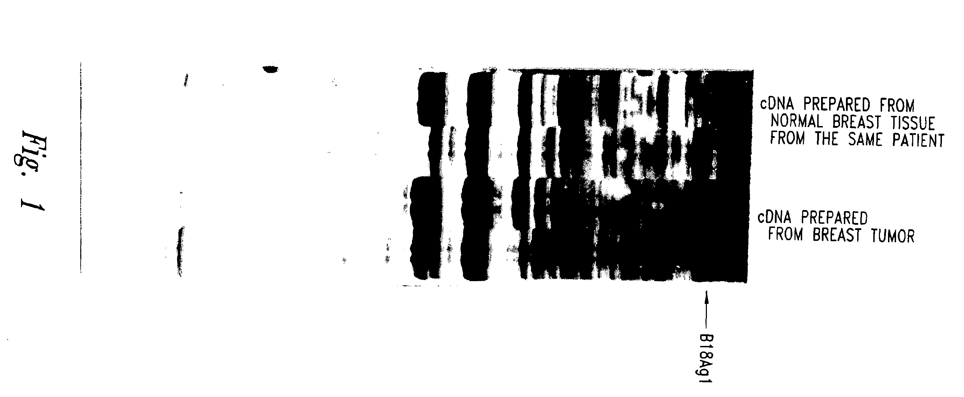

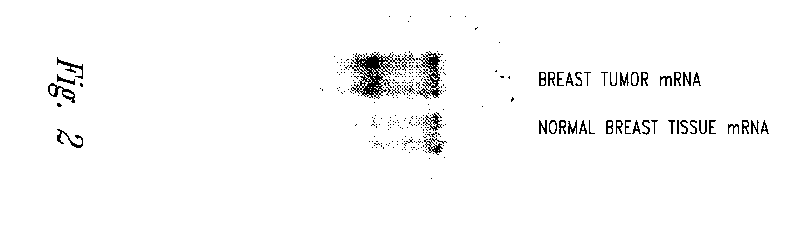

B18Ag1-specific PCR primers were synthesized using computer analysis guidelines. RT-PCR amplification (94°C, 30 seconds; 60°C → 42°C, 30 seconds; 72°C, 30 seconds for 40 cycles) confirmed that B18Ag1 represents an actual mRNA sequence present at relatively high levels in the patient's breast tumor tissue. The primers used in amplification were B18Ag1-1 (CTG CCT GAG CCA CAA ATG) (SEQ ID NO.:128) and B18Ag1-4 (CCG GAG GAG GAA GCT AGA GGA ATA) (SEQ ID NO.:129) at a 3.5 mM magnesium concentration and a pH of 8.5, and B18Ag1-2 (ATG GCT ATT TTC GGG GCC TGA CA) (SEQ ID NO.:126) and B18Ag1-3 (CCG GTA TCT CCT CGT GGT TATT) (SEQ ID NO.:127) at 2 mM magnesium at pH 9.5. The same experiments showed exceedingly low to nonexistent levels of expression in this patient's normal breast tissue *(see* Figure 1). RT-PCR experiments were then used to show that B18Ag1 mRNA is present in nine other breast tumor samples (from Brazilian and American patients) but absent in, or at exceedingly low levels in, the normal breast tissue corresponding to each cancer patient. RT-PCR analysis has also shown that the B18Agl transcript is not present in various normal tissues (including lymph node, myocardium and liver) and present at relatively low levels in PBMC and lung tissue. The presence of B18Ag1 mRNA in breast tumor samples, and its absence from normal breast tissue, has been confirmed by Northern blot analysis, as shown in Figure 2.

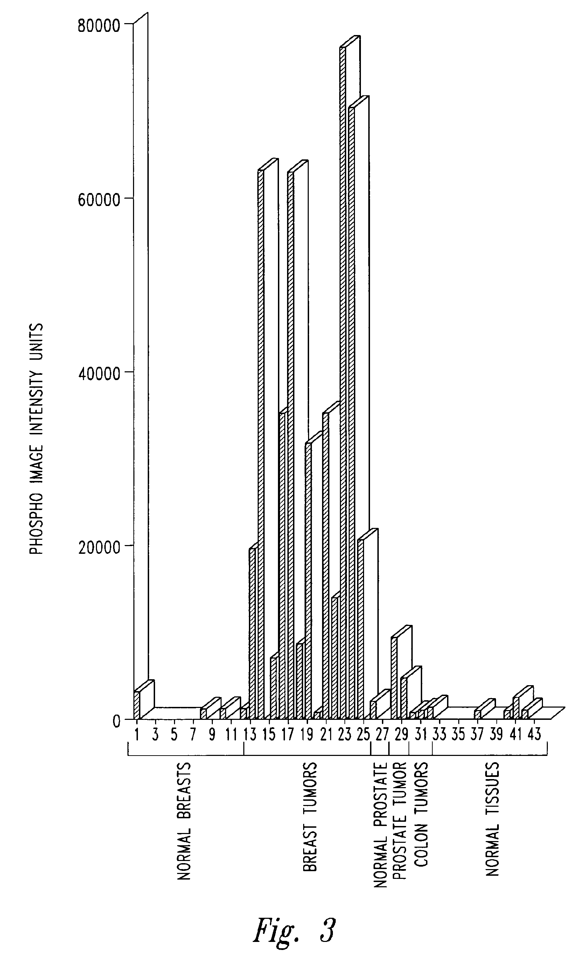

The differential expression of B18Ag1 in breast tumor tissue was also confirmed by RNase protection assays. Figure 3 shows the level of B18Ag1 mRNA in various tissue types as determined in four different RNase protection assays. Lanes 1-12 represent various normal breast tissue samples, lanes 13-25 represent various breast tumor samples; lanes 26-27 represent normal prostate samples; lanes 28-29 represent prostate tumor samples; lanes 30-32 represent colon tumor samples; lane 33 represents normal aorta; lane 34 represents normal small intestine; lane 35 represents normal skin, lane 36 represents normal lymph node; lane 37 represents normal ovary; lane 38 represents normal liver; lane 39 represents normal skeletal muscle; lane 40 represents a first normal stomach sample, lane 41 represents a second normal stomach sample; lane 42 represents a normal lung; lane 43 represents normal kidney; and lane 44 represents normal pancreas. Interexperimental comparison was facilitated by including a positive control RNA of known B-actin message abundance in each assay and normalizing the results of the different assays with respect to this positive control.

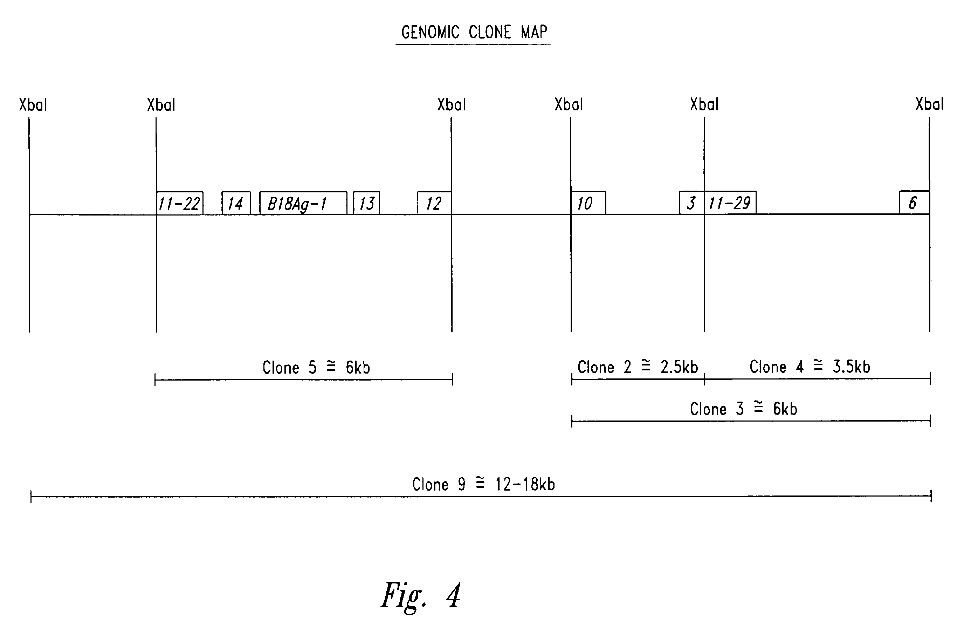

RT-PCR and Southern Blot analysis has shown the B18Ag1 locus to be present in human genomic DNA as a single copy endogenous retroviral element. A genomic clone of approximately 12-18 kb was isolated using the initial B18Ag1 sequence as a probe. Four additional subclones were also isolated by XbaI digestion. Additional retroviral sequences obtained from the ends of the XbaI digests of these clones (located as shown in Figure 4) are shown as SEQ ID NO:3 - SEQ ID NO:10, where SEQ ID NO:3 shows the location of the sequence labeled 10 in Figure 4, SEQ ID NO:4 shows the location of the sequence labeled 11-29, SEQ ID NO:5 shows the location of the sequence labeled 3, SEQ ID NO:6 shows the location of the sequence labeled 6, SEQ ID NO:7 shows the location of the sequence labeled 12, SEQ ID NO:8 shows the location of the sequence labeled 13, SEQ ID NO:9 shows the location of the sequence labeled 14 and SEQ ID NO: 1 0 shows the location of the sequence labeled 11-22.

Subsequent studies demonstrated that the 12-18 kb genomic clone contains a retroviral element of about 7.75 kb, as shown in Figures 5A and 5B. The sequence of this retroviral element is shown in SEQ ID NO: 141. The numbered line at the top of Figure 5A represents the sense strand sequence of the retroviral genomic clone. The box below this line shows the position of selected restriction sites. The arrows depict the different overlapping clones used to sequence the retroviral element. The direction of the arrow shows whether the single-pass subclone sequence corresponded to the sense or anti-sense strand. Figure 5B is a schematic diagram of the retroviral element containing B18Ag1 depicting the organization of viral genes within the element. The open boxes correspond to predicted reading frames, starting with a methionine, found throughout the element. Each of the six likely reading frames is shown, as indicated to the left of the boxes, with frames 1-3 corresponding to those found on the sense strand.

Using the cDNA of SEQ ID NO:1 as a probe, a longer cDNA was obtained (SEQ ID NO:227) which contains minor nucleotide differences (less than 1 %) compared to the genomic sequence shown in SEQ ID NO:141.

### B. Preparation of cDNA Molecules Encoding Other Breast Tumor-Specific Polypeptides

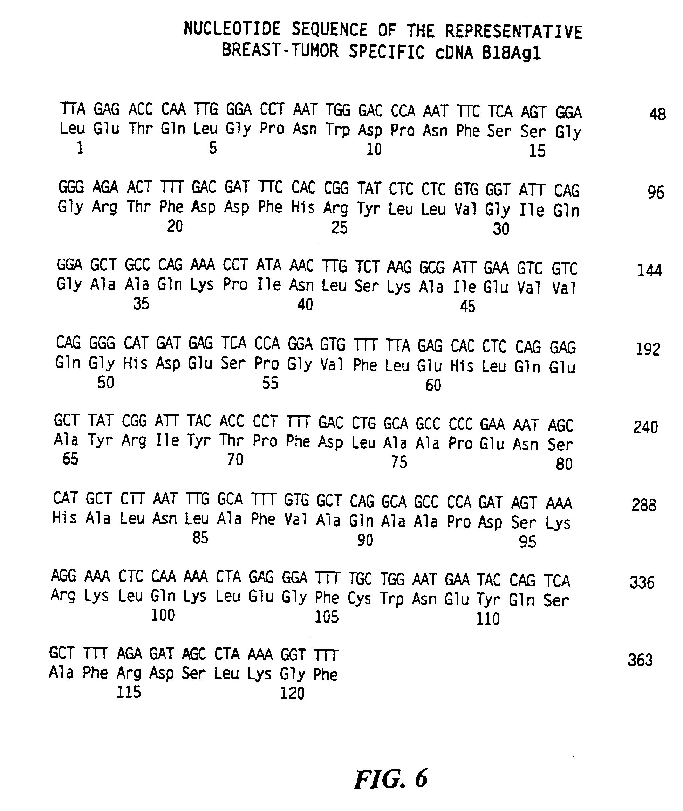

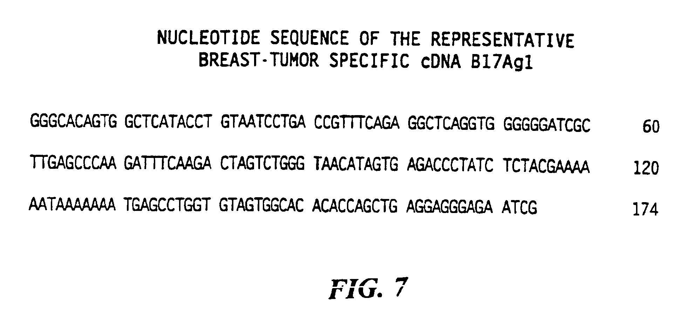

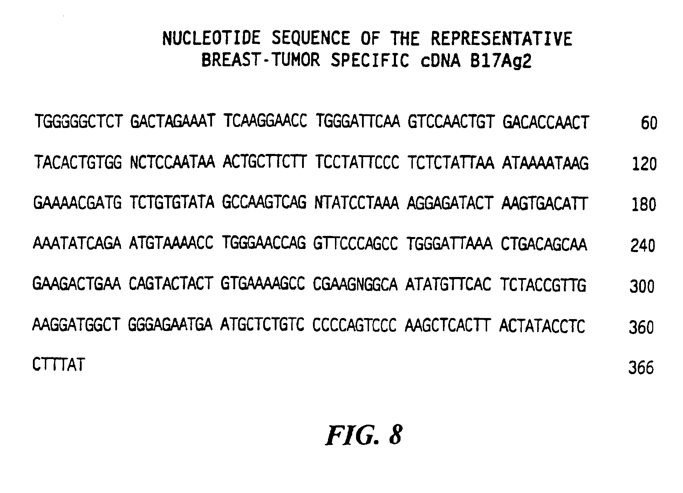

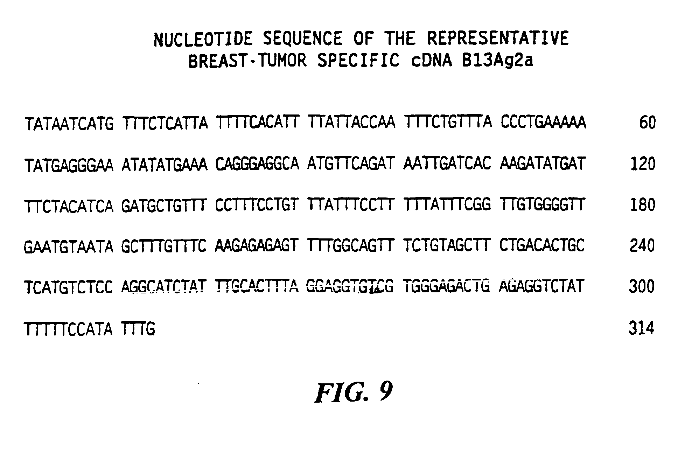

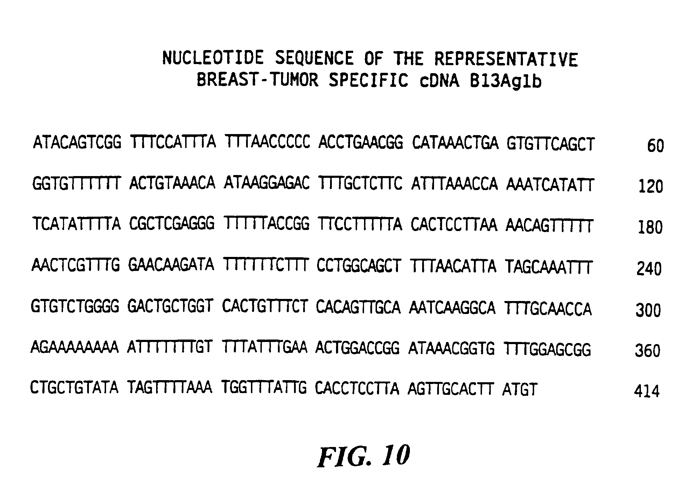

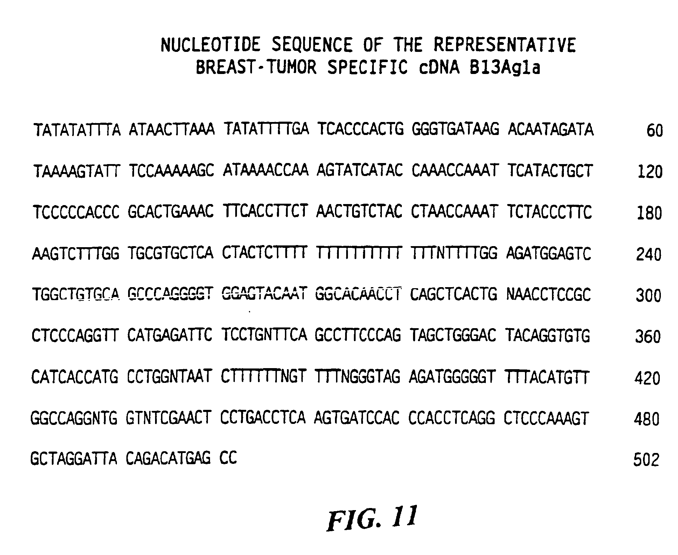

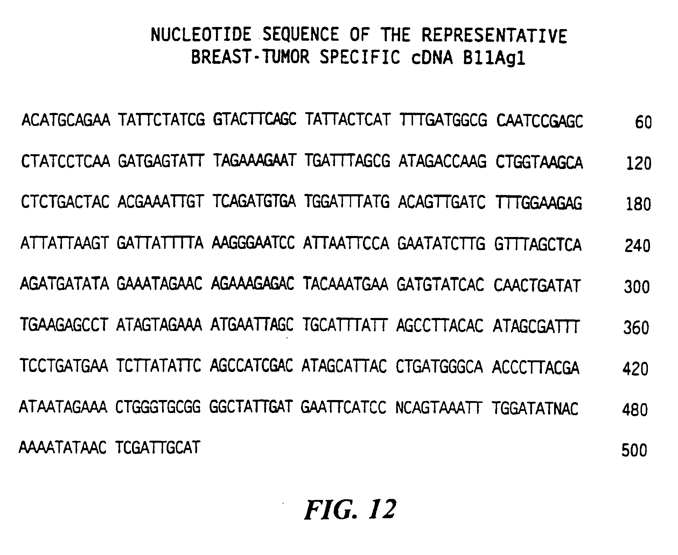

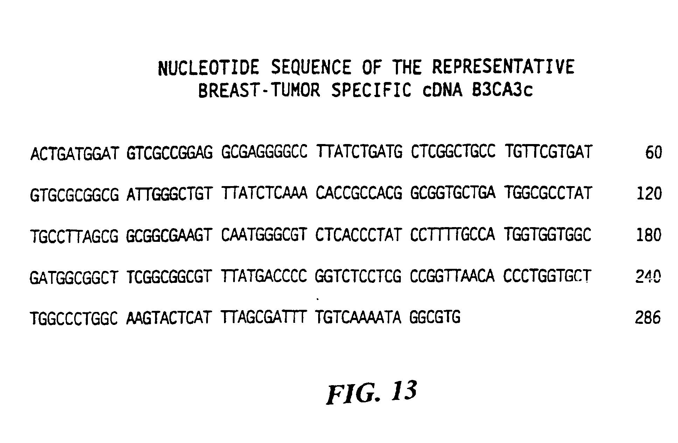

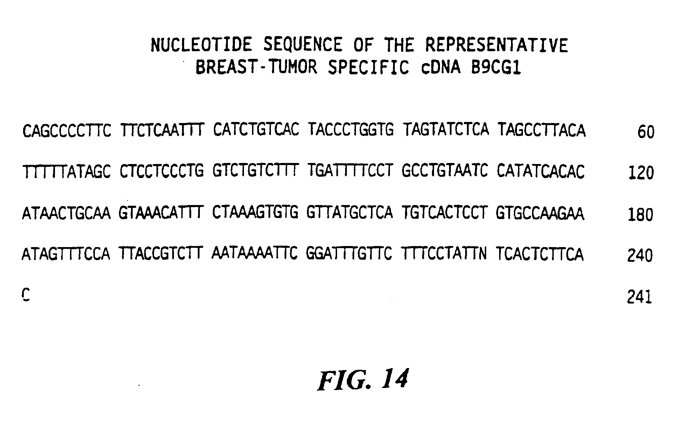

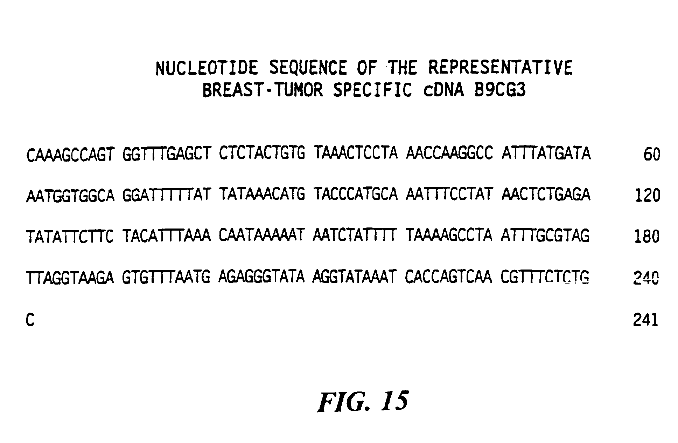

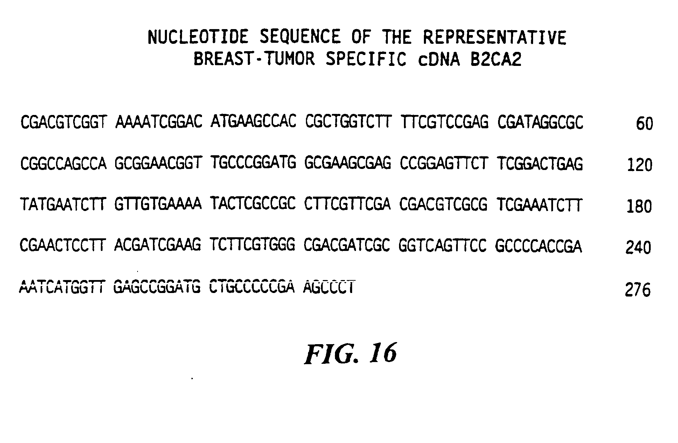

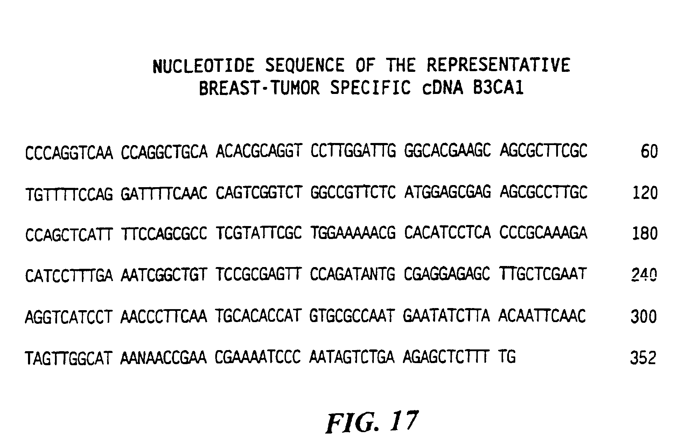

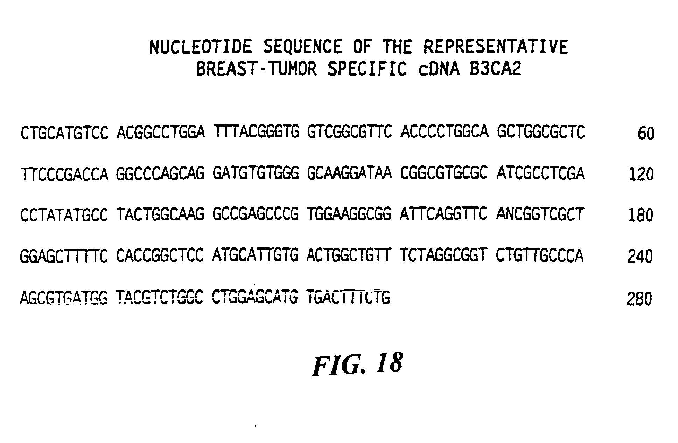

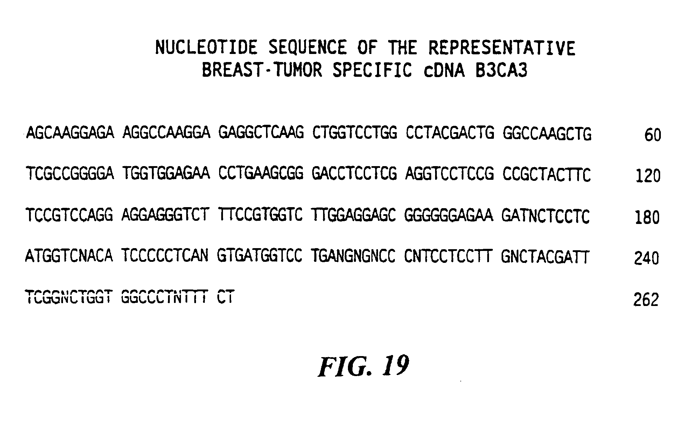

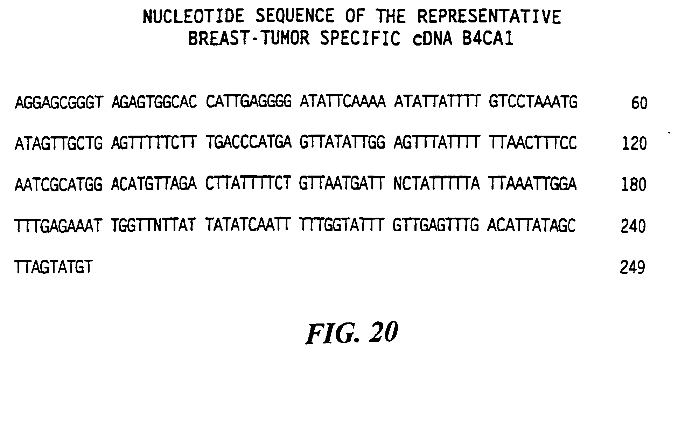

Normal RNA and tumor RNA was prepared and mRNA was isolated and converted into cDNA using a (dT)₁₂AG anchored 3' primer, as described above. Differential display PCR was then executed using the randomly chosen primers SEQ ID NO.: 87-125. Amplification conditions were as noted above, and bands observed to be specific to the RNA fingerprint pattern of the tumor were cut out of a silver stained gel, subcloned into either the T-vector (Novagen, Madison, WI) or the pCRII vector (Invitrogen, San Diego, CA) and sequenced. The sequences are provided in SEQ ID NO:11 - SEQ ID NO:86. Of the 79 sequences isolated, 67 were found to be novel (SEQ ID NO.:11-77) *(see also* Figures 6-20). Subsequent studies identified an additional 84 sequences (SEQ ID NOS: 142-226), of which 72 appeared to be novel (SEQ ID NOS:142, 143, 146-152, 154-166, 168-176, 178-192, 194-198, 200-204, 206, 207, 209-214, 216, 218, 219, 221-227). To the best of the inventors' knowledge none of the previously identified sequences have heretofore been shown to be expressed at a greater level in human breast tumor tissue than in normal breast tissue.

Table I shows the level of representative breast tumor-specific transcripts present in normal breast tissue (columns BNI -BN7), breast tumor samples (columns BTI-BT12) and normal prostate, kidney, liver, lung, skin, small intestine, stomach, myocardium, lymph node, pancreas, skeletal muscle, ovary and aorta, as determined by RT-PCR analysis. A 0-3 grading scale for message abundance is used, with 0 denoting no detectable message and 3 a message level comparable to the control message (glyceraldehyde 3-phosphate dehydrogenase). The lack of data in a given box indicates that the tissue has not been tested for the presence or absence of that specific antigen.

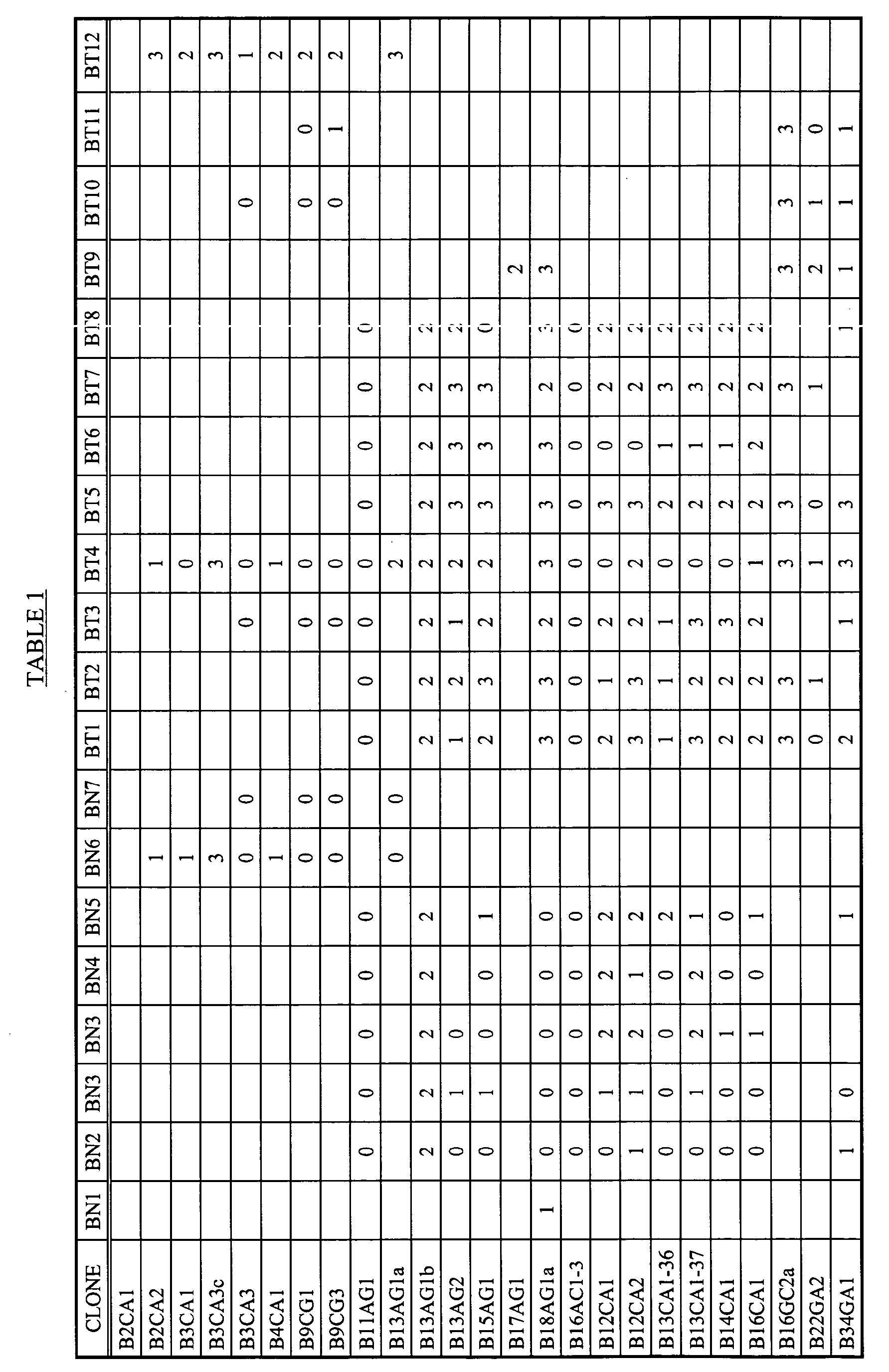

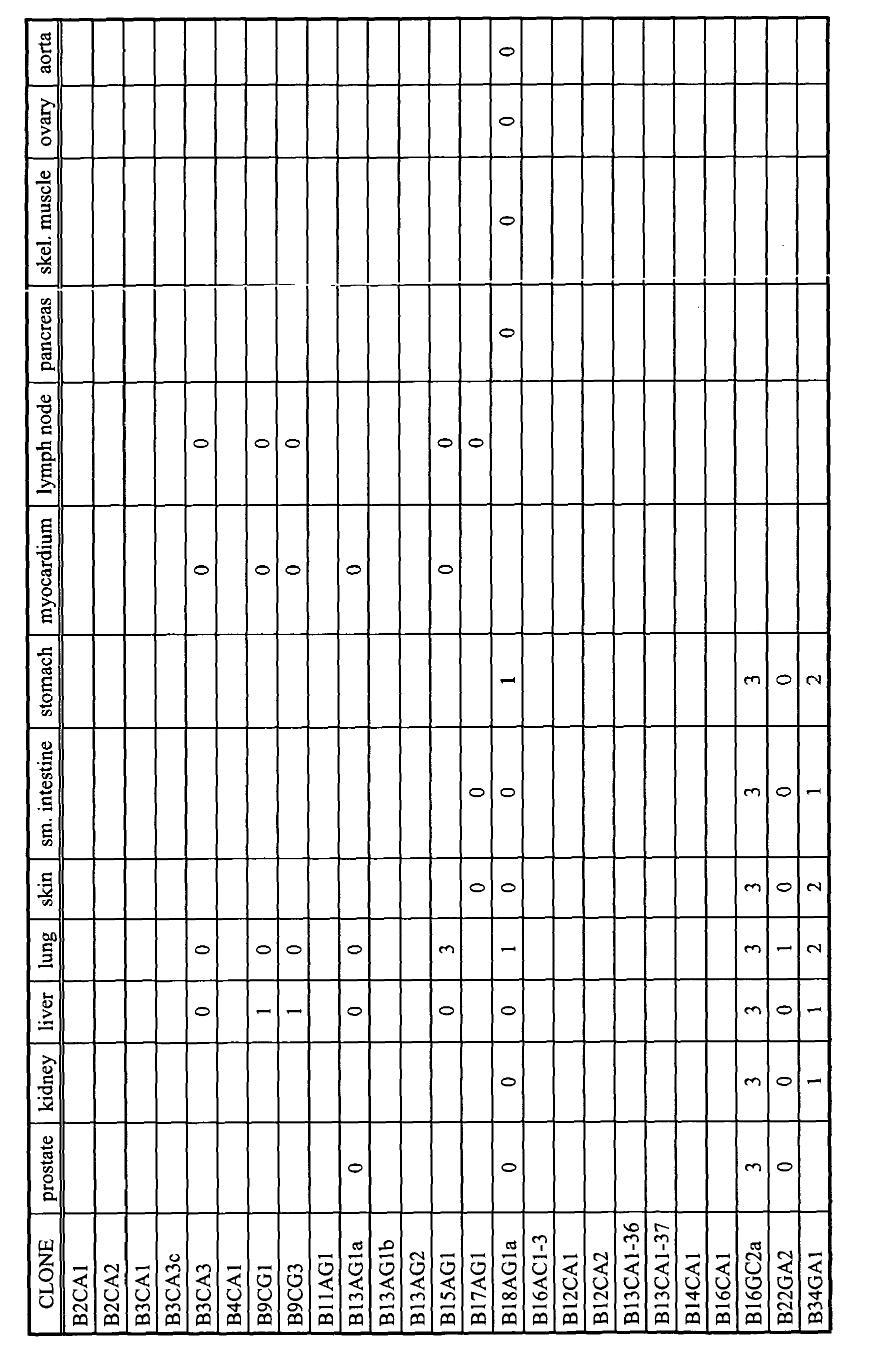

### Example 2

### Preparation of B18Ag1 DNA from Human Genomic DNA

This Example illustrates the preparation of B18Ag1 DNA by amplification from human genomic DNA.

B18Ag1 DNA may be prepared from 250 ng human genomic DNA using 20 pmol of B18Ag1 specific primers, 500 pmol dNTPS and 1 unit of *Taq* DNA polymerase (Perkin Elmer, Branchburg, NJ) using the following amplification parameters: 94°C for 30 seconds denaturing, 30 seconds 60°C to 42°C touchdown annealing in 2°C increments every two cycles and 72°C extension for 30 seconds. The last increment (a 42°C annealing temperature) should cycle 25 times. Primers were selected using computer analysis. Primers synthesized were B18Ag1-1, B18Ag1-2, B18Ag1-3, and B18Ag1-4. Primer pairs that may be used are 1+3, 1+4, 2+3, and 2+4.

Following gel electrophoresis, the band corresponding to B18Ag1 DNA may be excised and cloned into a suitable vector.

### Example 3

### Preparation of B18Ag1 DNA from Breast Tumor cDNA

This Example illustrates the preparation of B18Ag1 DNA by amplification from human breast tumor cDNA.

First strand cDNA is synthesized from RNA prepared from human breast tumor tissue in a reaction mixture containing 500 ng poly A+ RNA, 200 pmol of the primer (T)₁₂AG (*i.e*., TTT TTT TTT TTT AG) (SEQ ID NO: 130), 1X first strand reverse transcriptase buffer, 6.7 mM DTT, 500 mmol dNTPs, and 1 unit AMV or MMLV reverse transcriptase (from any supplier, such as Gibco-BRL (Grand Island, NY)) in a final volume of 30 µ1. After first strand synthesis, the cDNA is diluted approximately 25 fold and 1 µl is used for amplification as described in Example 2. While some primer pairs can result in a heterogeneous population of transcripts, the primers B18Ag1-2 (5'ATG GCT ATT TTC GGG GGC TGA CA) (SEQ ID NO: 126) and B18Ag1-3 (5'CCG GTA TCT CCT CGT GGG TAT T) (SEQ ID NO: 127) yield a single 151 bp amplification product.

### Example 4

### Identification of B-cell and T-cell Epitopes of B18Ag1

This Example illustrates the identification of B18Ag1 epitopes.

The B18Ag1 sequence can be screened using a variety of computer algorithms. To determine B-cell epitopes, the sequence can be screened for hydrophobicity and hydrophilicity values using the method of Hopp, Prog. Clin. Biol. Res. 172B:367-77 (1985) or, alternatively, Cease et al., J. Exp. Med. 164:1779-84 (1986) or Spouge et al., J. Immunol. 138:204-12 (1987). Additional Class II MHC (antibody or B-cell) epitopes can be predicted using programs such as AMPHI (*e.g.*, Margalit et al., J. Immunol. 138:2213 (1987)) or the methods of Rothbard and Taylor *(e.g.,*EMBO J. 7:93 (1988)).

Once peptides (15-20 amino acids long) are identified using these techniques, individual peptides can be synthesized using automated peptide synthesis equipment (available from manufacturers such as Applied Biosystems, Inc., Foster City, CA) and techniques such as Merrifield synthesis. Following synthesis, the peptides can used to screen sera harvested from either normal or breast cancer patients to determine whether patients with breast cancer possess antibodies reactive with the peptides. Presence of such antibodies in breast cancer patient would confirm the immunogenicity of the specific B-cell epitope in question. The peptides can also be tested for their ability to generate a serologic or humoral immune in animals (mice, rats, rabbits, chimps etc.) following immunization *in vivo.* Generation of a peptide-specific antiserum following such immunization further confirms the immunogenicity of the specific B-cell epitope in question.

To identify T-cell epitopes, the B18Ag1 sequence can be screened using different computer algorithms which are useful in identifying 8-10 amino acid motifs within the B18Ag1 sequence which are capable of binding to HLA Class I MHC molecules. *(see, e.g.,* Rammensee et al., Immunogenetics 41:178-228 (1995)). Following synthesis such peptides can be tested for their ability to bind to class I MHC using standard binding assays (*e.g*., Sette et al., J. Immunol. 153:5586-92 (1994)) and more importantly can be tested for their ability to generate antigen reactive cytotoxic T-cells following *in vitro* stimulation of patient or normal peripheral mononuclear cells using, for example, the methods of Bakker et al., Cancer Res. 55:5330-34 (1995); Visseren et al., J. Immunol. 154:3991-98 (1995); Kawakami et al., J. Immunol. 154:3961-68 (1995); and Kast et al., J. Immunol. 152:3904-12 (1994). Successful *in vitro* generation of T-cells capable of killing autologous (bearing the same Class I MHC molecules) tumor cells following *in vitro* peptide stimulation further confirms the immunogenicity of the B18Ag1 antigen. Furthermore, such peptides may be used to generate murine peptide and B18Ag1 reactive cytotoxic T-cells following *in vivo* immunization in mice rendered transgenic for expression of a particular human MHC Class I haplotype (Vitiello et al., J. Exp. Med. 173:1007-15 (1991).

A representative list of predicted B18Ag1 B-cell and T-cell epitopes, broken down according to predicted HLA Class I MHC binding antigen, is shown below:
Predicted Th Motifs (B-cell epitopes) (SEQ ID NOS.: 131-133)
   SSGGRTFDDFHRYLLVGI
   QGAAQKPINLSKXIEWQGHDE
   SPGVFLEHLQEAYRIYTPFDLSA
Predicted HLA A2.1 Motifs (T-cell epitopes) (SEQ ID NOS.: 134-140)
   YLLVGIQGA
   GAAQKPINL
   NLSKXIEVV
   EVVQGHDES
   HLQEAYRIY
   NLAFVAQAA
   FVAQAAPDS

### Example 5

### Characterization of Breast Tumor Genes Discovered by Differential Display PCR

The specificity and sensitivity of the breast tumor genes discovered by differential display PCR were determined using RT-PCR. This procedure enabled the rapid evaluation of breast tumor gene mRNA expression semiquantitatively without using large amounts of RNA. Using gene specific primers, mRNA expression levels in a variety of tissues were examined, including 8 breast tumors, 5 normal breasts, 2 prostate tumors, 2 colon tumors, 1 lung tumor, and 14 other normal adult human tissues, including normal prostate, colon, kidney, liver, lung, ovary, pancreas, skeletal muscle, skin, stomach and testes.

To ensure the semiquantitative nature of the RT-PCR, β-actin was used as internal control for each of the tissues examined. Serial dilutions of the first strand cDNAs were prepared and RT-PCR assays performed using β-actin specific primers. A dilution was then selected that enabled the linear range amplification of β-actin template, and which was sensitive enough to reflect the difference in the initial copy number. Using this condition, the β-actin levels were determined for each reverse transcription reaction from each tissue. DNA contamination was minimized by DNase treatment and by assuring a negative result when using first strand cDNA that was prepared without adding reverse transcriptase.

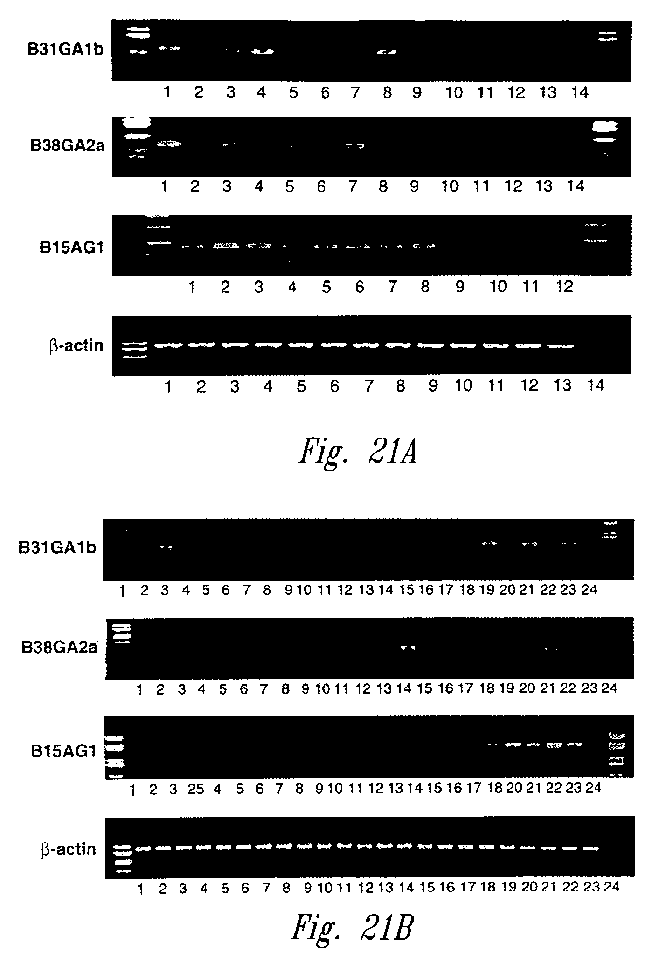

Using gene specific primers, the mRNA expression levels were determined in a variety of tissues. To date 32 genes have been successfully examined by RT-PCR, three of which exhibit good specificity and sensitivity for breast tumors. Figures 21A and 21B depict the results for these three genes: B15AG-1 (SEQ ID NO:27), B31GAlb (SEQ ID NO: 148) and B38GA2a (SEQ ID NO. 157).

From the foregoing, it will be appreciated that, although specific embodiments of the invention have been described herein for the purpose of illustration, various modifications may be made without deviating from the spirit and scope of the invention.

## Claims

1. An isolated DNA molecule, comprising:
(a) a nucleotide sequence selected from the group consisting of SEQ ID NO:211, SEQ ID NO:1, SEQ ID NO:3 - SEQ ID NO:77 and SEQ ID NOS:142, 143, 146-152, 154-166, 168-176, 178-192, 194-198, 200-204, 206, 207, 209, 210, 212-214, 216, 218, 219, 221-227;
(b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained; or
(c) a nucleotide sequence encoding an epitope of a polypeptide encoded by at least one sequence selected from the group consisting of SEQ ID NO:211, SEQ ID NO:1, SEQ ID NO:3 - SEQ ID NO:77 and SEQ ID NOS:142, 143, 146-152, 154-166, 168-176, 178-192, 194-198, 200-204, 206, 207, 209, 210, 212-214, 216, 218, 219, 221-227.

2. An isolated DNA molecule encoding an epitope of a polypeptide, wherein said polypeptide is encoded by a nucleotide sequence that:
(a) hybridizes to a sequence selected from the group consisting of SEQ ID NO:211, SEQ ID NO:1, SEQ ID NO:3 - SEQ ID NO:77 and SEQ ID NOS:142, 143, 146-152, 154-166, 168-176, 178-192, 194-198, 200-204, 206, 207, 209, 210, 212-214, 216, 218, 219, 221-227 under stringent conditions; and
(b) is at least 80% identical to a sequence selected from the group consisting of SEQ ID NO:211, SEQ ID NO:1, SEQ ID NO:3 - SEQ ID NO:77 and SEQ ID NOS:142, 143, 146-152, 154-166, 168-176, 178-192, 194-198, 200-204, 206, 207, 209, 210, 212-214, 216, 218, 219, 221-227;
and wherein RNA corresponding to said nucleotide sequence is expressed at a greater level in human breast tumor tissue than in normal breast tissue.

3. An isolated DNA molecule encoding an epitope of a polypeptide, wherein said polypeptide is encoded by:
(a) a nucleotide sequence transcribed from the sequence of SEQ ID NO: 141; or
(b) a variant of said nucleotide sequence that contains one or more nucleotide substitutions, deletions, insertions and/or modifications at no more than 20% of the nucleotide positions, such that the antigenic and/or immunogenic properties of the polypeptide encoded by the nucleotide sequence are retained.

4. An isolated DNA or RNA molecule comprising a nucleotide sequence complementary to a DNA molecule according to any one of claims 1-3.

5. A recombinant expression vector comprising a DNA molecule according to any one of claims 1-3.

6. A host cell transformed or transfected with an expression vector according to claim 5.

7. A polypeptide comprising an amino acid sequence encoded by a DNA molecule according to any one of claims 1-3.

8. A polypeptide according to claim 7 wherein said polypeptide comprises an epitope of an amino acid sequence encoded by at least one nucleotide sequence selected from the group consisting of SEQ ID NO:211, SEQ ID NO:1, SEQ ID NO:3 - SEQ ID NO:77 and SEQ ID NOS:142, 143, 146-152, 154-166, 168-176, 178-192, 194-198, 200-204, 206, 207, 209, 210, 212-214, 216, 218, 219, 221-227.

9. A monoclonal antibody that binds to a polypeptide according to claim 7.

10. A method for determining the presence of breast cancer in a patient comprising detecting, within a biological sample, at least one polypeptide according to claim 7, and therefrom determining the presence of breast cancer in the patient.

11. A method for determining the presence of breast cancer in a patient comprising detecting within a biological sample, at least one polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions.

12. The method of claims 10 or 11 wherein the biological sample is a portion of a breast tumor.

13. The method of claim 10 wherein the step of detecting comprises contacting the biological sample with a monoclonal antibody according to claim 9.

14. The method of claim 11 wherein the step of detecting comprises contacting the biological sample with a monoclonal antibody that binds to a polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions.

15. A method for determining the presence of breast cancer in a patient comprising detecting, within a biological sample, an RNA molecule encoding at least one polypeptide according to claim 7, and therefrom determining the presence of breast cancer in the patient.

16. A method for determining the presence of breast cancer in a patient comprising detecting, within a biological sample, at least one RNA molecule encoding a polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions; and therefrom determining the presence of breast cancer in the patient.

17. The method of claims 15 or 16 wherein the biological sample is a portion of a breast tumor.

18. The method of claim 15 wherein the step of detecting comprises:
(a) preparing cDNA from RNA molecules within the biological sample; and
(b) specifically amplifying cDNA molecules that are capable of encoding at least a portion of a polypeptide according to claim 7, and therefrom determining the presence of breast cancer in the patient.

19. The method of claim 16 wherein the step of detecting comprises:
(a) preparing cDNA from RNA molecules within the biological sample; and
(b) specifically amplifying cDNA molecules that are capable of encoding at least a portion of a polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions; and therefrom determining the presence of breast cancer in the patient.

20. A polypeptide according to claim 7 for use within a method for detecting the presence of breast cancer in a patient.

21. A polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions, for use within a method for detecting the presence of breast cancer in a patient.

22. A method for monitoring the progression of breast cancer in a patient, comprising:
(a) detecting an amount, in a biological sample, of at least one polypeptide according to claim 7 at a first point in time;
(b) repeating step (a) at a subsequent point in time; and
(c) comparing the amounts of polypeptide detected in steps (a) and (b), and therefrom monitoring the progression of breast cancer in the patient.

23. A method for monitoring the progression of breast cancer in a patient, comprising:
(a) detecting in a biological sample an amount of at least one polypeptide at a first point in time, the polypeptide being encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions;
(b) repeating step (a) at a subsequent point in time; and
(c) comparing the amounts of polypeptide detected in steps (a) and (b), and therefrom monitoring the progression of breast cancer in the patient.

24. The method of claims 22 or 23 wherein the biological sample is a portion of a breast tumor.

25. The method of claim 22 wherein the step of detecting comprises contacting a portion of the biological sample with a monoclonal antibody according to claim 9.

26. The method of claim 23 wherein the step of detecting comprises contacting the biological sample with a monoclonal antibody that binds to a polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions.

27. The method of either of claims 20 or 22 wherein said polypeptide comprises an epitope of an amino acid sequence encoded by at least one nucleotide sequence selected from the group consisting of SEQ ID NO:211, SEQ ID NO:1, SEQ ID NO:3 - SEQ SEQ ID NOS:142, 143, 146-152, 154-166, 168-176, 178-192, 194-198, 200-204, 206, 207, 209, 210, 212-214, 216, 218, 219, 221-227.

28. A method for monitoring the progression of breast cancer in a patient, comprising:
(a) detecting an amount, within a biological sample, of at least one RNA molecule encoding a polypeptide according to claim 7 at a first point in time;
(b) repeating step (a) at a subsequent point in time; and
(c) comparing the amounts of RNA molecules detected in steps (a) and (b), and therefrom monitoring the progression of breast cancer in the patient.

29. The method of claim 28 wherein the step of detecting comprises:
(a) preparing cDNA from RNA molecules within the biological sample; and
(b) specifically amplifying cDNA molecules that are capable of encoding at least a portion of a polypeptide according to claim 7.

30. A method for monitoring the progression of breast cancer in a patient, comprising:
(a) detecting an amount, within a biological sample, of at least one RNA molecule at a first point in time, the RNA molecule encoding a polypeptide encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions;
(b) repeating step (a) at a subsequent point in time; and
(c) comparing the amounts of RNA molecules detected in steps (a) and (b), and therefrom monitoring the progression of breast cancer in the patient.

31. A pharmaceutical composition, comprising a polypeptide according to claim 7 and a physiologically acceptable carrier.

32. A pharmaceutical composition for inhibiting the development of breast cancer, comprising a polypeptide and a physiologically acceptable carrier, the polypeptide being encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions.

33. A vaccine, comprising a polypeptide according to claim 7 and an immune response enhancer.

34. A vaccine, comprising a DNA molecule according to any one of claims 1-3.

35. A vaccine, comprising a recombinant expression vector comprising a DNA molecule according to any one of claims 1-3.

36. A vaccine for inhibiting the development of breast cancer, comprising a polypeptide and an immune response enhancer, the polypeptide being encoded by a nucleotide sequence selected from the group consisting of SEQ ID NO: 78-86 and SEQ ID NOS:144, 145, 153, 167, 177, 193, 199, 205, 208, 215, 217, 220, and sequences that hybridize thereto under stringent conditions.

37. A diagnostic kit comprising:
(a) one or more monoclonal antibodies according to claim 9; and
(b) a detection reagent.

38. A diagnostic kit comprising:
(a) one or more monoclonal antibodies that bind to a polypeptide encoded by a nucleotide sequence selected from the group consisting of sequences provided in SEQ ID 78-86 and SEQ ID NOS:144, 145, 153, 167, 177, 193, 199, 205, 208, 215, 217, 220; and
(b) a detection reagent.

39. The kit of any one of claims 37 or 38 wherein the monoclonal antibody(s) are immobilized on a solid support.

40. A diagnostic kit comprising a first polymerase chain reaction primer and a second polymerase chain reaction primer, the first and second primers each comprising at least about 10 contiguous nucleotides of an RNA molecule according to claim 4.

41. A diagnostic kit comprising a first polymerase chain reaction primer and a second polymerase chain reaction primer, the first and second primers each comprising at least about 10 contiguous nucleotides of an RNA molecule encoding a polypeptide encoded by a nucleotide sequence selected from the group consisting of SEQ ID NOS:78-86 and SEQ ID NOS:144, 145, 153, 167, 177, 193, 199, 205, 208, 215, 217, 220.

42. A diagnostic kit comprising at least one oligonucleotide probe, the oligonucleotide probe containing at least about 15 contiguous nucleotides of a DNA molecule according to claim 4.

43. A diagnostic kit comprising at least one oligonucleotide probe, the oligonucleotide probe comprising at least about 15 contiguous nucleotides of a DNA sequence selected from the group consisting of SEQ ID NOS:78-86 and SEQ ID NOS:144, 145, 153, 167, 177, 193, 199, 205, 208, 215, 217, 220.


SEQUENCE LISTING

(1) GENERAL INFORMATION:

(i) APPLICANT: Corixa Corporation

(ii) TITLE OF INVENTION: COMPOSITIONS AND METHODS FOR THE
TREATMENT AND DIAGNOSIS OF BREAST CANCER

(iii) NUMBER OF SEQUENCES: 227

(iv) CORRESPONDENCE ADDRESS:
(A) ADDRESSEE: SEED and BERRY LLP
(B) STREET: 6300 Columbia Center, 701 Fifth Avenue
(C) CITY: Seattle
(D) STATE: Washington
(E) COUNTRY: USA
(F) ZIP: 98104-7092

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER:
(B) FILING DATE: 10-JAN-1997
(C) CLASSIFICATION:

(viii) ATTORNEY/AGENT INFORMATION:
(A) NAME: Maki, David J.
(B) REGISTRATION NUMBER: 31,392
(C) REFERENCE/DOCKET NUMBER: 210121.419PC

(ix) TELECOMMUNICATION INFORMATION:
(A) TELEPHONE: (206) 622-4900
(B) TELEFAX: (206) 682-6031

(2) INFORMATION FOR SEQ ID NO:1:

(i) SEQUENCE CHARACTERISTICS:
(A) LENGTH: 363 base pairs
(B) TYPE: nucleic acid
(C) STRANDEDNESS: single
(D) TOPOLOGY: linear

(ix) FEATURE:
(A) NAME/KEY: CDS
(B) LOCATION: 1..363

(xi) SEQUENCE DESCRIPTION: SEQ ID NO:1:
TTA GAG ACC CAA TTG GGA CCT AAT TGG GAC CCA AAT TTC TCA AGT GGA 48
GGG AGA ACT TTT GAC GAT TTC CAC CGG TAT CTC CTC GTG GGT ATT CAG 96
GGA GCT GCC CAG AAA CCT ATA AAC TTG TCT AAG GCG ATT GAA GTC GTC 144
CAG GGG CAT GAT GAG TCA CCA GGA GTG TTT TTA GAG CAC CTC CAG GAG 192
GCT TAT CGG ATT TAC ACC CCT TTT GAC CTG GCA GCC CCC GAA AAT AGC 240
CAT GCT CTT AAT TTG GCA TTT GTG GCT CAG GCA GCC CCA GAT AGT AAA 288
AGG AAA CTC CAA AAA CTA GAG GGA TTT TGC TGG AAT GAA TAC CAG TCA 336
GCT TTT AGA GAT AGC CTA AAA GGT TTT 363

(2) INFORMATION FOR SEQ ID NO:2:

(i) SEQUENCE CHARACTERISTICS:
(A) LENGTH: 121 amino acids
(B) TYPE: amino acid
(D) TOPOLOGY: linear

(ii) MOLECULE TYPE: protein

(xi) SEQUENCE DESCRIPTION: SEQ ID NO:2:

(2) INFORMATION FOR SEQ ID NO:3:

(i) SEQUENCE CHARACTERISTICS:
(A) LENGTH: 1101 base pairs
(B) TYPE: nucleic acid
(C) STRANDEDNESS: single
(D) TOPOLOGY: linear

(xi) SEQUENCE DESCRIPTION: SEQ ID NO:3:

TCTTAGAATC TTCATACCCC GAACTCTTGG GAAAACTTTA ATCAGTCACC TACAGTCTAC 60

CACCCATTTA GGAGGAGCAA AGCTACCTCA GCTCCTCCGG AGCCGTTTTA AGATCCCCCA 120

TCTTCAAAGC CTAACAGATC AAGCAGCTCT CCGGTGCACA ACCTGCGCCC AGGTAAATGC 180

CAAAAAAGGT CCTAAACCCA GCCCAGGCCA CCGTCTCCAA GAAAACTCAC CAGGAGAAAA 240

GTGGGAAATT GACTTTACAG AAGTAAAACC ACACCGGGCT GGGTACAAAT ACCTTCTAGT 300

ACTGGTAGAC ACCTTCTCTG GATGGACTGA AGCATTTGCT ACCAAAAACG AAACTGTCAA 360

TATGGTAGTT AAGTTTTTAC TCAATGAAAT CATCCCTCGA CGTGGGCTGC CTGTTGCCAT 420

AGGGTCTGAT AATGGAACGG CCTTCGCCTT GTCTATAGTT TAATCAGTCA GTAAGGCGTT 480

AAACATTCAA TGGAAGCTCC ATTGTGCCTA TCGACCCAGA GCTCTGGGCA AGTAGAACGC 540

ATGAACTGCA CCCTAAAAAA ACACTCTTAC AAAATTAATC TTAAAAACCG GTGTTAATTG 600

TGTTAGTCTC CTTCCCTTAG CCCTACTTAG AGTTAAGGTG CACCCCTTAC TGGGCTGGGT 660

TCTTTACCTT TTGAAATCAT NTTTNGGAAG GGGCTGCCTA TCTTTNCTTA ACTAAAAAAN 720

GCCCATTTGG CAAAAATTTC NCAACTAATT TNTACGTNCC TACGTCTCCC CAACAGGTAN 780

AAAAATCTNC TGCCCTTTTC AAGGAACCAT CCCATCCATT CCTNAACAAA AGGCCTGCCN 840

TTCTTCCCCC AGTTAACTNT TTTTTNTTAA AATTCCCAAA AAANGAACCN CCTGCTGGAA 900

AAACNCCCCC CTCCAANCCC CGGCCNAAGN GGAAGGTTCC CTTGAATCCC NCCCCCNCNA 960

ANGGCCCGGA ACCNTTAAAN TNGTTCCNGG GGGTNNGGCC TAAAAGNCCN ATTTGGTAAA 1020

CCTANAAATT TTTTCTTTTN TAAAAACCAC NNTTTNNTTT TTCTTAAACA AAACCCTNTT 1080

TNTAGNANCN TATTTCCCNC C 1101

(2) INFORMATION FOR SEQ ID NO:4:

(i) SEQUENCE CHARACTERISTICS:

(A) LENGTH: 1087 base pairs

(B) TYPE: nucleic acid

(C) STRANDEDNESS: single

(D) TOPOLOGY: linear

(xi) SEQUENCE DESCRIPTION: SEQ ID NO:4:

TCTAGAGCTG CGCCTGGATC CCGCCACAGT GAGGAGACCT GAAGACCAGA GAAAACACAG 60

CAAGTAGGCC CTTTAAACTA CTCACCTGTG TTGTCTTCTA ATTTATTCTG TTTTATTTTG 120

TTTCCATCAT TTTAAGGGGT TAAAATCATC TTGTTCAGAC CTCAGCATAT AAAATGACCC 180

ATCTGTAGAC CTCAGGCTCC AACCATACCC CAAGAGTTGT CTGGTTTTGT TTAAATTACT 240

GCCAGGTTTC AGCTGCAGAT ATCCCTGGAA GGAATATTCC AGATTCCCTG AGTAGTTTCC 300

AGGTTAAAAT CCTATAGGCT TCTTCTGTTT TGAGGAAGAG TTCCTGTCAG AGAAAAACAT 360

GATTTTGGAT TTTTAACTTT AATGCTTGTG AAACGCTATA AAAAAAATTT TCTACCCCTA 420

GCTTTAAAGT ACTGTTAGTG AGAAATTAAA ATTCCTTCAG GAGGATTAAA CTGCCATTTC 480

AGTTACCCTA ATTCCAAATG TTTTGGTGGT TAGAATCTTC TTTAATGTTC TTGAAGAAGT 540

GTTTTATATT TTCCCATCNA GATAAATTCT CTCNCNCCTT NNTTTTNTNT CTNNTTTTTT 600

AAAACGGANT CTTGCTCCGT TGTCCANGCT GGGAATTTTN TTTTGGCCAA TCTCCGCTNC 660

CTTGCAANAA TNCTGCNTCC CAAAATTACC NCCTTTTTCC CACCTCCACC CCNNGGAATT 720

ACCTGGAATT ANAGGCCCCC NCCCCCCCCC CGGCTAATTT GTTTTTGTTT TTAGTAAAAA 780

ACGGGTTTCC TGTTTTAGTT AGGATGGCCC ANNTCTGACC CCNTNATCNT CCCCCTCNGC 840

CCTCNAATNT TNGGNNTANG GCTTACCCCC CCCNGNNGTT TTTCCTCCAT TNAAATTTTC 900

TNTGGANTCT TGAATNNCGG GTTTTCCCTT TTAAACCNAT TTTTTTTTTN NNNCCCCCAN 960

TTTTNCCTCC CCCNTNTNTA ANGGGGGTTT CCCAANCCGG GTCCNCCCCC ANGTCCCCAA 1020

TTTTTCTCCC CCCCCCTCTT TTTTCTTTNC CCCAAAANTC CTATCTTTTC CTNNAAATAT 1080

CNANTNT 1087

(2) INFORMATION FOR SEQ ID NO:5:

(i) SEQUENCE CHARACTERISTICS:
(A) LENGTH: 1010 base pairs
(B) TYPE: nucleic acid
(C) STRANDEDNESS: single
(D) TOPOLOGY: linear

(xi) SEQUENCE DESCRIPTION: SEQ ID NO:5:

TCTAGACCAA GAAATGGGAG GATTTTAGAG TGACTGATGA TTTCTCTATC ATCTGCAGTT 60

AGTAAACATT CTCCACAGTT TATGCAAAAA GTAACAAAAC CACTGCAGAT GACAAACACT 120

AGGTAACACA CATACTATCT CCCAAATACC TACCCACAAG CTCAACAATT TTAAACTGTT 180

AGGATCACTG GCTCTAATCA CCATGACATG AGGTCACCAC CAAACCATCA AGCGCTAAAC 240

AGACAGAATG TTTCCACTCC TGATCCACTG TGTGGGAAGA AGCACCGAAC TTACCCACTG 300

GGGGGCCTGC NTCANAANAA AAGCCCATGC CCCCGGGTNT NCCTTTNAAC CGGAACGAAT 360

NAACCCACCA TCCCCACANC TCCTCTGTTC NTGGGCCCTG CATCTTGTGG CCTCNTNTNC 420

TTTNGGGGAN ACNTGGGGAA GGTACCCCAT TTCNTTGACC CCNCNANAAA ACCCCNGTGG 480

CCCTTTGCCC TGATTCNCNT GGGCCTTTTC TCTTTTCCCT TTTGGGTTGT TTAAATTCCC 540

AATGTCCCCN GAACCCTCTC CNTNCTGCCC AAAACCTACC TAAATTNCTC NCTANGNNTT 600

TTCTTGGTGT TNCTTTTCAA AGGTNACCTT NCCTGTTCAN NCCCNACNAA AATTTNTTCC 660

NTATNNTGGN CCCNNAAAAA NNNATCNNCC CNAATTGCCC GAATTGGTTN GGTTTTTCCT 720

NCTGGGGGAA ACCCTTTAAA TTTCCCCCTT GGCCGGCCCC CCTTTTTTCC CCCCTTTNGA 780

AGGCAGGNGG TTCTTCCCGA ACTTCCAATT NCAACAGCCN TGCCCATTGN TGAAACCCTT 840

TTCCTAAAAT TAAAAAATAN CCGGTTNNGG NNGGCCTCTT TCCCCTCCNG GNGGGNNGNG 900

AAANTCCTTA CCCCNAAAAA GGTTGCTTAG CCCCCNGTCC CCACTCCCCC NGGAAAAATN 960

AACCTTTTCN AAAAAAGGAA TATAANTTTN CCACTCCTTN GTTCTCTTCC 1010

(2) INFORMATION FOR SEQ ID NO:6:

(i) SEQUENCE CHARACTERISTICS:
(A) LENGTH: 950 base pairs
(B) TYPE: nucleic acid
(C) STRANDEDNESS: single
(D) TOPOLOGY: linear

(xi) SEQUENCE DESCRIPTION: SEQ ID NO:6:

TCTAGAGCTC GCGGCCGCGA GCTCTAATAC GACTCACTAT AGGGCGTCGA CTCGATCTCA 60

GCTCACTGCA ATCTCTGCCC CCGGGGTCAT GCGATTCTCC TGCCTCAGCC TTCCAAGTAG 120

CTGGGATTAC AGGCGTGCAA CACCACACCC GGCTAATTTT GTATTTTTAA TAGAGATGGG 180

GTTTTCCCTT GTTGGCCANN ATGGTCTCNA ACCCCTGACC TCNNGTGATC CCCCCNCCCN 240

NGANCTCNNA CTGCTGGGGA TNNCCGNNNN NNNCCTCCCN NCNCNNNNNN NCNCNNTCCN 300

TNNTCCTTNC TCNNNNNNNN CNNTCNNTCC NNCTTCTCNC CNNNTNTTNT CNNCNNCCNN 360

CNNNCCNCNT NCCCNCNNNT TCNCNTNCNN TNTCCNNCNN NNTCNNCNNN CNNNNCNTNN 420

CCNNTACNTC NTNNNCNNNT CCNTCTNTNN CCTCNNCNNT CNCTNCNCNT TNTCTCCTCN 480

NTNNNNNNCT CCNNNNNTCT CNTCNCNNCN TNCCTCNNTN NCCNCNCCCC NCCTCNCNNC 540

CTNNTTTNNN CNNCNNNTCC NTNCCNTTCN NNTCCNNTNN CNNCNTCNCN NNCNTTNTTC 600

CCNCCNNTTC CTTNCNCNTN NNNTNTCNNN CNCNTCNNTC NTTTNCTCCT NNNTCCCNNC 660

TCNNTTCNCC CNNNTCCNCC CCCCNCCTNT CTCTCNCCCN NNTNNNTNTN NNNCNTCCNC 720

TNTCNCNTTC NTCNNTNCNT TNCTNTCNNC NNCNNTNCNC TNCCNTNTNT CTNNNTCNCN 780

TCNCNTNTCN CCNTCCNTTN CTNTCTCCTN TNTCCTTCCC CTCNCCTNCT CNTTCNCCNC 840

CCNNTNTNTN TNNCNCCNNT NCTNNNCNNC CNTCNTTTCN TCTCTNCTNN NNNTNNCCTC 900

NNCCCNTNCC CTNNTNCNCT NCTNNTACCN TNCTNCTCCN TCTTCCTTCC 950

(2) INFORMATION FOR SEQ ID NO:7:

(i) SEQUENCE CHARACTERISTICS:
(A) LENGTH: 1086 base pairs
(B) TYPE: nucleic acid
(C) STRANDEDNESS: single
(D) TOPOLOGY: linear

(xi) SEQUENCE DESCRIPTION: SEQ ID NO:7:

TCTAGAGCTC GCGGCCGCGA GCTCAATTAA CCCTCACTAA AGGGAGTCGA CTCGATCAGA 60

CTGTTACTGT GTCTATGTAG AAAGAAGTAG ACATAAGAGA TTCCATTTTG TTCTGTACTA 120

AGAAAAATTC TTCTGCCTTG AGATGCTGTT AATCTGTAAC CCTAGCCCCA ACCCTGTGCT 180

CACAGAGACA TGTGCTGTGT TGACTCAAGG TTCAATGGAT TTAGGGCTAT GCTTTGTTAA 240

AAAAGTGCTT GAAGATAATA TGCTTGTTAA AAGTCATCAC CATTCTCTAA TCTCAAGTAC 300

CCAGGGACAC AATACACTGC GGAAGGCCGC AGGGACCTCT GTCTAGGAAA GCCAGGTATT 360

GTCCAAGATT TCTCCCCATG TGATAGCCTG AGATATGGCC TCATGGGAAG GGTAAGACCT 420

GACTGTCCCC CAGCCCGACA TCCCCCAGCC CGACATCCCC CAGCCCGACA CCCGAAAAGG 480

GTCTGTGCTG AGGAAGATTA NTAAAAGAGG AAGGCTCTTT GCATTGAAGT AAGAAGAAGG 540

CTCTGTCTCC TGCTCGTCCC TGGGCAATAA AATGTCTTGG TGTTAAACCC GAATGTATGT 600

TCTACTTACT GAGAATAGGA GAAAACATCC TTAGGGCTGG AGGTGAGACA CCCTGGCGGC 660

ATACTGCTCT TTAATGCACG AGATGTTTGT NTAATTGCCA TCCAGGGCCA NCCCCTTTCC 720

TTAACTTTTT ATGANACAAA AACTTTGTTC NCTTTTCCTG CGAACCTCTC CCCCTATTAN 780

CCTATTGGCC TGCCCATCCC CTCCCCAAAN GGTGAAAANA TGTTCNTAAA TNCGAGGGAA 840

TCCAAAACNT TTTCCCGTTG GTCCCCTTTC CAACCCCGTC CCTGGGCCNN TTTCCTCCCC 900

AACNTGTCCC GGNTCCTTCN TTCCCNCCCC CTTCCCNGAN AAAAAACCCC GTNTGANGGN 960

GCCCCCTCAA ATTATAACCT TTCCNAAACA AANNGGTTCN AAGGTGGTTT GNTTCCGGTG 1020

CGGCTGGCCT TGAGGTCCCC CCTNCACCCC AATTTGGAAN CCNGTTTTTT TTATTGCCCN 1080

NTCCCC 1086

(2) INFORMATION FOR SEQ ID NO:8:

(i) SEQUENCE CHARACTERISTICS:
(A) LENGTH: 1177 base pairs
(B) TYPE: nucleic acid
(C) STRANDEDNESS: single
(D) TOPOLOGY: linear

(xi) SEQUENCE DESCRIPTION: SEQ ID NO:8:

NCCNTTTAGA TGTTGACAAN NTAAACAAGC NGCTCAGGCA GCTGAAAAAA GCCACTGATA 60

AAGCATCCTG GAGTATCAGA GTTTACTGTT AGATCAGCCT CATTTGACTT CCCCTCCCAC 120

ATGGTGTTTA AATCCAGCTA CACTACTTCC TGACTCAAAC TCCACTATTC CTGTTCATGA 180

CTGTCAGGAA CTGTTGGAAA CTACTGAAAC TGGCCGACCT GATCTTCAAA ATGTGCCCCT 240

AGGAAAGGTG GATGCCACCG TGTTCACAGA CAGTACCNCC TTCCTCGAGA AGGGACTACG 300

AGGGGCCGGT GCANCTGTTA CCAAGGAGAC TNATGTGTTG TGGGCTCAGG CTTTACCANC 360

AAACACCTCA NCNCNNAAGG CTGAATTGAT CGCCCTCACT CAGGCTCTCG GATGGGGTAA 420

GGGATATTAA CGTTAACACT GACAGCAGGT ACGCCTTTGC TACTGTGCAT GTACGTGGAG 480

CCATCTACCA GGAGCGTGGG CTACTCACTC GGCAGGTGGC TGTNATCCAC TGTAAANGGA 540

CATCAAAAGG AAAACNNGGC TGTTGCCCGT GGTAACCANA AANCTGATCN NCAGCTCNAA 600

GATGCTGTGT TGACTTTCAC TCNCNCCTCT TAAACTTGCT GCCCACANTC TCCTTTCCCA 660

ACCAGATCTG CCTGACAATC CCCATACTCA AAAAAAAAAN AANACTGGCC CCGAACCCNA 720

ACCAATAAAA ACGGGGANGG TNGGTNGANC NNCCTGACCC AAAAATAATG GATCCCCCGG 780

GCTGCAGGAA TTCAATTCAN CCTTATCNAT ACCCCCAACN NGGNGGGGGG GGCCNGTNCC 840

CATTNCCCCT NTATTNATTC TTTNNCCCCC CCCCCGGCNT CCTTTTTNAA CTCGTGAAAG 900

GGAAAACCTG NCTTACCAAN TTATCNCCTG GACCNTCCCC TTCCNCGGTN GNTTANAAAA 960

AAAAGCCCNC ANTCCCNTCC NAAATTTGCA CNGAAAGGNA AGGAATTTAA CCTTTATTTT 1020

TTNNTCCTTT ANTTTGTNNN CCCCCTTTTA CCCAGGCGAA CNGCCATCNT TTAANAAAAA 1080

AAANAGAANG TTTATTTTTC CTTNGAACCA TCCCAATANA AANCACCCGC NGGGGAACGG 1140

GGNGGNAGGC CNCTCACCCC CTTTNTGTNG GNGGGNC 1177

(2) INFORMATION FOR SEQ ID NO:9:

(i) SEQUENCE CHARACTERISTICS:
(A) LENGTH: 1146 base pairs
(B) TYPE: nucleic acid
(C) STRANDEDNESS: single
(D) TOPOLOGY: linear

(xi) SEQUENCE DESCRIPTION: SEQ ID NO:9:

NCCNNTTNNT GATGTTGTCT TTTTGGCCTC TCTTTGGATA CTTTCCCTCT CTTCAGAGGT 60

GAAAAGGGTC AAAAGGAGCT GTTGACAGTC ATCCCAGGTG GGCCAATGTG TCCAGAGTAC 120

AGACTCCATC AGTGAGGTCA AAGCCTGGGG CTTTTCAGAG AAGGGAGGAT TATGGGTTTT 180

CCAATTATAC AAGTCAGAAG TAGAAAGAAG GGACATAAAC CAGGAAGGGG GTGGAGCACT 240

CATCACCCAG AGGGACTTGT GCCTCTCTCA GTGGTAGTAG AGGGGCTACT TCCTCCCACC 300

ACGGTTGCAA CCAAGAGGCA ATGGGTGATG AGCCTACAGG GGACATANCC GAGGAGACAT 360

GGGATGACCC TAAGGGAGTA GGCTGGTTTT AAGGCGGTGG GACTGGGTGA GGGAAACTCT 420

CCTCTTCTTC AGAGAGAAGC AGTACAGGGC GAGCTGAACC GGCTGAAGGT CGAGGCGAAA 480

ACACGGTCTG GCTCAGGAAG ACCTTGGAAG TAAAATTATG AATGGTGCAT GAATGGAGCC 540

ATGGAAGGGG TGCTCCTGAC CAAACTCAGC CATTGATCAA TGTTAGGGAA ACTGATCAGG 600

GAAGCCGGGA ATTTCATTAA CAACCCGCCA CACAGCTTGA ACATTGTGAG GTTCAGTGAC 660

CCTTCAAGGG GCCACTCCAC TCCAACTTTG GCCATTCTAC TTTGCNAAAT TTCCAAAACT 720

TCCTTTTTTA AGGCCGAATC CNTANTCCCT NAAAAACNAA AAAAAATCTG CNCCTATTCT 780

GGAAAAGGCC CANCCCTTAC CAGGCTGGAA GAAATTTTNC CTTTTTTTTT TTTTTGAAGG 840

CNTTTNTTAA ATTGAACCTN AATTCNCCCC CCCAAAAAAA AACCCNCCNG GGGGGCGGAT 900

TTCCAAAAAC NAATTCCCTT ACCAAAAAAC AAAAACCCNC CCTTNTTCCC TTCCNCCCTN 960

TTCTTTTAAT TAGGGAGAGA TNAAGCCCCC CAATTTCCNG GNCTNGATNN GTTTCCCCCC 1020

CCCCCATTTT CCNAAACTTT TTCCCANCNA GGAANCCNCC CTTTTTTTNG GTCNGATTNA 1080

NCAACCTTCC AAACCATTTT TCCNNAAAAA NTTTGNTNGG NGGGAAAAAN ACCTNNTTTT 1140

ATAGAN 1146

(2) INFORMATION FOR SEQ ID NO:10:

(i) SEQUENCE CHARACTERISTICS:
(A) LENGTH: 545 base pairs
(B) TYPE: nucleic acid
(C) STRANDEDNESS: single
(D) TOPOLOGY: linear

(xi) SEQUENCE DESCRIPTION: SEQ ID NO:10:

CTTCATTGGG TACGGGCCCC CTCGAGGTCG ACGGTATCGA TAAGCTTGAT ATCGAATTCC 60

TGCAGCCCGG GGGATCCACT AGTTCTAGAG TCAGGAAGAA CCACCAACCT TCCTGATTTT 120

TATTGGCTCT GAGTTCTGAG GCCAGTTTTC TTCTTCTGTT GAGTATGCGG GATTGTCAGG 180

CAGATCTGGC TGTGGAAAGG AGACTGTGGG CAGCAAGTTT AGAGGCGTGA CTGAAAGTCA 240

CACTGCATCT TGAGCTGCTG AATCAGCTTT CTGGTTACCA CGGGCAACAG CCGTGTTTTC 300

CTTTTGATGT CCTTTACAGT GGATTACAGC CACCTGCTGA GGTGAGTAGC CCACGCTCCT 360

GGTAGATGGC TCCACGTACA TGCACAGTAG CAAAGGCGTA CCTGCTGTCA GTGTTAACGT 420

TAATATCCTT ACCCCATCGG AGAGCCTGAG TGAGGGCGAT CAATTCAGCC CTTTTGTGCT 480

GAGGTGTTTG CTGGTTAAGC CCTGAACCCA CAACACATCT GTCTCCATGG TAACAGCTGC 540

ACCGG 545

(2) INFORMATION FOR SEQ ID NO:11:

(i) SEQUENCE CHARACTERISTICS:
(A) LENGTH: 196 base pairs
(B) TYPE: nucleic acid
(C) STRANDEDNESS: single
(D) TOPOLOGY: linear

(xi) SEQUENCE DESCRIPTION: SEQ ID NO:11:

TCTCCTAGGC TGGGCACAGT GGCTCATACC TGTAATCCTG ACCGTTTCAG AGGCTCAGGT 60

GGGGGGATCG CTTGAGCCCA AGATTTCAAG ACTAGTCTGG GTAACATAGT GAGACCCTAT 120

CTCTACGAAA AAATAAAAAA ATGAGCCTGG TGTAGTGGCA CACACCAGCT GAGGAGGGAG 180

AATCGAGCCT AGGAGA 196

(2) INFORMATION FOR SEQ ID NO:12:

(i) SEQUENCE CHARACTERISTICS:
(A) LENGTH: 388 base pairs
(B) TYPE: nucleic acid
(C) STRANDEDNESS: single
(D) TOPOLOGY: linear

(xi) SEQUENCE DESCRIPTION: SEQ ID NO:12:

TCTCCTAGGC TTGGGGGCTC TGACTAGAAA TTCAAGGAAC CTGGGATTCA AGTCCAACTG 60

TGACACCAAC TTACACTGTG GNCTCCAATA AACTGCTTCT TTCCTATTCC CTCTCTATTA 120

AATAAAATAA GGAAAACGAT GTCTGTGTAT AGCCAAGTCA GNTATCCTAA AAGGAGATAC 180

TAAGTGACAT TAAATATCAG AATGTAAAAC CTGGGAACCA GGTTCCCAGC CTGGGATTAA 240

ACTGACAGCA AGAAGACTGA ACAGTACTAC TGTGAAAAGC CCGAAGNGGC AATATGTTCA 300

CTCTACCGTT GAAGGATGGC TGGGAGAATG AATGCTCTGT CCCCCAGTCC CAAGCTCACT 360

TACTATACCT CCTTTATAGC CTAGGAGA 388

(2) INFORMATION FOR SEQ ID NO:13:

(i) SEQUENCE CHARACTERISTICS:
(A) LENGTH: 337 base pairs

(B) TYPE: nucleic acid
(C) STRANDEDNESS: single
(D) TOPOLOGY: linear

(xi) SEQUENCE DESCRIPTION: SEQ ID NO:13:

TAGTAGTTGC CTATAATCAT GTTTCTCATT ATTTTCACAT TTTATTAACC AATTTCTGTT 60

TACCCTGAAA AATATGAGGG AAATATATGA AACAGGGAGG CAATGTTCAG ATAATTGATC 120

ACAAGATATG ATTTCTACAT CAGATGCTCT TTCCTTTCCT GTTTATTTCC TTTTTATTTC 180

GGTTGTGGGG TCGAATGTAA TAGCTTTGTT TCAAGAGAGA GTTTTGGCAG TTTCTGTAGC 240

TTCTGACACT GCTCATGTCT CCAGGCATCT ATTTGCACTT TAGGAGGTGT CGTGGGAGAC 300

TGAGAGGTCT ATTTTTTCCA TATTTGGGCA ACTACTA 337

(2) INFORMATION FOR SEQ ID NO:14:

(i) SEQUENCE CHARACTERISTICS:
(A) LENGTH: 571 base pairs
(B) TYPE: nucleic acid
(C) STRANDEDNESS: single
(D) TOPOLOGY: linear

(xi) SEQUENCE DESCRIPTION: SEQ ID NO:14:

TAGTAGTTGC CATACAGTGC CTTTCCATTT ATTTAACCCC CACCTGAACG GCATAAACTG 60

AGTGTTCAGC TGGTGTTTTT TACTGTAAAC AATAAGGAGA CTTTGCTCTT CATTTAAACC 120

AAAATCATAT TTCATATTTT ACGCTCGAGG GTTTTTACCG GTTCCTTTTT ACACTCCTTA 180

AAACAGTTTT TAAGTCGTTT GGAACAAGAT ATTTTTTCTT TCCTGGCAGC TTTTAACATT 240

ATAGCAAATT TGTGTCTGGG GGACTGCTGG TCACTGTTTC TCACAGTTGC AAATCAAGGC 300

ATTTGCAACC AAGAAAAAAA AATTTTTTTG TTTTATTTGA AACTGGACCG GATAAACGGT 360

GTTTGGAGCG GCTGCTGTAT ATAGTTTTAA ATGGTTTATT GCACCTCCTT AAGTTGCACT 420

TATGTGGGGG GGGGNTTTTG NATAGAAAGT NTTTANTCAC ANAGTCACAG GGACTTTTNT 480

CTTTTGGNNA CTGAGCTAAA AAGGGCTGNT TTTCGGGTGG GGGCAGATGA AGGCTCACAG 540

GAGGCCTTTC TCTTAGAGGG GGGAACTNCT A 571

(2) INFORMATION FOR SEQ ID NO:15:

(i) SEQUENCE CHARACTERISTICS:
(A) LENGTH: 548 base pairs
(B) TYPE: nucleic acid
(C) STRANDEDNESS: single
(D) TOPOLOGY: linear

(xi) SEQUENCE DESCRIPTION: SEQ ID NO:15:

TATATATTTA ATAACTTAAA TATATTTTGA TCACCCACTG GGGTGATAAG ACAATAGATA 60

TAAAAGTATT TCCAAAAAGC ATAAAACCAA AGTATCATAC CAAACCAAAT TCATACTGCT 120

TCCCCCACCC GCACTGAAAC TTCACCTTCT AACTGTCTAC CTAACCAAAT TCTACCCTTC 180

AAGTCTTTGG TGCGTGCTCA CTACTCTTTT TTTTTTTTTT TTTNTTTTGG AGATGGAGTC 240

TGGCTGTGCA GCCCAGGGGT GGAGTACAAT GGCACAACCT CAGCTCACTG NAACCTCCGC 300

CTCCCAGGTT CATGAGATTC TCCTGNTTCA GCCTTCCCAG TAGCTGGGAC TACAGGTGTG 360

CATCACCATG CCTGGNTAAT CTTTTTTNGT TTTNGGGTAG AGATGGGGGT TTTACATGTT 420

GGCCAGGNTG GTNTCGAACT CCTGACCTCA AGTGATCCAC CCACCTCAGG CTCCCAAAGT 480

GCTAGGATTA CAGACATGAG CCACTGNGCC CAGNCCTGGT GCATGCTCAC TTCTCTAGGC 540

AACTACTA 548

(2) INFORMATION FOR SEQ ID NO:16:

(i) SEQUENCE CHARACTERISTICS:
(A) LENGTH: 638 base pairs
(B) TYPE: nucleic acid
(C) STRANDEDNESS: single
(D) TOPOLOGY: linear

(xi) SEQUENCE DESCRIPTION: SEQ ID NO:16:

TTCCGTTATG CACATGCAGA ATATTCTATC GGTACTTCAG CTATTACTCA TTTTGATGGC 60

GCAATCCGAG CCTATCCTCA AGATGAGTAT TTAGAAAGAA TTGATTTAGC GATAGACCAA 120

GCTGGTAAGC ACTCTGACTA CACGAAATTG TTCAGATGTG ATGGATTTAT GACAGTTGAT 180

CTTTGGAAGA GATTATTAAG TGATTATTTT AAAGGGAATC CATTAATTCC AGAATATCTT 240

GGTTTAGCTC AAGATGATAT AGAAATAGAA CAGAAAGAGA CTACAAATGA AGATGTATCA 300

CCAACTGATA TTGAAGAGCC TATAGTAGAA AATGAATTAG CTGCATTTAT TAGCCTTACA 360

CATAGCGATT TTCCTGATGA ATCTTATATT CAGCCATCGA CATAGCATTA CCTGATGGGC 420

AACCTTACGA ATAATAGAAA CTGGGTGCGG GGCTATTGAT GAATTCATCC NCAGTAAATT 480

TGGATATNAC AAAATATAAC TCGATTGCAT TTGGATGATG GAATACTAAA TCTGGCAAAA 540

GTAACTTTGG AGCTACTAGT AACCTCTCTT TTTGAGATGC AAAATTTTCT TTTAGGGTTT 600

CTTATTCTCT ACTTTACGGA TATTGGAGCA TAACGGGA 638

(2) INFORMATION FOR SEQ ID NO:17:

(i) SEQUENCE CHARACTERISTICS:
(A) LENGTH: 286 base pairs
(B) TYPE: nucleic acid
(C) STRANDEDNESS: single
(D) TOPOLOGY: linear

(xi) SEQUENCE DESCRIPTION: SEQ ID NO:17:

ACTGATGGAT GTCGCCGGAG GCGAGGGGCC TTATCTGATG CTCGGCTGCC TGTTCGTGAT 60

GTGCGCGGCG ATTGGGCTGT TTATCTCAAA CACCGCCACG GCGGTGCTGA TGGCGCCTAT 120

TGCCTTAGCG GCGGCGAAGT CAATGGGCGT CTCACCCTAT CCTTTTGCCA TGGTGGTGGC 180

GATGGCGGCT TCGGCGGCGT TTATGACCCC GGTCTCCTCG CCGGTTAACA CCCTGGTGCT 240

TGGCCCTGGC AAGTACTCAT TTAGCGATTT TGTCAAAATA GGCGTG 286

(2) INFORMATION FOR SEQ ID NO:18:

(i) SEQUENCE CHARACTERISTICS:
(A) LENGTH: 262 base pairs
(B) TYPE: nucleic acid
(C) STRANDEDNESS: single
(D) TOPOLOGY: linear

(xi) SEQUENCE DESCRIPTION: SEQ ID NO:18:

TCGGTCATAG CAGCCCCTTC TTCTCAATTT CATCTGTCAC TACCCTGGTG TAGTATCTCA 60

TAGCCTTACA TTTTTATAGC CTCCTCCCTG GTCTGTCTTT TGATTTTCCT GCCTGTAATC 120

CATATCACAC ATAACTGCAA GTAAACATTT CTAAAGTGTG GTTATGCTCA TGTCACTCCT 180

GTGNCAAGAA ATAGTTTCCA TTACCGTCTT AATAAAATTC GGATTTGTTC TTTNCTATTN 240

TCACTCTTCA CCTATGACCG AA 262

(2) INFORMATION FOR SEQ ID NO:19:

(i) SEQUENCE CHARACTERISTICS:
(A) LENGTH: 261 base pairs
(B) TYPE: nucleic acid
(C) STRANDEDNESS: single
(D) TOPOLOGY: linear

(xi) SEQUENCE DESCRIPTION: SEQ ID NO:19:

TCGGTCATAG CAAAGCCAGT GGTTTGAGCT CTCTACTGTG TAAACTCCTA AACCAAGGCC 60

ATTTATGATA AATGGTGGCA GGATTTTTAT TATAAACATG TACCCATGCA AATTTCCTAT 120

AACTCTGAGA TATATTCTTC TACATTTAAA CAATAAAAAT AATCTATTTT TAAAAGCCTA 180

ATTTGCGTAG TTAGGTAAGA GTGTTTAATG AGAGGGTATA AGGTATAAAT CACCAGTCAA 240

CGTTTCTCTG CCTATGACCG A 261

(2) INFORMATION FOR SEQ ID NO:20:

(i) SEQUENCE CHARACTERISTICS:

(A) LENGTH: 294 base pairs
(B) TYPE: nucleic acid
(C) STRANDEDNESS: single
(D) TOPOLOGY: linear

(xi) SEQUENCE DESCRIPTION: SEQ ID NO:20:

TACAACGAGG CGACGTCGGT AAAATCGGAC ATGAAGCCAC CGCTGGTCTT TTCGTCCGAG 60

CGATAGGCGC CGGCCAGCCA GCGGAACGGT TGCCCGGATG GCGAAGCGAG CCGGAGTTCT 120

TCGGACTGAG TATGAATCTT GTTGTGAAAA TACTCGCCGC CTTCGTTCGA CGACGTCGCG 180

TCGAAATCTT CGANCTCCTT ACGATCGAAG TCTTCGTGGG CGACGATCGC GGTCAGTTCC 240

GCCCCACCGA AATCATGGTT GAGCCGGATG CTGNCCCCGA AGNCCTCGTT TGTN 294

(2) INFORMATION FOR SEQ ID NO:21:

(i) SEQUENCE CHARACTERISTICS:
(A) LENGTH: 208 base pairs
(B) TYPE: nucleic acid
(C) STRANDEDNESS: single
(D) TOPOLOGY: linear

(xi) SEQUENCE DESCRIPTION: SEQ ID NO:21:

TTGGTAAAGG GCATGGACGC AGACGCCTGA CGTTTGGCTG AAAATCTTTC ATTGATTCGT 60

ATCAATGAAT AGGAAAATTC CCAAAGAGGG AATGTCCTGT TGCTCGCCAG TTTTTNTGTT 120

GTTCTCATGG ANAAGGCAAN GAGCTCTTCA GACTATTGGN ATTNTCGTTC GGTCTTCTGC 180

CAACTAGTCG NCTTGCNANG ATCTTCAT 208

(2) INFORMATION FOR SEQ ID NO:22:

(i) SEQUENCE CHARACTERISTICS:
(A) LENGTH: 287 base pairs
(B) TYPE: nucleic acid
(C) STRANDEDNESS: single
(D) TOPOLOGY: linear

(xi) SEQUENCE DESCRIPTION: SEQ ID NO:22:

NCCNTTGAGC TGAGTGATTG AGATNTGTAA TGGTTGTAAG GGTGATTCAG GCGGATTAGG 60

GTGGCGGGTC ACCCGGCAGT GGGTCTCCCG ACAGGCCAGC AGGATTTGGG GCAGGTACGG 120

NGTGCGCATC GCTCGACTAT ATGCTATGGC AGGCGAGCCG TGGAAGGNGG ATCAGGTCAC 180

GGCGCTGGAG CTTTCCACGG TCCATGNATT GNGATGGCTG TTCTAGGCGG CTGTTGCCAA 240

GCGTGATGGT ACGCTGGCTG GAGCATTGAT TTCTGGTGCC AAGGTGG 287

(2) INFORMATION FOR SEQ ID NO:23:

(i) SEQUENCE CHARACTERISTICS:
(A) LENGTH: 204 base pairs
(B) TYPE: nucleic acid
(C) STRANDEDNESS: single
(D) TOPOLOGY: linear

(xi) SEQUENCE DESCRIPTION: SEQ ID NO:23:

TTGGGTAAAG GGAGCAAGGA GAAGGCATGG AGAGGCTCAN GCTGGTCCTG GCCTACGACT 60

GGGCCAAGCT GTCGCCGGGG ATGGTGGAGA ACTGAAGCGG GACCTCCTCG AGGTCCTCCG 120

NCGTTACTTC NCCGTCCAGG AGGAGGGTCT TTCCGTGGTC TNGGAGGAGC GGGGGGAGAA 180

GATNCTCCTC ATGGTCNACA TCCC 204

(2) INFORMATION FOR SEQ ID NO:24:

(i) SEQUENCE CHARACTERISTICS:
(A) LENGTH: 264 base pairs
(B) TYPE: nucleic acid
(C) STRANDEDNESS: single
(D) TOPOLOGY: linear

(xi) SEQUENCE DESCRIPTION: SEQ ID NO:24:

TGGATTGGTC AGGAGCGGGT AGAGTGGCAC CATTGAGGGG ATATTCAAAA ATATTATTTT 60

GTCCTAAATG ATAGTTGCTG AGTTTTTCTT TGACCCATGA GTTATATTGG AGTTTATTTT 120

TTAACTTTCC AATCGCATGG ACATGTTAGA CTTATTTTCT GTTAATGATT NCTATTTTTA 180

TTAAATTGGA TTTGAGAAAT TGGTTNTTAT TATATCAATT TTTGGTATTT GTTGAGTTTG 240

ACATTATAGC TTAGTATGTG ACCA 264

(2) INFORMATION FOR SEQ ID NO:25:

(i) SEQUENCE CHARACTERISTICS:
(A) LENGTH: 376 base pairs
(B) TYPE: nucleic acid
(C) STRANDEDNESS: single
(D) TOPOLOGY: linear

(xi) SEQUENCE DESCRIPTION: SEQ ID NO:25:

TTACAACGAG GGGAAACTCC GTCTCTACAA AAATTAAAAA ATTAGCCAGG TGTGGTGGTG 60

TGCACCCGCA ATCCCAGCTA CTTGGGAGGT TGAGACACAA GANTCACCTA NATGTGGGAG 120

GTCAAGGTTG CATGAGTCAT GATTGTGCCA CTGCACTCCA GCCTGGGTGA CAGACCGAGA 180

CCCTGCCTCA ANAGANAANG AATAGGAAGT TCAGAAATCN TGGNTGTGGN GCCCAGCAAT 240

CTGCATCTAT NCAACCCCTG CAGGCAANGC TGATGCAGCC TANGTTCAAG AGCTGCTGTT 300

TCTGGAGGCA GCAGTTNGGG CTTCCATCCA GTATCACGGC CACACTCGCA CNAGCCATCT 360

GTCCTCCGTN TGTNAC 376

(2) INFORMATION FOR SEQ ID NO:26:

(i) SEQUENCE CHARACTERISTICS:
(A) LENGTH: 372 base pairs
(B) TYPE: nucleic acid
(C) STRANDEDNESS: single
(D) TOPOLOGY: linear

(xi) SEQUENCE DESCRIPTION: SEQ ID NO:26:

TTACAACGAG GGGAAACTCC GTCTCTACAA AAATTAAAAA ATTAGCCAGG TGTGGTGGTG 60

TGCACCTGTA ATCCCAGCTA CTTGGGCGGC TGAGACACAA GAACCACCTA AATGTGGGAG 120

GGTCAAGGTT GCATGAGTCA TGATCGCGCC ACTGCACTCC AGCCTGGGTG ACAGACTGAG 180

ACCCTGCCTC AAAAGAAAAA GAATAGGAAG TTCAGAAACC CTGGGTGTGG NGCCCAGCAA 240

TCTGCATTTA AACAATCCCT GCAGGCAATG CTGATGCAGC CTAAGTTCAA GAGCTGCTGT 300

TCTGGAGGCA GNAGTAAGGG CTTCCATCCA GCATCACGGN CAACACTGCA AAAGCACCTG 360

TCCTCGTTGG TA 372

(2) INFORMATION FOR SEQ ID NO:27:

(i) SEQUENCE CHARACTERISTICS:
(A) LENGTH: 477 base pairs
(B) TYPE: nucleic acid
(C) STRANDEDNESS: single
(D) TOPOLOGY: linear

(xi) SEQUENCE DESCRIPTION: SEQ ID NO:27:

TTCTGTCCAC ATCTACAAGT TTTATTTATT TTGTGGGTTT TCAGGGTGAC TAAGTTTTTC 60

CCTACATTGA AAAGAGAAGT TGCTAAAAGG TGCACAGGAA ATCATTTTTT TAAGTGAATA 120

TGATAATATG GGTCCGTGCT TAATACAACT GAGACATATT TGTTCTCTGT TTTTTTAGAG 180

TCACCTCTTA AAGTCCAATC CCACAATGGT GAAAAAAAAA TAGAAAGTAT TTGTTCTACC 240

TTTAAGGAGA CTGCAGGGAT TCTCCTTGAA AACGGAGTAT GGAATCAATC TTAAATAAAT 300

ATGAAATTGG TTGGTCTTCT GGGATAAGAA ATTCCCAACT CAGTGTGCTG AAATTCACCT 360

GACTTTTTTT GGGAAAAAAT AGTCGAAAAT GTCAATTTGG TCCATAAAAT ACATGTTACT 420

ATTAAAAGAT ATTTAAAGAC AAATTCTTTC AGAGCTCTAA GATTGGTGTG GACAGAA 477

(2) INFORMATION FOR SEQ ID NO:28:

(i) SEQUENCE CHARACTERISTICS:
(A) LENGTH: 438 base pairs
(B) TYPE: nucleic acid

(C) STRANDEDNESS: single
(D) TOPOLOGY: linear

(xi) SEQUENCE DESCRIPTION: SEQ ID NO:28:

TCTNCAACCT CTTGANTGTC AAAAACCTTN TAGGCTATCT CTAAAAGCTG ACTGGTATTC 60

ATTCCAGCAA AATCCCTCTA GTTTTTGGAG TTTCCTTTTA CTATCTGGGG CTGCCTGAGC 120

CACAAATGCC AAATTAAGAG CATGGCTATT TTCGGGGGCT GACAGGTCAA AAGGGGTGTA 180

AATCCGATAA GCCTCCTGGA GGTGCTCTAA AAACACTCCT GGTGACTCAT CATGCCCCTG 240

GACGACTTCA ATCGNCTTAG ACAAGTTTAT AGGTTTCTGG GCAGCTCCCT GAATACCCAC 300

GAGGAGATAC CGGTGGAAAT CGTCAAAAGT TCTCCCTCCA CTTGAGAAAT TTGGGTCCCA 360

ATTAGGTCCC AATTGGGTCT CTAATCACTA TTCCTCTAGC TTCCTCCTCC GGNCTATTGG 420

TTGATGTGAG GTTGAAGA 438

(2) INFORMATION FOR SEQ ID NO:29:

(i) SEQUENCE CHARACTERISTICS:
(A) LENGTH: 620 base pairs
(B) TYPE: nucleic acid
(C) STRANDEDNESS: single
(D) TOPOLOGY: linear

(xi) SEQUENCE DESCRIPTION: SEQ ID NO:29:

AAGAGGGTAC CAGCCCCAAG CCTTGACAAC TTCCATAGGG TGTCAAGCCT GTGGGTGCAC 60

AGAAGTCAAA AATTGAGTTT TGGGATCCTC AGCCTAGATT TCAGAGGATA TAAAGAAACA 120

CCTAACACCT AGATATTCAG ACAAAAGTTT ACTACAGGGA TGAAGCTTTC ACGGAAAACC 180

TCTACTAGGA AAGTACAGAA GAGAAATGTG GGTTTGGAGC CCCCAAACAG AATCCCCTCT 240

AGAACACTGC CTAATGAAAC TGTGAGAAGA TGGCCACTGT CATCCAGACA CCAGAATGAT 300

AGACCCACCA AAAACTTATG CCATATTGCC TATAAAACCT ACAGACACTC AATGCCAGCC 360

CCATGAAAAA AAAACTCAGA AGAAGACTGT NCCCTACAAT GCCACCGGAG CAGAACTGCC 420

CCAGGCCATG GAAGCACAGC TCTTATATCA ATGTGACCTG GATGTTGAGA CATGGAATCC 480

NANGAAATCN TTTTAANACT TCCACGGTTN AATGACTGCC CTATTANATT CNGAACTTAN 540

ATCCNGGCCT GTGACCTCTT TGCTTTGGCC ATTCCCCCTT TTTGGAATGG CTNTTTTTTT 600

CCCATGCCTG TNCCCTCTTA 620

(2) INFORMATION FOR SEQ ID NO:30:

(i) SEQUENCE CHARACTERISTICS:
(A) LENGTH: 100 base pairs
(B) TYPE: nucleic acid
(C) STRANDEDNESS: single
(D) TOPOLOGY: linear

(xi) SEQUENCE DESCRIPTION: SEQ ID NO:30:

TTACAACGAG GGGGTCAATG TCATAAATGT CACAATAAAA CAATCTCTTC TTTTTTTTTT 60

TTTTTTTTTT TTTTTTTTTT TTTTTTTTTT TTTTTTTTTT 100

(2) INFORMATION FOR SEQ ID NO:31:

(i) SEQUENCE CHARACTERISTICS:
(A) LENGTH: 762 base pairs
(B) TYPE: nucleic acid
(C) STRANDEDNESS: single
(D) TOPOLOGY: linear

(xi) SEQUENCE DESCRIPTION: SEQ ID NO:31:

TAGTCTATGC CCCCGACAGA GCAGAATTAA ATTGGAAGTT GCCCTCCGGA CTTTCTACCC 60

ACACTCTTCC TGAAAAGAGA AAGAAAAGAG GCAGGAAAGA GGTTAGGATT TCATTTTCAA 120

GAGTCAGCTA ATTAGGAGAG CAGAGTTTAG ACAGCAGTAG GCACCCCATG ATACAAACCA 180

TGGACAAAGT CCCTGTTTAG TAACTGCCAG ACATGATCCT GCTCAGGTTT TGAAATCTCT 240

CTGCCCATAA AAGATGGAGA GCAGGAGTGC CATCCACATC AACACGTGTC CAAGAAAGAG 300

TCTCAGGGAG ACAAGGGTAT CAAAAAACAA GATTCTTAAT GGGAAGGAAA TCAAACCAAA 360

AAATTAGATT TTTCTCTACA TATATATAAT ATACAGATAT TTAACACATT ATTCCAGAGG 420

TGGCTCCAGT CCTTGGGGCT TGAGAGATGG TGAAAACTTT TGTTCCACAT TAACTTCTGC 480

TCTCAAATTC TGAAGTATAT CAGAATGGGA CAGGCAATGT TTTGCTCCAC ACTGGGGCAC 540

AGACCCAAAT GGTTCTGTGC CCGAAGAAGA GAAGCCCGAA AGACATGAAG GATGCTTAAG 600

GGGGGTTGGG AAAGCCAAAT TGGTANTATC TTTTCCTCCT GCCTGTGTTC CNGAAGTCTC 660

CNCTGAAGGA ATTCTTAAAA CCCTTTGTGA GGAAATGCCC CCTTACCATG ACAANTGGTC 720

CCATTGCTTT TAGGGNGATG GAAACACCAA GGGTTTTGAT CC 762

(2) INFORMATION FOR SEQ ID NO:32:

(i) SEQUENCE CHARACTERISTICS:
(A) LENGTH: 276 base pairs
(B) TYPE: nucleic acid
(C) STRANDEDNESS: single
(D) TOPOLOGY: linear

(xi) SEQUENCE DESCRIPTION: SEQ ID NO:32:

TAGTCTATGC GTGTATTAAC CTCCCCTCCC TCAGTAACAA CCAAAGAGGC AGGAGCTGTT 60

ATTACCAACC CCATTTTACA GATGCATCAA TAATGACAGA GAAGTGAAGT GACTTGCGCA 120

CACAACCAGT AAATTGGCAG AGTCAGATTT GAATCCATGG AGTCTGGTCT GCACTTTCAA 180

TCACCGAATA CCCTTTCTAA GAAACGTGTG CTGAATGAGT GCATGGATAA ATCAGTGTCT 240

ACTCAACATC TTTGCCTAGA TATCCCGCAT AGACTA 276

(2) INFORMATION FOR SEQ ID NO:33:

(i) SEQUENCE CHARACTERISTICS:
(A) LENGTH: 477 base pairs
(B) TYPE: nucleic acid
(C) STRANDEDNESS: single
(D) TOPOLOGY: linear

(xi) SEQUENCE DESCRIPTION: SEQ ID NO:33:

TAGTAGTTGC CAAATATTTG AAAATTTACC CAGAAGTGAT TGAAAACTTT TTGGAAACAA 60

AAACAAATAA AGCCAAAAGG TAAAATAAAA ATATCTTTGC ACTCTCGTTA TTACCTATCC 120

ATAACTTTTT CACCGTAAGC TCTCCTGCTT GTTAGTGTAG TGTGGTTATA TTAAACTTTT 180

TAGTTATTAT TTTTTATTCA CTTTTCCACT AGAAAGTCAT TATTGATTTA GCACACATGT 240

TGATCTCATT TCATTTTTTC TTTTTATAGG CAAAATTTGA TGCTATGCAA CAAAAATACT 300

CAAGCCCATT ATCTTTTTTC CCCCCGAAAT CTGAAAATTG CAGGGGACAG AGGGAAGTTA 360

TCCCATTAAA AAATTGTAAA TATGTTCAGT TTATGTTTAA AAATGCACAA AACATAAGAA 420

AATTGTGTTT ACTTGAGCTG CTGATTGTAA GCAGTTTTAT CTCAGGGGCA ACTACTA 477

(2) INFORMATION FOR SEQ ID NO:34:

(i) SEQUENCE CHARACTERISTICS:
(A) LENGTH: 631 base pairs
(B) TYPE: nucleic acid
(C) STRANDEDNESS: single
(D) TOPOLOGY: linear

(xi) SEQUENCE DESCRIPTION: SEQ ID NO:34:

TAGTAGTTGC CAATTCAGAT GATCAGAAAT GCTGCTTTCC TCAGCATTGT CTTGTTAAAC 60

CGCATGCCAT TTGGAACTTT GGCAGTGAGA AGCCAAAAGG AAGAGGTGAA TGACATATAT 120

ATATATATAT ATTCAATGAA AGTAAAATGT ATATGCTCAT ATACTTTCTA GTTATCAGAA 180

TGAGTTAAGC TTTATGCCAT TGGGCTGCTG CATATTTTAA TCAGAAGATA AAAGAAAATC 240

TGGGCATTTT TAGAATGTGA TACATGTTTT TTTAAAACTG TTAAATATTA TTTCGATATT 300

TGTCTAAGAA CCGGAATGTT CTTAAAATTT ACTAAAACAG TATTGTTTGA GGAAGAGAAA 360

ACTGTACTGT TTGCCATTAT TACAGTCGTA CAAGTGCATG TCAAGTCACC CACTCTCTCA 420

GGCATCAGTA TCCACCTCAT AGCTTTACAC ATTTTGACGG GGAATATTGC AGCATCCTCA 480

GGCCTGACAT CTGGGAAAGG CTCAGATCCA CCTACTGCTC CTTGCTCGTT GATTTGTTTT 540

AAAATATTGT GCCTGGTGTC ACTTTTAAGC CACAGCCCTG CCTAAAAGCC AGCAGAGAAC 600

AGAACCCGCA CCATTCTATA GGCAACTACT A 631

(2) INFORMATION FOR SEQ ID NO:35:

(i) SEQUENCE CHARACTERISTICS:
(A) LENGTH: 578 base pairs
(B) TYPE: nucleic acid
(C) STRANDEDNESS: single
(D) TOPOLOGY: linear

(xi) SEQUENCE DESCRIPTION: SEQ ID NO:35:

TAGTAGTTGC CATCCCATAT TACAGAAGGC TCTGTATACA TGACTTATTT GGAAGTGATC 60

TGTTTTCTCT CCAAACCCAT TTATCGTAAT TTCACCAGTC TTGGATCAAT CTTGGTTTCC 120

ACTGATACCA TGAAACCTAC TTGGAGCAGA CATTGCACAG TTTTCTGTGG TAAAAACTAA 180

AGGTTTATTT GCTAAGCTGT CATCTTATGC TTAGTATTTT TTTTTTACAG TGGGGAATTG 240

CTGAGATTAC ATTTTGTTAT TCATTAGATA CTTTGGGATA ACTTGACACT GTCTTCTTTT 300

TTTCGCTTTT AATTGCTATC ATCATGCTTT TGAAACAAGA ACACATTAGT CCTCAAGTAT 360

TACATAAGCT TGCTTGTTAC GCCTGGTGGT TTAAAGGACT ATCTTTGGCC TCAGGTTCAC 420

AAGAATGGGC AAAGTGTTTC CTTATGTTCT GTAGTTCTCA ATAAAAGATT GCCAGGGGCC 480

GGGTACTGTG GCTCGCACTG TAATCCCAGC ACTTTGGGAA GCTGAGGCTG GCGGATCATG 540

TTAGGGCAGG TGTTCGAAAC CAGCCTGGGC AACTACTA 578

(2) INFORMATION FOR SEQ ID NO:36:

(i) SEQUENCE CHARACTERISTICS:
(A) LENGTH: 583 base pairs
(B) TYPE: nucleic acid
(C) STRANDEDNESS: single
(D) TOPOLOGY: linear

(xi) SEQUENCE DESCRIPTION: SEQ ID NO:36:

TAGTAGTTGC CTGTAATCCC AGCAACTCAG GAGGCTGGGG CAGGAGAATC AGTTGAACCT 60

GGGAGGCAGA AGTTGTAATT AGCAAAGATC GCACCATTGC ACTTCAGCCT GGGCAACAAG 120

AGTGAGATTC CATCTCAAAA ACAAAAAAAA GAAAAAGAAA AGAAAAGGAA AAAACGTATA 180

AACCCAGCCA AAACAAAATG ATCATTCTTT TAATAAGCAA GACTAATTTA ATGTGTTTAT 240

TTAATCAAAG CAGTTGAATC TTCTGAGTTA TTGGTGAAAA TACCCATGTA GTTAATTTAG 300

GGTTCTTACT TGGGTGAACG TTTGATGTTC ACAGGTTATA AAATGGTTAA CAAGGAAAAT 360

GATGCATAAA GAATCTTATA AACTACTAAA AATAAATAAA ATATAAATGG ATAGGTGCTA 420

TGGATGGAGT TTTTGTGTAA TTTAAAATCT TGAAGTCATT TTGGATGCTC ATTGGTTGTC 480

TGGTAATTTC CATTAGGAAA AGGTTATGAT ATGGGGAAAC TGTTTCTGGA AATTGCGGAA 540

TGTTTCTCAT CTGTAAAATG CTAGTATCTC AGGGCAACTA CTA 583

(2) INFORMATION FOR SEQ ID NO:37:

(i) SEQUENCE CHARACTERISTICS:
(A) LENGTH: 716 base pairs
(B) TYPE: nucleic acid
(C) STRANDEDNESS: single
(D) TOPOLOGY: linear

(xi) SEQUENCE DESCRIPTION: SEQ ID NO:37:

GATCTACTAG TCATNTGGAT TCTATCCATG GCAGCTAAGC CTTTCTGAAT GGATTCTACT 60

GCTTTCTTGT TCTTTAATCC AGACCCTTAT ATATGTTTAT GTTCACAGGC AGGGCAATGT 120

TTAGTGAAAA CAATTCTAAA TTTTTTATTT TGCATTTTCA TGCTAATTTC CGTCACACTC 180

CAGCAGGCTT CCTGGGAGAA TAAGGAGAAA TACAGCTAAA GACATTGTCC CTGCTTACTT 240

ACAGCCTAAT GGTATGCAAA ACCACTTCAA TAAAGTAACA GGAAAAGTAC TAACCAGGTA 300

GAATGGACCA AAACTGATAT AGAAAAATCA GAGGAAGAGA GGAACAAATA TTTACTGAGT 360

CCTAGAATGT ACAAGGCTTT TTAATTACAT ATTTTATGTA AGGCCTGCAA AAAACAGGTG 420

AGTAATCAAC ATTTGTCCCA TTTTACATAT AAGGAAACTG AAGCTTAAAT TGAATAATTT 480

AATGCATAGA TTTTATAGTT AGACCATGTT CAGGTCCCTA TGTTATACTT ACTAGCTGTA 540

TGAATATGAG AAAATAATTT TGTTATTTTC TTGGCATCAG TATTTTCATC TGCAAAATAA 600

AGCTAAAGTT ATTTAGCAAA CAGTCAGCAT AGTGCCTGAT ACATAGTAGG TGCTCCAAAC 660

ATGATTACNC TANTATTNGG TATTANAAAA ATCCAATATA GGCNTGGATA AAACCG 716

(2) INFORMATION FOR SEQ ID NO:38:

(i) SEQUENCE CHARACTERISTICS:

(A) LENGTH: 688 base pairs
(B) TYPE: nucleic acid
(C) STRANDEDNESS: single
(D) TOPOLOGY: linear

(xi) SEQUENCE DESCRIPTION: SEQ ID NO:38:

TTCTGTCCAC ATATCATCCC ACTTTAATTG TTAATCAGCA AAACTTTCAA TGAAAAATCA 60

TCCATTTTAA CCAGGATCAC ACCAGGAAAC TGAAGGTGTA TTTTTTTTTA CCTTAAAAAA 120

AAAAAAAAAA ACCAAACAAA CCAAAACAGA TTAACAGCAA AGAGTTCTAA AAAATTTACA 180

TTTCTCTTAC AACTGTCATT CAGAGAACAA TAGTTCTTAA GTCTGTTAAA TCTTGGCATT 240

AACAGAGAAA CTTGATGAAN AGTTGTACTT GGAATATTGT GGATTTTTTT TTTTGTCTAA 300

TCTCCCCCTA TTGTTTTGCC AACAGTAATT TAAGTTTGTG TGGAACATCC CCGTAGTTGA 360

AGTGTAAACA ATGTATAGGA AGGAATATAT GATAAGATGA TGCATCACAT ATGCATTACA 420

TGTAGGGACC TTCACAACTT CATGCACTCA GAAAACATGC TTGAAGAGGA GGAGAGGACG 480

GCCCAGGGTC ACCATCCAGG TGCCTTGAGG ACAGAGAATG CAGAAGTGGC ACTGTTGAAA 540

TTTAGAAGAC CATGTGTGAA TGGTTTCAGG CCTGGGATGT TTGCCACCAA GAAGTGCCTC 600

CGAGAAATTT CTTTCCCATT TGGAATACAG GGTGGCTTGA TGGGTACGGT GGGTGACCCA 660

ACGAAGAAAA TGAAATTCTG CCCTTTCC 688

(2) INFORMATION FOR SEQ ID NO:39:

(i) SEQUENCE CHARACTERISTICS:
(A) LENGTH: 585 base pairs

(B) TYPE: nucleic acid
(C) STRANDEDNESS: single
(D) TOPOLOGY: linear

(xi) SEQUENCE DESCRIPTION: SEQ ID NO:39:

TAGTAGTTGC CGCNNACCTA AAANTTGGAA AGCATGATGT CTAGGAAACA TANTAAAATA 60

GGGTATGCCT ATGTGCTACA GAGAGATGTT AGCATTTAAA GTGCATANTT TTATGTATTT 120

TGACAAATGC ATATNCCTCT ATAATCCACA ACTGATTACG AAGCTATTAC AATTAAAAAG 180

TTTGGCCGGG CGTGGTGGGC GGTGGCTGAC GCCTGTAATC CCAGCACTTT GGGAGGCCGA 240

GGCACGCGGA TCACGAGGTC GGGAGTTCAA GACCATCCTG GCTAACACGG TGAAAGTCCA 300

TCTCTACTAA AAATACGAAA AAATTACCCC GGCGTGGTGG CGGGCGCCTG TAGTCCCAGC 360

TACTCCGGAG GCTGAGGCAG GAGAATGGCG TGAACCCAGG ACACGGAGCT TGCAGTGTGC 420

CAACATCACG TCACTGCCCT CCAGCCTGGG GGACAGGAAC AAGANTCCCG TCCTCANAAA 480

AGAAAAATAC TACTNATANT TTCNACTTTA TTTTAANTTA CACAGAACTN CCTCTTGGTA 540

CCCCCTTACC ATTCATCTCA CCCACCTCCT ATAGGGCACN NCTAA 585

(2) INFORMATION FOR SEQ ID NO:40:

(i) SEQUENCE CHARACTERISTICS:
(A) LENGTH: 475 base pairs
(B) TYPE: nucleic acid
(C) STRANDEDNESS: single
(D) TOPOLOGY: linear

(xi) SEQUENCE DESCRIPTION: SEQ ID NO:40:

TCTGTCCACA CCAATCTTAG AAGCTCTGAA AAGAATTTGT CTTTAAATAT CTTTTAATAG 60

TAACATGTAT TTTATGGACC AAATTGACAT TTTCGACTGT TTTTTCCAAA AAAGTCAGGT 120

GAATTTCAGC ACACTGAGTT GGGAATTTCT TATCCCAGAA GACCAACCAA TTTCATATTT 180

ATTTAAGATT GATTCCATAC TCCGTTTTCA AGGAGAATCC CTGCAGTCTC CTTAAAGGTA 240

GAACAAATAC TTCCTATTTT TTTTTCACCA TTGTGGGATT GGACTTTAAG AGGTGACTCT 300

AAAAAAACAG AGAACAAATA TGTCTCAGTT GTATTAAGCA CGGACCCATA TTATCATATT 360

CACTTAAAAA AATGATTTCC TGTGCACCTT TTGGCAACTT CTCTTTTCAA TGTAGGGAAA 420

AACTTAGTCA CCCTGAAAAC CCACAAAATA AATAAAACTT GTAGATGTGG ACAGA 475

(2) INFORMATION FOR SEQ ID NO:41:

(i) SEQUENCE CHARACTERISTICS:
(A) LENGTH: 423 base pairs
(B) TYPE: nucleic acid
(C) STRANDEDNESS: single
(D) TOPOLOGY: linear

(xi) SEQUENCE DESCRIPTION: SEQ ID NO:41:

TAAGAGGGTA CATCGGGTAA GAACGTAGGC ACATCTAGAG CTTAGAGAAG TCTGGGGTAG 60

GAAAAAAATC TAAGTATTTA TAAGGGTATA GGTAACATTT AAAAGTAGGG CTAGCTGACA 120

TTATTTAGAA AGAACACATA CGGAGAGATA AGGGCAAAGG ACTAAGACCA GAGGAACACT 180

AATATTTAGT GATCACTTCC ATTCTTGGTA AAAATAGTAA CTTTTAAGTT AGCTTCAAGG 240

AAGATTTTTG GCCATGATTA GTTGTCAAAA GTTAGTTCTC TTGGGTTTAT ATTACTAATT 300

TTGTTTTAAG ATCCTTGTTA GTGCTTTAAT AAAGTCATGT TATATCAAAC GCTCTAAAAC 360

ATTGTAGCAT GTTAAATGTC ACAATATACT TACCATTTGT TGTATATGGC TGTACCCTCT 420

CTA 423

(2) INFORMATION FOR SEQ ID NO:42:

(i) SEQUENCE CHARACTERISTICS:
(A) LENGTH: 527 base pairs
(B) TYPE: nucleic acid
(C) STRANDEDNESS: single
(D) TOPOLOGY: linear

(xi) SEQUENCE DESCRIPTION: SEQ ID NO:42:

TCTCCTAGGC TAATGTGTGT GTTTCTGTAA AAGTAAAAAG TTAAAAATTT TAAAAATAGA 60

AAAAAGCTTA TAGAATAAGA ATATGAAGAA AGAAAATATT TTTGTACATT TGCACAATGA 120

GTTTATGTTT TAAGCTAAGT GTTATTACAA AAGAGCCAAA AAGGTTTTAA AAATTAAAAC 180

GTTTGTAAAG TTACAGTACC CTTATGTTAA TTTATAATTG AAGAAAGAAA AACTTTTTTT 240

TATAAATGTA GTGTAGCCTA AGCATACAGT ATTTATAAAG TCTGGCAGTG TTCAATAATG 300

TCCTAGGCCT TCACATTCAC TCACTGACTC ACCCAGAGCA ACTTCCAGTC CTGTAAGCTC 360

CATTCGTGGT AAGTGCCCTA TACAGGTGCA CCATTTATTT TACAGTATTT TTACTGTACC 420

TTCTCTATGT TTCCATATGT TTCGATATAC AAATACCACT GGTTACTATN GCCCNACAGG 480

TAATTCCAGT AACACGGCCT GTATACGTCT GGTANCCCTA GNGAAGA 527

(2) INFORMATION FOR SEQ ID NO:43:

(i) SEQUENCE CHARACTERISTICS:
(A) LENGTH: 331 base pairs
(B) TYPE: nucleic acid
(C) STRANDEDNESS: single
(D) TOPOLOGY: linear

(xi) SEQUENCE DESCRIPTION: SEQ ID NO:43:

TCTTCAACCT CGTAGGACAA CTCTCATATG CCTGGGCACT ATTTTTAGGT TACTACCTTG 60

GCTGCCCTTC TTTAAGAAAA AAAAAAGAAG AAAAAAGAAC TTTTCCACAA GTTTCTCTTC 120

CTCTAGTTGG AAAATTAGAG AAATCATGTT TTTAATTTTG TGTTATTTCA GATCACAAAT 180

TCAAACACTT GTAAACATTA AGCTTCTGTT CAATCCCCTG GGAAGAGGAT TCATTCTGAT 240

ATTTACGGTT CAAAAGAAGT TGTAATATTG TGCTTGGAAC ACAGAGAACC AGTTATTAAC 300

TTCCTACTAC TATTATATAA TAAATAATAA C 331

(2) INFORMATION FOR SEQ ID NO:44:

(i) SEQUENCE CHARACTERISTICS:
(A) LENGTH: 592 base pairs
(B) TYPE: nucleic acid
(C) STRANDEDNESS: single
(D) TOPOLOGY: linear

(xi) SEQUENCE DESCRIPTION: SEQ ID NO:44:

GGCTTAGTAG TTGCCAGGCA AAATARCGTT GATTCTCCTC AGGAGCCACC CCCAACACCC 60

CTGTTTGCTT CTAGACCTAT ACCTAGACTA AAGTCCCAGC AGACCCCTAG AGGTGAGGTT 120

CAGAGTGACC CTTGAGGAGA TGTGCTACAC TAGAAAAGAA CTGCTTGAGT TTTCTAATTT 180

ATATAAGCAG AAATCTGGAG AAGAGTCATA GGAATGGATA TTAAGGGTGT GAGATAATGG 240

CGGAAGGAAT ATAGAGTTGG ATCAGGCTGG ACTTATTGAT TTGAACCCAC TAAGTAGAGA 300

TTCTGCTTTT GATGTTGCAG CTCAGGGAGT TAAAAAAGGT TTTAATGGTT CTAATAGTTT 360

ATTTGCTTGG TTAGCTGAAA TATGGATAAA AGATGGCCCA CTGTGAGCAA GCTGGAAATG 420

CCTGATCTCT CTCAGTTTAA TGTAGAGGAA GGGATCCAAA AGTTTAGGGA GANTTGGATG 480

CTGGRAKTGG ATTGGTCACT TTGRGACCTA CCCWTCCCAG CTGGGAGGGT CCAGAAGATA 540

CACCCTTGAC CAACGCTTTG CGAAATGGAT TTGTGATGGC GGCAACTACT AA 592

(2) INFORMATION FOR SEQ ID NO:45:

(i) SEQUENCE CHARACTERISTICS:
(A) LENGTH: 567 base pairs
(B) TYPE: nucleic acid
(C) STRANDEDNESS: single
(D) TOPOLOGY: linear

(xi) SEQUENCE DESCRIPTION: SEQ ID NO:45:

GGCTTAGTAG TTGCCATTGC GAGTGCTTGC TCAACGAGCG TTGAACATGG CGGATTGTCT 60

AGATTCAACG GATTTGAGTT TTACCAGCAA AGCGAACCAA GCGCGGCCCA GAGAATTATG 120

GGTTGGTTGG CTTTGAAAAG ATGGAAATCC TGTAGGCCTA GTCAGAAAAG CCTTCTTGCA 180

GAACAGTTGG TTCTCGGGCG AACGCTCATC AAGATGCCCA TTGGAAAGGC TAGCGTGTAT 240

TTGGGAGAGC CTGATAGCGT GTCTTCTGAT GATGTTTGTG CTTGGACAGT GACAAAAGAT 300

ATGCAAAGCA AGTCCGAACT AGACGTCAAG CTTCGTGAGC AAATTATTGT AGACTCCTAC 360

TTATACTGTG AGGAATGATA GCCAAGGGTG GGGACTTTAA GACTAAGGTG GTTTGTACTT 420

GCCCCGATGA TCCCAGGCAG AAAGAMCTGA TCGCTAGTTT TATACGGGCA ACTACTAAGC 480

CGAATTCCAG CACACTGGCG GCCGTTACTA ATTGGATCCG ANCTCGGTAC CAGCTTGATG 540

CATASCTTGA GTTWTCTATA NTGTCNC 567

(2) INFORMATION FOR SEQ ID NO:46:

(i) SEQUENCE CHARACTERISTICS:
(A) LENGTH: 908 base pairs
(B) TYPE: nucleic acid
(C) STRANDEDNESS: single
(D) TOPOLOGY: linear

(xi) SEQUENCE DESCRIPTION: SEQ ID NO:46:

GAGCGAAAGA CCGAGGGCAG NGNNTANGNG CGANGAAGCG GAGAGGGCCA AAAAGCAACC 60

GCTTTCCCCG GGGGGTGCCG ATTCATTAAG GCAGGTGGAG GACAGGTTTC CCGATGGAAG 120

GCGGCAGGGG CGCAAGCAAT TAATGTGAGT AGGCCATTCA TTAGCACCCG GGCTTAACAT 180

TTAAGCTTCG GGTTGGTATG TGGTGGGAAT TGTGAGCGGA TAACAATTTC ACACAGGAAA 240

CAGCTATGAC CATGATTACG CCAAGCTATT TAGGTGACAT TATAGAATAA CTCAAGTTAT 300

GCATCAAGCT TGGTACCGAG TTCGGATCCA CTAGTAACGG CCGCCAGTGT GTGGAATTCG 360

GCTTAGTAGT TGCCGACCAT GGAGTGCTAC CTAGGCTAGA ATACCTGAGY TCCTCCCTAG 420

CCTCACTCAC ATTAAATTGT ATCTTTTCTA CATTAGATGT CCTCAGCGCC TTATTTCTGC 480

TGGACWATCG ATAAATTAAT CCTGATAGGA TGATAGCAGC AGATTAATTA CTGAGAGTAT 540

GTTAATGTGT CATCCCTCCT ATATAACGTA TTTGCATTTT AATGGAGCAA TTCTGGAGAT 600

AATCCCTGAA GGCAAAGGAA TGAATCTTGA GGGTGAGAAA GCCAGAATCA GTGTCCAGCT 660

GCAGTTGTGG GAGAAGGTGA TATTATGTAT GTCTCAGAAG TGACACCATA TGGGCAACTA 720

CTAAGCCCGA ATTCCAGCAC ACTGGCGGGC GTTACTAATG GATCCGAGCT CGGTACCAAG 780

CTTGATGCAT AGCTTGAGTA TCTATAGTGT CACTAAATAG CCTGGCGTTA TCATGGTCAT 840

AGCTGTTTCC TGTGTGAAAT TGTTATCCGC TCCCAATTCC CCCCACCATA CGAGCCGGAA 900

CATAAAGT 908

(2) INFORMATION FOR SEQ ID NO:47:

(i) SEQUENCE CHARACTERISTICS:
(A) LENGTH: 480 base pairs
(B) TYPE: nucleic acid
(C) STRANDEDNESS: single
(D) TOPOLOGY: linear

(xi) SEQUENCE DESCRIPTION: SEQ ID NO:47:

TGCCAACAAG GAAAGTTTTA AATTTCCCCT TGAGGATTCT TGGTGATCAT CAAATTCAGT 60

GGTTTTTAAG GTTGTTTTCT GTCAAATAAC TCTAACTTTA AGCCAAACAG TATATGGAAG 120

CACAGATAKA ATATTACACA GATAAAAGAG GAGTTGATCT AAAGTARAGA TAGTTGGGGG 180

CTTTAATTTC TGGAACCTAG GTCTCCCCAT CTTCTTCTGT GCTGAGGAAC TTCTTGGAAG 240

CGGGGATTCT AAAGTTCTTT GGAAGACAGT TTGAAAACCA CCATGTTGTT CTCAGTACCT 300

TTATTTTTAA AAAGTAGGTG AACATTTTGA GAGAGAAAAG GGCTTGGTTG AGATGAAGTC 360

CCCCCCCCCC CTTTTTTTTT TTTTAGCTGA AATAGATACC CTATGTTNAA RGAARGGATT 420

ATTATTTACC ATGCCAYTAR SCACATGCTC TTTGATGGGC NYCTCCSTAC CCTCCTTAAG 480

(2) INFORMATION FOR SEQ ID NO:48:

(i) SEQUENCE CHARACTERISTICS:
(A) LENGTH: 591 base pairs
(B) TYPE: nucleic acid
(C) STRANDEDNESS: single
(D) TOPOLOGY: linear

(xi) SEQUENCE DESCRIPTION: SEQ ID NO:48:

AAGAGGGTAC CGAGTGGAAT TTCCGCTTCA CTAGTCTGGT GTGGCTAGTC GGTTTCGTGG 60

TGGCCAACAT TACGAACTTC CAACTCAACC GTTCTTGGAC GTTCAAGCGG GAGTACCGGC 120

GAGGATGGTG GCGTGAATTC TGGCCTTTCT TTGCCGTGGG ATCGGTAGCC GCCATCATCG 180

GTATGTTTAT CAAGATCTTC TTTACTAACC CGACCTCTCC GATTTACCTG CCCGAGCCGT 240

GGTTTAACGA GGGGAGGGGG ATCCAGTCAC GCGAGTACTG GTCCCAGATC TTCGCCATCG 300

TCGTGACAAT GCCTATCAAC TTCGTCGTCA ATAAGTTGTG GACCTTCCGA ACGGTGAAGC 360

ACTCCGAAAA CGTCCGGTGG CTGCTGTGCG GTGACTCCCA AAATCTTGAT AACAACAAGG 420

TAACCGAATC GCGCTAAGGA ACCCCGGCAT CTCGGGTACT CTGCATATGC GTACCCCTTA 480

AGCCGAATTC CAGCACACTG GCGGCCGTTA CTAATTGGAT CCGAACTCCG TAACCAAGCC 540

TGATGCGTAA CTTGAGTTAT TCTATAGTGT CCCTAAAATA ACCTGGCGTT A 591

(2) INFORMATION FOR SEQ ID NO:49:

(i) SEQUENCE CHARACTERISTICS:
(A) LENGTH: 454 base pairs
(B) TYPE: nucleic acid
(C) STRANDEDNESS: single
(D) TOPOLOGY: linear

(xi) SEQUENCE DESCRIPTION: SEQ ID NO:49:

AAGAGGGTAC CTGCCTTGAA ATTTAAATGT CTAAGGAAAR TGGGAGATGA TTAAGAGTTG 60

GTGTGGCYTA GTCACACCAA AATGTATTTA TTACATCCTG CTCCTTTCTA GTTGACAGGA 120

AAGAAAGCTG CTGTGGGGAA AGGAGGGATA AATACTGAAG GGATTTACTA AACAAATGTC 180

CATCACAGAG TTTTCCTTTT TTTTTTTTTG AGACAGAGTC TTGCTCTGTC ACCCAGGCTG 240

GAATGAAGWG GTATGATCTC AGTTGAATGC AACCTCTACC TCCTAGGTTC AAGCGATTCT 300

CATGCCTCAG CCTCCTGAGC AGCTGGGACT ATAGGCGCAT GCTACCATGC CAGGCTAATT 360

TTTATATTTT TATTAGAGAC GGGGTGTTGC CATGTTGGCC AGGCAGGTCT CGAACTCCTG 420

GGCCTCAGAT GATCTGCCCC ACCGTACCCT CTTA 454

(2) INFORMATION FOR SEQ ID NO:50:

(i) SEQUENCE CHARACTERISTICS:
(A) LENGTH: 463 base pairs
(B) TYPE: nucleic acid
(C) STRANDEDNESS: single
(D) TOPOLOGY: linear

(xi) SEQUENCE DESCRIPTION: SEQ ID NO:50:

AAGAGGGTAC CAAAAAAAAG AAAAAGGAAA AAAAGAAAAA CAACTTGTAT AAGGCTTTCT 60

GCTGCATACA GCTTTTTTTT TTTAAATAAA TGGTGCCAAC AAATGTTTTT GCATTCACAC 120

CAATTGCTGG TTTTGAAATC GTACTCTTCA AAGGTATTTG TGCAGATCAA TCCAATAGTG 180

ATGCCCCGTA GGTTTTGTGG ACTGCCCACG TTGTCTACCT TCTCATGTAG GAGCCATTGA 240

GAGACTGTTT GGACATGCCT GTGTTCATGT AGCCGTGATG TCCGGGGGCC GTGTACATCA 300

TGTTACCGTG GGGTGGGGTC TGCATTGGCT GCTGGGCATA TGGCTGGGTG CCCATCATGC 360

CCATCTGCAT CTGCATAGGG TATTGGGGCG TTTGATCCAT ATAGCCATGA TTGCTGTGGT 420

AGCCACTGTT CATCATTGGC TGGGACATGC TGTTACCCTC TTA 463

(2) INFORMATION FOR SEQ ID NO:51:

(i) SEQUENCE CHARACTERISTICS:
(A) LENGTH: 399 base pairs
(B) TYPE: nucleic acid
(C) STRANDEDNESS: single
(D) TOPOLOGY: linear

(xi) SEQUENCE DESCRIPTION: SEQ ID NO:51:

CTTCAACCTC CCAAAGTGCT GGGATTACAG GACTGAGCCA CCACGCTCAG CCTAAGCCTC 60

TTTTTCACTA CCCTCTAAGC GATCTACCAC AGTGATGAGG GGCTAAAGAG CAGTGCAATT 120

TGATTACAAT AATGGAACTT AGATTTATTA ATTAACAATT TTTCCTTAGC ATGTTGGTTC 180

CATAATTATT AAGAGTATGG ACTTACTTAG AAATGAGCTT TCATTTTAAG AATTTCATCT 240

TTGACCTTCT CTATTAGTCT GAGCAGTATG ACACTATACG TATTTTATTT AACTAACCTA 300

CCTTGAGCTA TTACTTTTTA AAAGGCTATA TACATGAATG TGTATTGTCA ACTGTAAAGC 360

CCCACAGTAT TTAATTATAT CATGATGTCT TTGAGGTTG 399

(2) INFORMATION FOR SEQ ID NO:52:

(i) SEQUENCE CHARACTERISTICS:
(A) LENGTH: 392 base pairs
(B) TYPE: nucleic acid
(C) STRANDEDNESS: single
(D) TOPOLOGY: linear

(xi) SEQUENCE DESCRIPTION: SEQ ID NO:52:

CTTCAACCTC AATCAACCTT GGTAATTGAT AAAATCATCA CTTAACTTTC TGATATAATG 60

GCAATAATTA TCTGAGAAAA AAAAGTGGTG AAAGATTAAA CTTGCATTTC TCTCAGAATC 120

TTGAAGGATA TTTGAATAAT TCAAAAGCGG AATCAGTAGT ATCAGCCGAA GAAACTCACT 180

TAGCTAGAAC GTTGGACCCA TGGATCTAAG TCCCTGCCCT TCCACTAACC AGCTGATTGG 240

TTTTGTGTAA ACCTCCTACA CGCTTGGGCT TGGTCGCCTC ATTTGTCAAA GTAAAGGCTG 300

AAATAGGAAG ATAATGAACC GTGTCTTTTT GGTCTCTTTT CCATCCATTA CTCTGATTTT 360

ACAAAGAGGC CTGTATTCCC CTGGTGAGGT TG 392

(2) INFORMATION FOR SEQ ID NO:53:

(i) SEQUENCE CHARACTERISTICS:
(A) LENGTH: 179 base pairs
(B) TYPE: nucleic acid
(C) STRANDEDNESS: single
(D) TOPOLOGY: linear

(xi) SEQUENCE DESCRIPTION: SEQ ID NO:53:

TTCGGGTGAT GCCTCCTCAG GCTACAGTGA AGACTGGATT ACAGAAAGGT GCCAGCGAGA 60

TTTCAGATTC CTGTAAACCT CTAAAGAAAA GGAGTCGCGC CTCAACTGAT GTAGAAATGA 120

CTAGTTCAGC ATACNGAGAC ACNTCTGACT CCGATTCTAG AGGACTGAGT GACCTGCAN 179

(2) INFORMATION FOR SEQ ID NO:54:

(i) SEQUENCE CHARACTERISTICS:
(A) LENGTH: 112 base pairs
(B) TYPE: nucleic acid
(C) STRANDEDNESS: single
(D) TOPOLOGY: linear

(xi) SEQUENCE DESCRIPTION: SEQ ID NO:54:

TTCGGGTGAT GCCTCCTCAG GCTACATCAT NATAGAAGCA AAGTAGAANA ATCNNGTTTG 60

TGCATTTTCC CACANACAAA ATTCAAATGA NTGGAAGAAA TTGGGANAGT AT 112

(2) INFORMATION FOR SEQ ID NO:55:

(i) SEQUENCE CHARACTERISTICS:
(A) LENGTH: 225 base pairs
(B) TYPE: nucleic acid
(C) STRANDEDNESS: single
(D) TOPOLOGY: linear

(xi) SEQUENCE DESCRIPTION: SEQ ID NO:55:

TGAGCTTCCG CTTCTGACAA CTCAATAGAT AATCAAAGGA CAACTTTAAC AGGGATTCAC 60

AAAGGAGTAT ATCCAAATGC CAATAAACAT ATAAAAAGGA ATTCAGCTTC ATCATCATCA 120

GAAGWATGCA AATTAAAACC ATAATGAGAA ACCACTATGT CCCACTAGAA TAGATAAAAT 180

CTTAAAAGAC TGGTAAAACC AAGTGTTGGT AAGGCAAGAG GAGCA 225

(2) INFORMATION FOR SEQ ID NO:56:

(i) SEQUENCE CHARACTERISTICS:
(A) LENGTH: 175 base pairs
(B) TYPE: nucleic acid
(C) STRANDEDNESS: single
(D) TOPOLOGY: linear

(xi) SEQUENCE DESCRIPTION: SEQ ID NO:56:

GCTCCTCTTG CCTTACCAAC ACATTCTCAA AAACCTGTTA GAGTCCTAAG CATTCTCCTG 60

TTAGTATTGG GATTTTACCC CTGTCCTATA AAGATGTTAT GTACCAAAAA TGAAGTGGAG 120

GGCCATACCC TGAGGGAGGG GAGGGATCTC TAGTGTTGTC AGAAGCGGAA GCTCA 175

(2) INFORMATION FOR SEQ ID NO:57:

(i) SEQUENCE CHARACTERISTICS:
(A) LENGTH: 223 base pairs
(B) TYPE: nucleic acid
(C) STRANDEDNESS: single
(D) TOPOLOGY: linear

(xi) SEQUENCE DESCRIPTION: SEQ ID NO:57:

AGCCATTTAC CACCCATGGA TGAATGGATT TTGTAATTCT AGCTGTTGTA TTTTGTGAAT 60

TTGTTAATTT TGTTGTTTTT CTGTGAAACA CATACATTGG ATATGGGAGG TAAAGGAGTG 120

TCCCAGTTGC TCCTGGTCAC TCCCTTTATA GCCATTACTG TCTTGTTTCT TGTAACTCAG 180

GTTAGGTTTT GGTCTCTCTT GCTCCACTGC AAAAAAAAAA AAA 223

(2) INFORMATION FOR SEQ ID NO:58:

(i) SEQUENCE CHARACTERISTICS:
(A) LENGTH: 211 base pairs
(B) TYPE: nucleic acid
(C) STRANDEDNESS: single
(D) TOPOLOGY: linear

(xi) SEQUENCE DESCRIPTION: SEQ ID NO:58:

GTTCGAAGGT GAACGTGTAG GTAGCGGATC TCACAACTGG GGAACTGTCA AAGACGAATT 60

AACTGACTTG GATCAATCAA ATGTGACTGA GGAAACACCT GAAGGTGAAG AACATCATCC 120

AGTGGCAGAC ACTGAAAATA AGGAGAATGA AGTTGAAGAG GTAAAAGAGG AGGGTCCAAA 180

AGAGATGACT TTGGATGGGT GGTAAATGGC T 211

(2) INFORMATION FOR SEQ ID NO:59:

(i) SEQUENCE CHARACTERISTICS:
(A) LENGTH: 208 base pairs
(B) TYPE: nucleic acid
(C) STRANDEDNESS: single
(D) TOPOLOGY: linear

(xi) SEQUENCE DESCRIPTION: SEQ ID NO:59:

GCTCCTCTTG CCTTACCAAC TTTGCACCCA TCATCAACCA TGTGGCCAGG TTTGCAGCCC 60

AGGCTGCACA TCAGGGGACT GCCTCGCAAT ACTTCATGCT GTTGCTGCTG ACTGATGGTG 120

CTGTGACGGA TGTGGAAGCC ACACGTGAGG CTGTGGTGCG TGCCTCGAAC CTGCCCATGT 180

CAGTGATCAT TATGGGTGGT AAATGGCT 208

(2) INFORMATION FOR SEQ ID NO:60:

(i) SEQUENCE CHARACTERISTICS:
(A) LENGTH: 171 base pairs
(B) TYPE: nucleic acid
(C) STRANDEDNESS: single
(D) TOPOLOGY: linear

(xi) SEQUENCE DESCRIPTION: SEQ ID NO:60:

AGCCATTTAC CACCCATACT AAATTCTAGT TCAAACTCCA ACTTCTTCCA TAAAACATCT 60

AACCACTGAC ACCAGTTGGC AATAGCTTCT TCCTTCTTTA ACCTCTTAGA GTATTTATGG 120

TCAATGCCAC ACATTTCTGC AACTGAATAA AGTTGGTAAG GCAAGAGGAG C 171

(2) INFORMATION FOR SEQ ID NO:61:

(i) SEQUENCE CHARACTERISTICS:
(A) LENGTH: 134 base pairs
(B) TYPE: nucleic acid
(C) STRANDEDNESS: single
(D) TOPOLOGY: linear

(xi) SEQUENCE DESCRIPTION: SEQ ID NO:61:

CGGGTGATGC CTCCTCAGGC TTTGGTGTGT CCACTCNACT CACTGGCCTC TTCTCCAGCA 60

ACTGGTGAAN ATGTCCTCAN GAAAANCNCC ACACGCNGCT CAGGGTGGGG TGGGAANCAT 120

CANAATCATC NGGC 134

(2) INFORMATION FOR SEQ ID NO:62:

(i) SEQUENCE CHARACTERISTICS:
(A) LENGTH: 145 base pairs
(B) TYPE: nucleic acid
(C) STRANDEDNESS: single
(D) TOPOLOGY: linear

(xi) SEQUENCE DESCRIPTION: SEQ ID NO:62:

AGAGGGTACA TATGCAACAG TATATAAAGG AAGAAGTGCA CTGAGAGGAA CTTCATCAAG 60

GCCATTTAAT CAATAAGTGA TAGAGTCAAG GCTCAACCCA GGTGTGACGG ATTCCAGGTC 120

CCAAGCTCCT TACTGGTACC CTCTT 145

(2) INFORMATION FOR SEQ ID NO:63:

(i) SEQUENCE CHARACTERISTICS:
(A) LENGTH: 297 base pairs
(B) TYPE: nucleic acid
(C) STRANDEDNESS: single
(D) TOPOLOGY: linear

(xi) SEQUENCE DESCRIPTION: SEQ ID NO:63:

TGCACTGAGA GGAATTCAAA GGGTTTATGC CAAAGAACAA ACCAGTCCTC TGCAGCCTAA 60

CTCATTTGTT TTTGGGCTGC GAAGCCATGT AGAGGGCGAT CAGGCAGTAG ATGGTCCCTC 120

CCACAGTCAG CGCCATGGTG GTCCGGTAAA GCATTTGGTC AGGCAGGCCT CGTTTCAGGT 180

AGACGGGCAC ACATCAGCTT TCTGGAAAAA CTTTTGTAGC TCTGGAGCTT TGTTTTTCCC 240

AGCATAATCA TACACTGTGG AATCGGAGGT CAGTTTAGTT GGTAAGGCAA GAGGAGC 297

(2) INFORMATION FOR SEQ ID NO:64:

(i) SEQUENCE CHARACTERISTICS:
(A) LENGTH: 300 base pairs
(B) TYPE: nucleic acid
(C) STRANDEDNESS: single
(D) TOPOLOGY: linear

(xi) SEQUENCE DESCRIPTION: SEQ ID NO:64:

GCACTGAGAG GAACTTCCAA TACTATGTTG AATAGGAGTG GTGAGAGAGG GCATCCTTGT 60

CTTGTGCCGG TTTTCAAAGG GAATGCTTCC AGCTTTTGCC CATTCAGTAT AATATTAAAG 120

AATGTTTTAC CATTTTCTGT CTTGCCTGTT TTTCTGTGTT TTTGTTGGTC TCTTCATTCT 180

CCATTTTTAG GCCTTTACAT GTTAGGAATA TATTTCTTTT AATGATACTT CACCTTTGGT 240

ATCTTTTGTG AGACTCTACT CATAGTGTGA TAAGCACTGG GTTGGTAAGG CAAGAGGAGC 300

(2) INFORMATION FOR SEQ ID NO:65:

(i) SEQUENCE CHARACTERISTICS:
(A) LENGTH: 203 base pairs
(B) TYPE: nucleic acid
(C) STRANDEDNESS: single
(D) TOPOLOGY: linear

(xi) SEQUENCE DESCRIPTION: SEQ ID NO:65:

GCTCCTCTTG CCTTACCAAC TCACCCAGTA TGTCAGCAAT TTTATCRGCT TTACCTACGA 60

AACAGCCTGT ATCCAAACAC TTAACACACT CACCTGAAAA GTTCAGGCAA CAATCGCCTT 120

CTCATGGGTC TCTCTGCTCC AGTTCTGAAC CTTTCTCTTT TCCTAGAACA TGCATTTARG 180

TCGATAGAAG TTCCTCTCAG TGC 203

(2) INFORMATION FOR SEQ ID NO:66:

(i) SEQUENCE CHARACTERISTICS:
(A) LENGTH: 344 base pairs
(B) TYPE: nucleic acid
(C) STRANDEDNESS: single
(D) TOPOLOGY: linear

(xi) SEQUENCE DESCRIPTION: SEQ ID NO:66:

TACGGGGACC CCTGCATTGA GAAAGCGAGA CTCACTCTGA AGCTGAAATG CTGTTGCCCT 60

TGCAGTGCTG GTAGCAGGAG TTCTGTGCTT TGTGGGCTAA GGCTCCTGGA TGACCCCTGA 120

CATGGAGAAG GCAGAGTTGT GTGCCCCTTC TCATGGCCTC GTCAAGGCAT CATGGACTGC 180

CACACACAAA ATGCCGTTTT TATTAACGAC ATGAAATTGA AGGAGAGAAC ACAATTCACT 240

GATGTGGCTC GTAACCATGG ATATGGTCAC ATACAGAGGT GTGATTATGT AAAGGTTAAT 300

TCCACCCACC TCATGTGGAA ACTAGCCTCA ATGCAGGGGT CCCA 344

(2) INFORMATION FOR SEQ ID NO:67:

(i) SEQUENCE CHARACTERISTICS:
(A) LENGTH: 157 base pairs
(B) TYPE: nucleic acid
(C) STRANDEDNESS: single
(D) TOPOLOGY: linear

(xi) SEQUENCE DESCRIPTION: SEQ ID NO:67:

GCACTGAGAG GAACTTCGTA GGGAGGTTGA ACTGGCTGCT GAGGAGGGGG AACAACAGGG 60

TAACCAGACT GATAGCCATT GGATGGATAA TATGGTGGTT GAGGAGGGAC ACTACTTATA 120

GCAGAGGGTT GTGTATAGCC TGAGGAGGCA TCACCCG 157

(2) INFORMATION FOR SEQ ID NO:68:

(i) SEQUENCE CHARACTERISTICS:
(A) LENGTH: 137 base pairs
(B) TYPE: nucleic acid

(C) STRANDEDNESS: single
(D) TOPOLOGY: linear

(xi) SEQUENCE DESCRIPTION: SEQ ID NO:68:

GCACTGAGAG GAACTTCTAG AAAGTGAAAG TCTAGACATA AAATAAAATA AAAATTTAAA 60

ACTCAGGAGA GACAGCCCAG CACGGTGGCT CACGCCTGTA ATCCCAGAAC TTTGGGAGCC 120

TGAGGAGGCA TCACCCG 137

(2) INFORMATION FOR SEQ ID NO:69:

(i) SEQUENCE CHARACTERISTICS:
(A) LENGTH: 137 base pairs
(B) TYPE: nucleic acid
(C) STRANDEDNESS: single
(D) TOPOLOGY: linear

(xi) SEQUENCE DESCRIPTION: SEQ ID NO:69:

CGGGTGATGC CTCCTCAGGC TGTATTTTGA AGACTATCGA CTGGACTTCT TATCAACTGA 60

AGAATCCGTT AAAAATACCA GTTGTATTAT TTCTACCTGT CAAAATCCAT TTCAAATGTT 120

GAAGTTCCTC TCAGTGC 137

(2) INFORMATION FOR SEQ ID NO:70:

(i) SEQUENCE CHARACTERISTICS:
(A) LENGTH: 220 base pairs
(B) TYPE: nucleic acid
(C) STRANDEDNESS: single
(D) TOPOLOGY: linear

(xi) SEQUENCE DESCRIPTION: SEQ ID NO:70:

AGCATGTTGA GCCCAGACAC GCAATCTGAA TGAGTGTGCA CCTCAAGTAA ATGTCTACAC 60

GCTGCCTGGT CTGACATGGC ACACCATCNC GTGGAGGGCA CASCTCTGCT CNGCCTACWA 120

CGAGGGCANT CTCATWGACA GGTTCCACCC ACCAAACTGC AAGAGGCTCA NNAAGTACTR 180

CCAGGGTMYA SGGACMASGG TGGGAYTYCA YCACWCATCT 220

(2) INFORMATION FOR SEQ ID NO:71:

(i) SEQUENCE CHARACTERISTICS:
(A) LENGTH: 353 base pairs
(B) TYPE: nucleic acid
(C) STRANDEDNESS: single
(D) TOPOLOGY: linear

(xi) SEQUENCE DESCRIPTION: SEQ ID NO:71:

CGTTAGGGTC TCTATCCACT GCTAAACCAT ACACCTGGGT AAACAGGGAC CATTTAACAT 60

TCCCANCTAA ATATGCCAAG TGACTTCACA TGTTTATCTT AAAGATGTCC AAAACGCAAC 120

TGATTTTCTC CCCTAAACCT GTGATGGTGG GATGATTAAN CCTGAGTGGT CTACAGCAAG 180

TTAAGTGCAA GGTGCTAAAT GAANGTGACC TGAGATACAG CATCTACAAG GCAGTACCTC 240

TCAACNCAGG GCAACTTTGC TTCTCANAGG GCATTTAGCA GTGTCTGAAG TAATTTCTGT 300

ATTACAACTC ACGGGGCGGG GGGTGAATAT CTANTGGANA GNAGACCCTA ACG 353

(2) INFORMATION FOR SEQ ID NO:72:

(i) SEQUENCE CHARACTERISTICS:
(A) LENGTH: 343 base pairs
(B) TYPE: nucleic acid
(C) STRANDEDNESS: single
(D) TOPOLOGY: linear

(xi) SEQUENCE DESCRIPTION: SEQ ID NO:72:

GCACTGAGAG GAACTTCCAA TACYATKATC AGAGTGAACA RGCARCCYAC AGAACAGGAG 60

AAAATGTTYG CAATCTCTCC ATCTGACAAA AGGCTAATAT CCAGAWTCTA AWAGGAACTT 120

AAACAAATTT ATGAGAAAAG AACARACAAC CTCAWCAAAA AGTGGGTGAA GGAWATGCTS 180

AAARGAAGAC ATYTATTCAG CCAGTAAACA YATGAAAAAA AGGCTCATSA TCACTGAWCA 240

TTAGAGAAAT GCAAATCAAA ACCACAATGA GATACCATCT YAYRCCAGTT AGAAYGGTGA 300

TCATTAAAAR STCAGGAAAC AACAGATGCT GGACAAGGTG TCA 343

(2) INFORMATION FOR SEQ ID NO:73:

(i) SEQUENCE CHARACTERISTICS:
(A) LENGTH: 321 base pairs
(B) TYPE: nucleic acid
(C) STRANDEDNESS: single
(D) TOPOLOGY: linear

(xi) SEQUENCE DESCRIPTION: SEQ ID NO:73:

GCACTGAGAG GAACTTCAGA GAGAGAGAGA GAGTTCCACC CTGTACTTGG GGAGAGAAAC 60

AGAAGGTGAG AAAGTCTTTG GTTCTGAAGC AGCTTCTAAG ATCTTTTCAT TTGCTTCATT 120

TCAAAGTTCC CATGCTGCCA AAGTGCCATC CTTTGGGGTA CTGTTTTCTG AGCTCCAGTG 180

ATAACTCATT TATACAAGGG AGATACCCAG AAAAAAAGTG AGCAAATCTT AAAAAGGTGG 240

CTTGAGTTCA GCCTTAAATA CCATCTTGAA ATGACACAGA GAAAGAANGA TGTTGGGTGG 300

GAGTGGATAG AGACCCTAAC G 321

(2) INFORMATION FOR SEQ ID NO:74:

(i) SEQUENCE CHARACTERISTICS:
(A) LENGTH: 321 base pairs
(B) TYPE: nucleic acid
(C) STRANDEDNESS: single
(D) TOPOLOGY: linear

(xi) SEQUENCE DESCRIPTION: SEQ ID NO:74:

GCACTGAGAG GAACTTCAGA GAGAGAGAGA GAGTTCCACC CTGTACTTGG GGAGAGAAAC 60

AGAAGGTGAG AAAGTCTTTG GTTCTGAAGC AGCTTCTAAG ATCTTTTCAT TTGCTTCATT 120

TCAAAGTTCC CATGCTGCCA AAGTGCCATC CTTTGGGGTA CTGTTTTCTG AGCTCCAGTG 180

ATAACTCATT TATACAAGGG AGATACCCAG AAAAAAAGTG AGCAAATCTT AAAAAGGTGG 240

CTTGAGTTCA GYCTTAAATA CCATCTTGAA ATGAMACAGA GAAAGAAGGA TGTTGGGTGG 300

GAGTGGATAG AGACCCTAAC G 321

(2) INFORMATION FOR SEQ ID NO:75:

(i) SEQUENCE CHARACTERISTICS:
(A) LENGTH: 317 base pairs

(B) TYPE: nucleic acid
(C) STRANDEDNESS: single
(D) TOPOLOGY: linear

(xi) SEQUENCE DESCRIPTION: SEQ ID NO:75:

GCACTGAGAG GAACTTCCAC ATGCACTGAG AAATGCATGT TCACAAGGAC TGAAGTCTGG 60

AACTCAGTTT CTCAGTTCCA ATCCTGATTC AGGTGTTTAC CAGCTACACA ACCTTAAGCA 120

AGTCAGATAA CCTTAGCTTC CTCATATGCA AAATGAGAAT GAAAAGTACT CATCGCTGAA 180

TTGTTTTGAG GATTAGAAAA ACATCTGGCA TGCAGTAGAA ATTCAATTAG TATTCATTTT 240

CATTCTTCTA AATTAAACAA ATAGGATTTT TAGTGGTGGA ACTTCAGACA CCAGAAATGG 300

GAGTGGATAG AGACCCT 317

(2) INFORMATION FOR SEQ ID NO:76:

(i) SEQUENCE CHARACTERISTICS:
(A) LENGTH: 244 base pairs
(B) TYPE: nucleic acid
(C) STRANDEDNESS: single
(D) TOPOLOGY: linear

(xi) SEQUENCE DESCRIPTION: SEQ ID NO:76:

CGTTAGGGTC TCTATCCACT CCCACTACTG ATCAAACTCT ATTTATTTAA TTATTTTTAT 60

CATACTTTAA GTTCTGGGAT ACACGTGCAG CATGCGCAGG TTTGTTGCAT AGGTATACAC 120

TTGCCATGGT GGTTTGCTGC ACCCATCAGT CCATCATCTA CATTAGGTAT TTCTCCTAAT 180

GCTATCCCTC CCCTAGCCCC TTACACCCCC AACAGGCTCT AGTGTGTGAA GTTCCTCTCA 240

GTGC 244

(2) INFORMATION FOR SEQ ID NO:77:

(i) SEQUENCE CHARACTERISTICS:
(A) LENGTH: 254 base pairs
(B) TYPE: nucleic acid
(C) STRANDEDNESS: single
(D) TOPOLOGY: linear

(xi) SEQUENCE DESCRIPTION: SEQ ID NO:77:

CGTTAGGGTC TCTATCCACT GAAATCTGAA GCACAGGAGG AAGAGAAGCA GTYCTAGTGA 60

GATGGCAAGT TCWTTTACCA CACTCTTTAA CATTTYGTTT AGTTTTAACC TTTATTTATG 120

GATAATAAAG GTTAATATTA ATAATGATTT ATTTTAAGGC ATTCCCRAAT TTGCATAATT 180

CTCCTTTTGG AGATACCCTT TTATCTCCAG TGCAAGTCTG GATCAAAGTG ATASAMAGAA 240

GTTCCTCTCA GTGC 254

(2) INFORMATION FOR SEQ ID NO:78:

(i) SEQUENCE CHARACTERISTICS:
(A) LENGTH: 355 base pairs
(B) TYPE: nucleic acid
(C) STRANDEDNESS: single
(D) TOPOLOGY: linear

(xi) SEQUENCE DESCRIPTION: SEQ ID NO:78:

TTCGATACAG GCAAACATGA ACTGCAGGAG GGTGGTGACG ATCATGATGT TGCCGATGGT 60

CCGGATGGNC ACGAAGACGC ACTGGANCAC GTGCTTACGT CCTTTTGCTC TGTTGATGGC 120

CCTGAGGGGA CGCAGGACCC TTATGACCCT CAGAATCTTC ACAACGGGAG ATGGCACTGG 180

ATTGANTCCC ANTGACACCA GAGACACCCC AACCACCAGN ATATCANTAT ATTGATGTAG 240

TTCCTGTAGA NGGCCCCCTT GTGGAGGAAA GCTCCATNAG TTGGTCATCT TCAACAGGAT 300

CTCAACAGTT TCCGATGGCT GTGATGGGCA TAGTCATANT TAACCNTGTN TCGAA 355

(2) INFORMATION FOR SEQ ID NO:79:

(i) SEQUENCE CHARACTERISTICS:
(A) LENGTH: 406 base pairs
(B) TYPE: nucleic acid
(C) STRANDEDNESS: single
(D) TOPOLOGY: linear

(xi) SEQUENCE DESCRIPTION: SEQ ID NO:79:

TAAGAGGGTA CCAGCAGAAA GGTTAGTATC ATCAGATAGC ATCTTATACG AGTAATATGC 60

CTGCTATTTG AAGTGTAATT GAGAAGGAAA ATTTTAGCGT GCTCACTGAC CTGCCTGTAG 120

CCCCAGTGAC AGCTAGGATG TGCATTCTCC AGCCATCAAG AGACTGAGTC AAGTTGTTCC 180

TTAAGTCAGA ACAGCAGACT CAGCTCTGAC ATTCTGATTC GAATGACACT GTTCAGGAAT 240

CGGAATCCTG TCGATTAGAC TGGACAGCTT GTGGCAAGTG AATTTGCCTG TAACAAGCCA 300

GATTTTTTAA AATTTATATT GTAAATAATG TGTGTGTGTG TGTGTGTATA TATATATATA 360

TGTACAGTTA TCTAAGTTAA TTTAAAAGTT GTTTGGTACC CTCTTA 406

(2) INFORMATION FOR SEQ ID NO:80:

(i) SEQUENCE CHARACTERISTICS:
(A) LENGTH: 327 base pairs
(B) TYPE: nucleic acid
(C) STRANDEDNESS: single
(D) TOPOLOGY: linear

(xi) SEQUENCE DESCRIPTION: SEQ ID NO:80:

TTTTTTTTTT TTTACTCGGC TCAGTCTAAT CCTTTTTGTA GTCACTCATA GGCCAGACTT 60

AGGGCTAGGA TGATGATTAA TAAGAGGGAT GACATAACTA TTAGTGGCAG GTTAGTTGTT 120

TGTAGGGCTC ATGGTAGGGG TAAAAGGAGG GCAATTTCTA GATCAAATAA TAAGAAGGTA 180

ATAGCTACTA AGAAGAATTT TATGGAGAAA GGGACGCGGG CGGGGGATAT AGGGTCGAAG 240

CCGCACTCGT AAGGGGTGGA TTTTTCTATG TAGCCGTTGA GTTGTGGTAG TCAAAATGTA 300

ATAATTATTA GTAGTAAGCC TAGGAGA 327

(2) INFORMATION FOR SEQ ID NO:81:

(i) SEQUENCE CHARACTERISTICS:
(A) LENGTH: 318 base pairs
(B) TYPE: nucleic acid
(C) STRANDEDNESS: single
(D) TOPOLOGY: linear

(xi) SEQUENCE DESCRIPTION: SEQ ID NO:81:

TAGTCTATGC GGTTGATTCG GCAATCCATT ATTTGCTGGA TTTTGTCATG TGTTTTGCCA 60

ATTGCATTCA TAATTTATTA TGCATTTATG CTTGTATCTC CTAAGTCATG GTATATAATC 120

CATGCTTTTT ATGTTTTGTC TGACATAAAC TCTTATCAGA GCCCTTTGCA CACAGGGATT 180

CAATAAATAT TAACACAGTC TACATTTATT TGGTGAATAT TGCATATCTG CTGTACTGAA 240

AGCACATTAA GTAACAAAGG CAAGTGAGAA GAATGAAAAG CACTACTCAC AACAGTTATC 300

ATGATTGCGC ATAGACTA 318

(2) INFORMATION FOR SEQ ID NO:82:

(i) SEQUENCE CHARACTERISTICS:
(A) LENGTH: 338 base pairs
(B) TYPE: nucleic acid
(C) STRANDEDNESS: single
(D) TOPOLOGY: linear

(xi) SEQUENCE DESCRIPTION: SEQ ID NO:82:

TCTTCAACCT CTACTCCCAC TAATAGCTTT TTGATGACTT CTAGCAAGCC TCGCTAACCT 60

CGCCTTACCC CCCACTATTA ACCTACTGGG AGAACTCTCT GTGCTAGTAA CCACGTTCTC 120

CTGATCAAAT ATCACTCTCC TACTTACAGG ACTCAACATA CTAGTCACAG CCCTATACTC 180

CCTCTACATA TTTACCACAA CACAATGGGG CTCACTCACC CACCACATTA ACAACATAAA 240

ACCCTCATTC ACACGAGAAA ACACCCTCAT GTTCATACAC CTATCCCCCA TTCTCCTCCT 300

ATCCCTCAAC CCCGACATCA TTACCGGGTT TTCCTCTT 338

(2) INFORMATION FOR SEQ ID NO:83:

(i) SEQUENCE CHARACTERISTICS:
(A) LENGTH: 111 base pairs
(B) TYPE: nucleic acid
(C) STRANDEDNESS: single
(D) TOPOLOGY: linear

(xi) SEQUENCE DESCRIPTION: SEQ ID NO:83:

AGCCATTTAC CACCCATCCA CAAAAAAAAA AAAAAAAAAG AAAAATATCA AGGAATAAAA 60

ATAGACTTTG AACAAAAAGG AACATTTGCT GGCCTGAGGA GGCATCACCC G 111

(2) INFORMATION FOR SEQ ID NO:84:

(i) SEQUENCE CHARACTERISTICS:
(A) LENGTH: 224 base pairs
(B) TYPE: nucleic acid
(C) STRANDEDNESS: single
(D) TOPOLOGY: linear

(xi) SEQUENCE DESCRIPTION: SEQ ID NO:84:

TCGGGTGATG CCTCCTCAGG CCAAGAAGAT AAAGCTTCAG ACCCCTAACA CATTTCCAAA 60

AAGGAAGAAA GGAGAAAAAA GGGCATCATC CCCGTTCCGA AGGGTCAGGG AGGAGGAAAT 120

TGAGGTGGAT TCACGAGTTG CGGACAACTC CTTTGATGCC AAGCGAGGTG CAGCCGGAGA 180

CTGGGGAGAG CGAGCCAATC AGGTTTTGAA GTTCCTCTCA GTGC 224

(2) INFORMATION FOR SEQ ID NO:85:

(i) SEQUENCE CHARACTERISTICS:
(A) LENGTH: 348 base pairs

(B) TYPE: nucleic acid
(C) STRANDEDNESS: single
(D) TOPOLOGY: linear

(xi) SEQUENCE DESCRIPTION: SEQ ID NO:85:
   GCACTGAGAG GAACTTCGTT GGAAACGGGT TTTTTTCATG TAAGGCTAGA CAGAAGAATT 60
   CTCAGTAACT TCCTTGTGTT GTGTGTATTC AACTCACASA GTTGAACGAT CCTTTACACA 120
   GAGCAGACTT GTAACACTCT TWTTGTGGAA TTTGCAAGTG GAGATTTCAG SCGCTTTGAA 180
   GTSAAAGGTA GAAAAGGAAA TATCTTCCTA TAAAAACTAG ACAGAATGAT TCTCAGAAAC 240
   TCCTTTGTGA TGTGTGCGTT CAACTCACAG AGTTTAACCT TTCWTTTCAT AGAAGCAGTT 300
   AGGAAACACT CTGTTTGTAA AGTCTGCAAG TGGATAGAGA CCCTAACG 348

(2) INFORMATION FOR SEQ ID NO:86:

(i) SEQUENCE CHARACTERISTICS:
(A) LENGTH: 293 base pairs
(B) TYPE: nucleic acid
(C) STRANDEDNESS: single
(D) TOPOLOGY: linear

(xi) SEQUENCE DESCRIPTION: SEQ ID NO:86:

GCACTGAGAG GAACTTCYTT GTGWTGTKTG YATTCAACTC ACAGAGTTGA ASSWTSMTTT 60

ACABAGWKCA GGCTTKCAAA CACTCTTTTT GTMGAATYTG CAAGWGGAKA TTTSRRCCRC 120

TTTGWGGYCW WYSKTMGAAW MGGRWATATC TTCWYATMRA AMCTAGACAG AAKSATTCTC 180

AKAAWSTYYY YTGTGAWGWS TGCRTTCAAC TCACAGAGKT KAACMWTYCT KYTSATRGAG 240

CAGTTWKGAA ACTCTMTTTC TTTGGATTCT GCAAGTGGAT AGAGACCCTA ACG 293

(2) INFORMATION FOR SEQ ID NO:87:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:87:

CTCCTAGGCT 10

(2) INFORMATION FOR SEQ ID NO:88:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:88:

AGTAGTTGCC 10

(2) INFORMATION FOR SEQ ID NO:89:

(i) SEQUENCE CHARACTERISTICS:
(A) LENGTH: 11 base pairs
(B) TYPE: nucleic acid
(C) STRANDEDNESS: single

(D) TOPOLOGY: linear

(xi) SEQUENCE DESCRIPTION: SEQ ID NO:89:

TTCCGTTATG C 11

(2) INFORMATION FOR SEQ ID NO:90:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:90:

TGGTAAAGGG 10

(2) INFORMATION FOR SEQ ID NO:91:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:91:

TCGGTCATAG 10

(2) INFORMATION FOR SEQ ID NO:92:

(i) SEQUENCE CHARACTERISTICS:

(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:92:

TACAACGAGG 10

(2) INFORMATION FOR SEQ ID NO:93:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:93:

TGGATTGGTC 10

(2) INFORMATION FOR SEQ ID NO:94:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:94:

CTTTCTACCC 10

(2) INFORMATION FOR SEQ ID NO:95:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:95:

TTTTGGCTCC 10

(2) INFORMATION FOR SEQ ID NO:96:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:96:

GGAACCAATC 10

(2) INFORMATION FOR SEQ ID NO:97:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:97:

TCGATACAGG 10

(2) INFORMATION FOR SEQ ID NO:98:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:98:

GGTACTAAGG 10

(2) INFORMATION FOR SEQ ID NO:99:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:99:

AGTCTATGCG 10

(2) INFORMATION FOR SEQ ID NO:100:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:100:

CTATCCATGG 10

(2) INFORMATION FOR SEQ ID NO:101:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:101:

TCTGTCCACA 10

(2) INFORMATION FOR SEQ ID NO:102:

(i) SEQUENCE CHARACTERISTICS:
(A) LENGTH: 10 base pairs
(B) TYPE: nucleic acid
(C) STRANDEDNESS: single
(D) TOPOLOGY: linear

(xi) SEQUENCE DESCRIPTION: SEQ ID NO:102:

AAGAGGGTAC 10

(2) INFORMATION FOR SEQ ID NO:103:

(i) SEQUENCE CHARACTERISTICS:
(A) LENGTH: 10 base pairs

(B) TYPE: nucleic acid
(C) STRANDEDNESS: single
(D) TOPOLOGY: linear

(xi) SEQUENCE DESCRIPTION: SEQ ID NO:103:

CTTCAACCTC 10

(2) INFORMATION FOR SEQ ID NO:104:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:104:

GCTCCTCTTG CCTTACCAAC 20

(2) INFORMATION FOR SEQ ID NO:105:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:105:

GTAAGTCGAG CAGTGTGATG 20

(2) INFORMATION FOR SEQ ID NO:106:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:106:

GTAAGTCGAG CAGTCTGATG 20

(2) INFORMATION FOR SEQ ID NO:107:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:107:

GACTTAGTGG AAAGAATGTA 20

(2) INFORMATION FOR SEQ ID NO:108:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:108:

GTAATTCCGC CAACCGTAGT 20

(2) INFORMATION FOR SEQ ID NO:109:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:109:

ATGGTTGATC GATAGTGGAA 20

(2) INFORMATION FOR SEQ ID NO:110:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:110:

ACGGGGACCC CTGCATTGAG 20

(2) INFORMATION FOR SEQ ID NO:111:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:111:

TATTCTAGAC CATTCGCTAC 20

(2) INFORMATION FOR SEQ ID NO:112:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:112:

ACATAACCAC TTTAGCGTTC 20

(2) INFORMATION FOR SEQ ID NO:113:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:113:

CGGGTGATGC CTCCTCAGGC 20

(2) INFORMATION FOR SEQ ID NO:114:

(i) SEQUENCE CHARACTERISTICS:
(A) LENGTH: 20 base pairs
(B) TYPE: nucleic acid

(C) STRANDEDNESS: single
(D) TOPOLOGY: linear

(xi) SEQUENCE DESCRIPTION: SEQ ID NO:114:

AGCATGTTGA GCCCAGACAC 20

(2) INFORMATION FOR SEQ ID NO:115:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:115:

GACACCTTGT CCAGCATCTG 20

(2) INFORMATION FOR SEQ ID NO:116:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:116:

TACGCTGCAA CACTGTGGAG 20

(2) INFORMATION FOR SEQ ID NO:117:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:117:

CGTTAGGGTC TCTATCCACT 20

(2) INFORMATION FOR SEQ ID NO:118:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:118:

AGACTGACTC ATGTCCCCTA 20

(2) INFORMATION FOR SEQ ID NO:119:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:119:

TCATCGCTCG GTGACTCAAG 20

(2) INFORMATION FOR SEQ ID NO:120:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:120:

CAAGATTCCA TAGGCTGACC 20

(2) INFORMATION FOR SEQ ID NO:121:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:121:

ACGTACTGGT CTTGAAGGTC 20

(2) INFORMATION FOR SEQ ID NO:122:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:122:

GACGCTTGGC CACTTGACAC 20

(2) INFORMATION FOR SEQ ID NO:123:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:123:

GTATCGACGT AGTGGTCTCC 20

(2) INFORMATION FOR SEQ ID NO:124:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:124:

TAGTGACATT ACGACGCTGG 20

(2) INFORMATION FOR SEQ ID NO:125:

(i) SEQUENCE CHARACTERISTICS:
(A) LENGTH: 20 base pairs
(B) TYPE: nucleic acid
(C) STRANDEDNESS: single

(D) TOPOLOGY: linear

(xi) SEQUENCE DESCRIPTION: SEQ ID NO:125:

CGGGTGATGC CTCCTCAGGC 20

(2) INFORMATION FOR SEQ ID NO:126:

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:126:

ATGGCTATTT TCGGGGGCTG ACA 23

(2) INFORMATION FOR SEQ ID NO:127:

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:127:

CCGGTATCTC CTCGTGGGTA TT 22

(2) INFORMATION FOR SEQ ID NO:128:

(i) SEQUENCE CHARACTERISTICS:

(A) LENGTH: 18 base pairs
(B) TYPE: nucleic acid
(C) STRANDEDNESS: single
(D) TOPOLOGY: linear

(xi) SEQUENCE DESCRIPTION: SEQ ID NO:128:

CTGCCTGAGC CACAAATG 18

(2) INFORMATION FOR SEQ ID NO:129:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:129:

CCGGAGGAGG AAGCTAGAGG AATA 24

(2) INFORMATION FOR SEQ ID NO:130:

(i) SEQUENCE CHARACTERISTICS:
(A) LENGTH: 14 base pairs
(B) TYPE: nucleic acid
(C) STRANDEDNESS: single
(D) TOPOLOGY: linear

(xi) SEQUENCE DESCRIPTION: SEQ ID NO:130:

TTTTTTTTTT TTAG 14

(2) INFORMATION FOR SEQ ID NO:131:

(i) SEQUENCE CHARACTERISTICS:
(A) LENGTH: 18 amino acids
(B) TYPE: amino acid
(C) STRANDEDNESS: single
(D) TOPOLOGY: linear

(xi) SEQUENCE DESCRIPTION: SEQ ID NO:131:

Ser Ser Gly Gly Arg Thr Phe Asp Asp Phe His Arg Tyr Leu Leu Val
1               5                   10                  15

Gly Ile

(2) INFORMATION FOR SEQ ID NO:132:

(i) SEQUENCE CHARACTERISTICS:
(A) LENGTH: 22 amino acids
(B) TYPE: amino acid
(C) STRANDEDNESS: single
(D) TOPOLOGY: linear

(xi) SEQUENCE DESCRIPTION: SEQ ID NO:132:

Gln Gly Ala Ala Gln Lys Pro Ile Asn Leu Ser Lys Xaa Ile Glu Val
1               5                   10                  15

Val Gln Gly His Asp Glu
            20

(2) INFORMATION FOR SEQ ID NO:133:

(i) SEQUENCE CHARACTERISTICS:
(A) LENGTH: 23 amino acids
(B) TYPE: amino acid
(C) STRANDEDNESS: single
(D) TOPOLOGY: linear

(xi) SEQUENCE DESCRIPTION: SEQ ID NO:133:

Ser Pro Gly Val Phe Leu Glu His Leu Gln Glu Ala Tyr Arg Ile Tyr
1               5                   10                  15

Thr Pro Phe Asp Leu Ser Ala
            20

(2) INFORMATION FOR SEQ ID NO:134:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:134:

Tyr Leu Leu Val Gly Ile Gln Gly Ala
1               5

(2) INFORMATION FOR SEQ ID NO:135:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:135:

Gly Ala Ala Gln Lys Pro Ile Asn Leu
1               5

(2) INFORMATION FOR SEQ ID NO:136:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:136:

Asn Leu Ser Lys Xaa Ile Glu Val Val
1               5

(2) INFORMATION FOR SEQ ID NO:137:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:137:

Glu Val Val Gln Gly His Asp Glu Ser
1               5

(2) INFORMATION FOR SEQ ID NO:138:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:138:

His Leu Gln Glu Ala Tyr Arg Ile Tyr
1               5

(2) INFORMATION FOR SEQ ID NO:139:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:139:

Asn Leu Ala Phe Val Ala Gln Ala Ala
1               5

(2) INFORMATION FOR SEQ ID NO:140:

(i) SEQUENCE CHARACTERISTICS:
(A) LENGTH: 9 amino acids
(B) TYPE: amino acid
(C) STRANDEDNESS: single
(D) TOPOLOGY: linear

(xi) SEQUENCE DESCRIPTION: SEQ ID NO:140:

Phe Val Ala Gln Ala Ala Pro Asp Ser
1               5

(2) INFORMATION FOR SEQ ID NO:141:

(i) SEQUENCE CHARACTERISTICS:
(A) LENGTH: 9388 base pairs
(B) TYPE: nucleic acid
(C) STRANDEDNESS: single
(D) TOPOLOGY: linear

(xi) SEQUENCE DESCRIPTION: SEQ ID NO:141:

GCTCGCGGCC GCGAGCTCAA TTAACCCTCA CTAAAGGGAG TCGACTCGAT CAGACTGTTA 60

CTGTGTCTAT GTAGAAAGAA GTAGACATAA GAGATTCCAT TTTGTTCTGT ACTAAGAAAA 120

ATTCTTCTGC CTTGAGATGC TGTTAATCTG TAACCCTAGC CCCAACCCTG TGCTCACAGA 180

GACATGTGCT GTGTTGACTC AAGGTTCAAT GGATTTAGGG CTATGCTTTG TTAAAAAAGT 240

GCTTGAAGAT AATATGCTTG TTAAAAGTCA TCACCATTCT CTAATCTCAA GTACCCAGGG 300

ACACAATACA CTGCGGAAGG CCGCAGGGAC CTCTGTCTAG GAAAGCCAGG TATTGTCCAA 360

GATTTCTCCC CATGTGATAG CCTGAGATAT GGCCTCATGG GAAGGGTAAG ACCTGACTGT 420

CCCCCAGCCC GACATCCCCC AGCCCGACAT CCCCCAGCCC GACACCCGAA AAGGGTCTGT 480

GCTGAGGAGG ATTAGTAAAA GAGGAAGGCC TCTTTGCAGT TGAGGTAAGA GGAAGGCATC 540

TGTCTCCTGC TCGTCCCTGG GCAATAGAAT GTCTTGGTGT AAAACCCGAT TGTATGTTCT 600

ACTTACTGAG ATAGGAGAAA ACATCCTTAG GGCTGGAGGT GAGACACGCT GGCGGCAATA 660

CTGCTCTTTA ATGCACCGAG ATGTTTGTAT AAGTGCACAT CAAGGCACAG CACCTTTCCT 720

TAAACTTATT TATGACACAG AGACCTTTGT TCACGTTTTC CTGCTGACCC TCTCCCCACT 780

ATTACCCTAT TGGCCTGCCA CATCCCCCTC TCCGAGATGG TAGAGATAAT GATCAATAAA 840

TACTGAGGGA ACTCAGAGAC CAGTGTCCCT GTAGGTCCTC CGTGTGCTGA GCGCCGGTCC 900

CTTGGGCTCA CTTTTCTTTC TCTATACTTT GTCTCTGTGT CTCTTTCTTT TCTCAGTCTC 960

TCGTTCCACC TGACGAGAAA TACCCACAGG TGTGGAGGGG CAGGCCACCC CTTCAATAAT 1020

TTACTAGCCT GTTCGCTGAC AACAAGACTG GTGGTGCAGA AGGTTGGGTC TTGGTGTTCA 1080

CCGGGTGGCA GGCATGGGCC AGGTGGGAGG GTCTCCAGCG CCTGGTGCAA ATCTCCAAGA 1140

AAGTGCAGGA AACAGCACCA AGGGTGATTG TAAATTTTGA TTTGGCGCGG CAGGTAGCCA 1200

TTCCAGCGCA AAAATGCGCA GGAAAGCTTT TGCTGTGCTT GTAGGCAGGT AGGCCCCAAG 1260

CACTTCTTAT TGGCTAATGT GGAGGGAACC TGCACATCCA TTGGCTGAAA TCTCCGTCTA 1320

TTTGAGGCTG ACTGAGCGCG TTCCTTTCTT CTGTGTTGCC TGGAAACGGA CTGTCTGCCT 1380

AGTAACATCT GATCACGTTT CCCATTGGCC GCCGTTTCCG GAAGCCCGCC CTCCCATTTC 1440

CGGAAGCCTG GCGCAAGGTT GGTCTGCAGG TGGCCTCCAG GTGCAAAGTG GGAAGTGTGA 1500

GTCCTCAGTC TTGGGCTATT CGGCCACGTG CCTGCCGGAC ATGGGACGCT GGAGGGTCAG 1560

CAGCGTGGAG TCCTGGCCTT TTGCGTCCAC GGGTGGGAAA TTGGCCATTG CCACGGCGGG 1620

AACTGGGACT CAGGCTGCCC CCCGGCCGTT TCTCATCCGT CCACCGGACT CGTGGGCGCT 1680

CGCACTGGCG CTGATGTAGT TTCCTGACCT CTGACCCGTA TTGTCTCCAG ATTAAAGGTA 1740

AAAACGGGGC TTTTTCAGCC CACTCGGGTA AAACGCCTTT TGATTTCTAG GCAGGTGTTT 1800

TGTTGCACGC CTGGGAGGGA GTGACCCGCA GGTTGAGGTT TATTAAAATA CATTCCTGGT 1860

TTATGTTATG TTTATAATAA AGCACCCCAA CCTTTACAAA ATCTCACTTT TTGCCAGTTG 1920

TATTATTTAG TGGACTGTCT CTGATAAGGA CAGCCAGTTA AAATGGAATT TTGTTGTTGC 1980

TAATTAAACC AATTTTTAGT TTTGGTGTTT GTCCTAATAG CAACAACTTC TCAGGCTTTA 2040

TAAAACCATA TTTCTTGGGG GAAATTTCTG TGTAAGGCAC AGCGAGTTAG TTTGGAATTG 2100

TTTTAAAGGA AGTAAGTTCC TGGTTTTGAT ATCTTAGTAG TGTAATGCCC AACCTGGTTT 2160

TTACTAACCC TGTTTTTAGA CTCTCCCTTT CCTTAAATCA CCTAGCCTTG TTTCCACCTG 2220

AATTGACTCT CCCTTAGCTA AGAGCGCCAG ATGGACTCCA TCTTGGCTCT TTCACTGGCA 2280

GCCCCTTCCT CAAGGACTTA ACTTGTGCAA GCTGACTCCC AGCACATCCA AGAATGCAAT 2340

TAACTGTTAA GATACTGTGG CAAGCTATAT CCGCAGTTCC GAGGAATTCA TCCGATTGAT 2400

TATGCCCAAA AGCCCCGCGT CTATCACCTT GTAATAATCT TAAAGCCCCT GCACCTGGAA 2460

CTATTAACTT TCCTGTAACC ATTTATCCTT TTAACTTTTT TGCTTACTTT ATTTCTGTAA 2520

AATTGTTTTA ACTAGACCTC CCCTCCCCTT TCTAAACCAA AGTATAAAAG AAGATCTAGC 2580

CCCTTCTTCA GAGCGGAGAG AATTTTGAGC ATTAGCCATC TCTTGGCGGC CAGCTAAATA 2640

AATGGACTTT TAATTTGTCT CAAAGTGTGG CGTTTTCTCT AACTCGCTCA GGTACGACAT 2700

TTGGAGGCCC CAGCGAGAAA CGTCACCGGG AGAAACGTCA CCGGGCGAGA GCCGGGCCCG 2760

CTGTGTGCTC CCCCGGAAGG ACAGCCAGCT TGTAGGGGGG AGTGCCACCT GAAAAAAAAA 2820

TTTCCAGGTC CCCAAAGGGT GACCGTCTTC CGGAGGACAG CGGATCGACT ACCATGCGGG 2880

TGCCCACCAA AATTCCACCT CTGAGTCCTC AACTGCTGAC CCCGGGGTCA GGTAGGTCAG 2940

ATTTGACTTT GGTTCTGGCA GAGGGAAGCG ACCCTGATGA GGGTGTCCCT CTTTTGACTC 3000

TGCCCATTTC TCTAGGATGC TAGAGGGTAG AGCCCTGGTT TTCTGTTAGA CGCCTCTGTG 3060

TCTCTGTCTG GGAGGGAAGT GGCCCTGACA GGGGCCATCC CTTGAGTCAG TCCACATCCC 3120

AGGATGCTGG GGGACTGAGT CCTGGTTTCT GGCAGACTGG TCTCTCTCTC TCTCTTTTTC 3180

TATCTCTAAT CTTTCCTTGT TCAGGTTTCT TGGAGAATCT CTGGGAAAGA AAAAAGAAAA 3240

ACTGTTATAA ACTCTGTGTG AATGGTGAAT GAATGGGGGA GGACAAGGGC TTGCGCTTGT 3300

CCTCCAGTTT GTAGCTCCAC GGCGAAAGCT ACGGAGTTCA AGTGGGCCCT CACCTGCGGT 3360

TCCGTGGCGA CCTCATAAGG CTTAAGGCAG CATCCGGCAT AGCTCGATCC GAGCCGGGGG 3420

TTTATACCGG CCTGTCAATG CTAAGAGGAG CCCAAGTCCC CTAAGGGGGA GCGGCCAGGC 3480

GGGCATCTGA CTGATCCCAT CACGGGACCC CCTCCCCTTG TTTGTCTAAA AAAAAAAAAA 3540

GAAGAAACTG TCATAACTGT TTACATGCCC TAGGGTCAAC TGTTTGTTTT ATGTTTATTG 3600

TTCTGTTCGG TGTCTATTGT CTTGTTTAGT GGTTGTCAAG GTTTTGCATG TCAGGACGTC 3660

GATATTGCCC AAGACGTCTG GGTAAGAACT TCTGCAAGGT CCTTAGTGCT GATTTTTTGT 3720

CACAGGAGGT TAAATTTCTC ATCAATCATT TAGGCTGGCC ACCACAGTCC TGTCTTTTCT 3780

GCCAGAAGCA AGTCAGGTGT TGTTACGGGA ATGAGTGTAA AAAAACATTC GCCTGATTGG 3840

GATTTCTGGC ACCATGATGG TTGTATTTAG ATTGTCATAC CCCACATCCA GGTTGATTGG 3900

ACCTCCTCTA AACTAAACTG GTGGTGGGTT CAAAACAGCC ACCCTGCAGA TTTCCTTGCT 3960

CACCTCTTTG GTCATTCTGT AACTTTTCCT GTGCCCTTAA ATAGCACACT GTGTAGGGAA 4020

ACCTACCCTC GTACTGCTTT ACTTCGTTTA GATTCTTACT CTGTTCCTCT GTGGCTACTC 4080

TCCCATCTTA AAAACGATCC AAGTGGTCCT TTTCCTCCTC CCTGCCCCCT ACCCCACACA 4140

TCTCGTTTTC CAGTGCGACA GCAAGTTCAG CGTCTCCAGG ACTTGGCTCT GCTCTCACTC 4200

CTTGAACCCT TAAAAGAAAA AGCTGGGTTT GAGCTATTTG CCTTTGAGTC ATGGAGACAC 4260

AAAAGGTATT TAGGGTACAG ATCTAGAAGA AGAGAGAGAA CACCTAGATC CAACTGACCC 4320

AGGAGATCTC GGGCTGGCCT CTAGTCCTCC TCCCTCAATC TTAAAGCTAC AGTGATGTGG 4380

CAAGTGGTAT TTAGCTGTTG TGGTTTTTCT GCTCTTTCTG GTCATGTTGA TTCTGTTCTT 4440

TCGATACTCC AGCCCCCCAG GGAGTGAGTT TCTCTGTCTG TGCTGGGTTT GATATCTATG 4500

TTCAAATCTT ATTAAATTGC CTTCAAAAAA AAAAAAAAAA GGGAAACACT TCCTCCCAGC 4560

CTTGTAAGGG TTGGAGCCCT CTCCAGTATA TGCTGCAGAA TTTTTCTCTC GGTTTCTCAG 4620

AGGATTATGG AGTCCGCCTT AAAAAAGGCA AGCTCTGGAC ACTCTGCAAA GTAGAATGGC 4680

CAAAGTTTGG AGTTGAGTGG CCCCTTGAAG GGTCACTGAA CCTCACAATT GTTCAAGCTG 4740

TGTGGCGGGT TGTTACTGAA ACTCCCGGCC TCCCTGATCA GTTTCCCTAC ATTGATCAAT 4800

GGCTGAGTTT GGTCAGGAGC ACCCCTTCCA TGGCTCCACT CATGCACCAT TCATAATTTT 4860

ACCTCCAAGG TCCTCCTGAG CCAGACCGTG TTTTCGCCTC GACCCTCAGC CGGTTCAGCT 4920

CGCCCTGTAC TGCCTCTCTC TGAAGAAGAG GAGAGTCTCC CTCACCCAGT CCCACCGCCT 4980

TAAAACCAGC CTACTCCCTT AGGGTCATCC CATGTCTCCT CGGCTATGTC CCCTGTAGGC 5040

TCATCACCCA TTGCCTCTTG GTTGCAACCG TGGTGGGAGG AAGTAGCCCC TCTACTACCA 5100

CTGAGAGAGG CACAAGTCCC TCTGGGTGAT GAGTGCTCCA CCCCCTTCCT GGTTTATGTC 5160

CCTTCTTTCT ACTTCTGACT TGTATAATTG GAAAACCCAT AATCCTCCCT TCTCTGAAAA 5220

GCCCCAGGCT TTGACCTCAC TGATGGAGTC TGTACTCTGG ACACATTGGC CCACCTGGGA 5280

TGACTGTCAA CAGCTCCTTT TGACCCTTTT CACCTCTGAA GAGAGGGAAA GTATCCAAAG 5340

AGAGGCCAAA AAGTACAACC TCACATCAAC CAATAGGCCG GAGGAGGAAG CTAGAGGAAT 5400

AGTGATTAGA GACCCAATTG GGACCTAATT GGGACCCAAA TTTCTCAAGT GGAGGGAGAA 5460

CTTTTGACGA TTTCCACCGG TATCTCCTCG TGGGTATTCA GGGAGCTGCT CAGAAACCTA 5520

TAAACTTGTC TAAGGCGACT GAAGTCGTCC AGGGGCATGA TGAGTCACCA GGAGTGTTTT 5580

TAGAGCACCT CCAGGAGGCT TATCGGATTT ACACCCCTTT TGACCTGGCA GCCCCCGAAA 5640

ATAGCCATGC TCTTAATTTG GCATTTGTGG CTCAGGCAGC CCCAGATAGT AAAAGGAAAC 5700

TCCAAAAACT AGAGGGATTT TGCTGGAATG AATACCAGTC AGCTTTTAGA GATAGCCTAA 5760

AAGGTTTTTG ACAGTCAAGA GGTTGAAAAA CAAAAACAAG CAGCTCAGGC AGCTGAAAAA 5820

AGCCACTGAT AAAGCATCCT GGAGTATCAG AGTTTACTGT TAGATCAGCC TCATTTGACT 5880

TCCCCTCCCA CATGGTGTTT AAATCCAGCT ACACTACTTC CTGACTCAAA CTCCACTATT 5940

CCTGTTCATG ACTGTCAGGA ACTGTTGGAA ACTACTGAAA CTGGCCGACC TGATCTTCAA 6000

AATGTGCCCC TAGGAAAGGT GGATGCCACC GTGTTCACAG ACAGTAGCAG CTTCCTCGAG 6060

AAGGGACTAC GAAAGGCCGG TGCAGCTGTT ACCATGGAGA CAGATGTGTT GTGGGCTCAG 6120

GCTTTACCAG CAAACACCTC AGCACAAAAG GCTGAATTGA TCGCCCTCAC TCAGGCTCTC 6180

CGATGGGGTA AGGATATTAA CGTTAACACT GACAGCAGGT ACGCCTTTGC TACTGTGCAT 6240

GTACGTGGAG CCATCTACCA GGAGCGTGGG CTACTCACCT CAGCAGGTGG CTGTAATCCA 6300

CTGTAAAGGA CATCAAAAGG AAAACACGGC TGTTGCCCGT GGTAACCAGA AAGCTGATTC 6360

AGCAGCTCAA GATGCAGTGT GACTTTCAGT CACGCCTCTA AACTTGCTGC CCACAGTCTC 6420

CTTTCCACAG CCAGATCTGC CTGACAATCC CGCATACTCA ACAGAAGAAG AAAACTGGCC 6480

TCAGAACTCA GAGCCAATAA AAATCAGGAA GGTTGGTGGA TTCTTCCTGA CTCTAGAATC 6540

TTCATACCCC GAACTCTTGG GAAAACTTTA ATCAGTCACC TACAGTCTAC CACCCATTTA 6600

GGAGGAGCAA AGCTACCTCA GCTCCTCCGG AGCCGTTTTA AGATCCCCCA TCTTCAAAGC 6660

CTAACAGATC AAGCAGCTCT CCGGTGCACA ACCTGCGCCC AGGTAAATGC CAAAAAAGGT 6720

CCTAAACCCA GCCCAGGCCA CCGTCTCCAA GAAAACTCAC CAGGAGAAAA GTGGGAAATT 6780

GACTTTACAG AAGTAAAACC ACACCGGGCT GGGTACAAAT ACCTTCTAGT ACTGGTAGAC 6840

ACCTTCTCTG GATGGACTGA AGCATTTGCT ACCAAAAACG AAACTGTCAA TATGGTAGTT 6900

AAGTTTTTAC TCAATGAAAT CATCCCTCGA CGTGGGCTGC CTGTTGCCAT AGGGTCTGAT 6960

AATGGACCGG CCTTCGCCTT GTCTATAGTT TAGTCAGTCA GTAAGGCGTT AAACATTCAA 7020

TGGAAGCTCC ATTGTGCCTA TCGACCCCAG AGCTCTGGGC AAGTAGAACG CATGAACTGC 7080

ACCCTAAAAA ACACTCTTAC AAAATTAATC TTAGAAACCG GTGTAAATTG TGTAAGTCTC 7140

CTTCCTTTAG CCCTACTTAG AGTAAGGTGC ACCCCTTACT GGGCTGGGTT CTTACCTTTT 7200

GAAATCATGT ATGGGAGGGC GCTGCCTATC TTGCCTAAGC TAAGAGATGC CCAATTGGCA 7260

AAAATATCAC AAACTAATTT ATTACAGTAC CTACAGTCTC CCCAACAGGT ACAAGATATC 7320

ATCCTGCCAC TTGTTCGAGG AACCCATCCC AATCCAATTC CTGAACAGAC AGGGCCCTGC 7380

CATTCATTCC CGCCAGGTGA CCTGTTGTTT GTTAAAAAGT TCCAGAGAGA AGGACTCCCT 7440

CCTGCTTGGA AGAGACCTCA CACCGTCATC ACGATGCCAA CGGCTCTGAA GGTGGATGGC 7500

ATTCCTGCGT GGATTCATCA CTCCCGCATC AAAAAGGCCA ACGGAGCCCA ACTAGAAACA 7560

TGGGTCCCCA GGGCTGGGTC AGGCCCCTTA AAACTGCACC TAAGTTGGGT GAAGCCATTA 7620

GATTAATTCT TTTTCTTAAT TTTGTAAAAC AATGCATAGC TTCTGTCAAA CTTATGTATC 7680

TTAAGACTCA ATATAACCCC CTTGTTATAA CTGAGGAATC AATGATTTGA TTCCCCAAAA 7740

ACACAAGTGG GGAATGTAGT GTCCAACCTG GTTTTTACTA ACCCTGTTTT TAGACTCTCC 7800

CTTTCCTTTA ATCACTCAGC CTTGTTTCCA CCTGAATTGA CTCTCCCTTA GCTAAGAGCG 7860

CCAGATGGAC TCCATCTTGG CTCTTTCACT GGCAGCCGCT TCCTCAAGGA CTTAACTTGT 7920

GCAAGCTGAC TCCCAGCACA TCCAAGAATG CAATTAACTG ATAAGATACT GTGGCAAGCT 7980

ATATCCGCAG TTCCCAGGAA TTCGTCCAAT TGATTACACC CAAAAGCCCC GCGTCTATCA 8040

CCTTGTAATA ATCTTAAAGC CCCTGCACCT GGAACTATTA ACGTTCCTGT AACCATTTAT 8100

CCTTTTAACT TTTTTGCCTA CTTTATTTCT GTAAAATTGT TTTAACTAGA CCCCCCCTCT 8160

CCTTTCTAAA CCAAAGTATA AAAGCAAATC TAGCCCCTTC TTCAGGCCGA GAGAATTTCG 8220

AGCGTTAGCC GTCTCTTGGC CACCAGCTAA ATAAACGGAT TCTTCATGTG TCTCAAAGTG 8280

TGGCGTTTTC TCTAACTCGC TCAGGTACGA CCGTGGTAGT ATTTTCCCCA ACGTCTTATT 8340

TTTAGGGCAC GTATGTAGAG TAACTTTTAT GAAAGAAACC AGTTAAGGAG GTTTTGGGAT 8400

TTCCTTTATC AACTGTAATA CTGGTTTTGA TTATTTATTT ATTTATTTAT TTTTTTTGAG 8460

AAGGAGTTTC ACTCTTGTTG CCCAGGCTGG AGTGCAATGG TGCGATCTTG GCTCACTGCA 8520

ACTTCCGCCT CCCAGGTTCA AGCGATTCTC CTGCCTCAGC CTCGAGAGTA GCTGGGATTA 8580

TAGGCATGCG CCACCACACC CAGCTAATTT TGTATTTTTA GTAAAGATGG GGTTTCTTCA 8640

TGTTGGTCAA GCTGGTCTGG AACTCCCCGC CTCGGGTGAT CTGCCCGCCT CGGCCTCCGA 8700

AAGTGCTGGG ATTACAGGTG TGATCCACCA CACCCAGCCG ATTTATATGT ATATAAATCA 8760

CATTCCTCTA ACCAAAATGT AGTGTTTCCT TCCATCTTGA ATATAGGCTG TAGACCCCGT 8820

GGGTATGGGA CATTGTTAAC AGTGAGACCA CAGCAGTTTT TATGTCATCT GACAGCATCT 8880

CCAAATAGCC TTCATGGTTG TCACTGCTTC CCAAGACAAT TCCAAATAAC ACTTCCCAGT 8940

GATGACTTGC TACTTGCTAT TGTTACTTAA TGTGTTAAGG TGGCTGTTAC AGACACTATT 9000

AGTATGTCAG GAATTACACC AAAATTTAGT GGCTCAAACA ATCATTTTAT TATGTATGTG 9060

GATTCTCATG GTCAGGTCAG GATTTCAGAC AGGGCACAAG GGTAGCCCAC TTGTCTCTGT 9120

CTATGATGTC TGGCCTCAGC ACAGGAGACT CAACAGCTGG GGTCTGGGAC CATTTGGAGG 9180

CTTGTTCCCT CACATCTGAT ACCTGGCTTG GGATGTTGGA AGAGGGGGTG AGCTGAGACT 9240

GAGTGCCTAT ATGTAGTGTT TCCATATGGC CTTGACTTCC TTACAGCCTG GCAGCCTCAG 9300

GGTAGTCAGA ATTCTTAGGA GGCACAGGGC TCCAGGGCAG ATGCTGAGGG GTCTTTTATG 9360

AGGTAGCACA GCAAATCCAC CCAGGATC 9388

(2) INFORMATION FOR SEQ ID NO:142:

(i) SEQUENCE CHARACTERISTICS:
(A) LENGTH: 419 base pairs
(B) TYPE: nucleic acid
(C) STRANDEDNESS: single
(D) TOPOLOGY: linear

(xi) SEQUENCE DESCRIPTION: SEQ ID NO:142:

TGTAAGTCGA GCAGTGTGAT GGAAGGAATG GTCTTTGGAG AGAGCATATC CATCTCCTCC 60

TCACTGCCTC CTAATGTCAT GAGGTACACT GAGCAGAATT AAACAGGGTA GTCTTAACCA 120

CACTATTTTT AGCTACCTTG TCAAGCTAAT GGTTAAAGAA CACTTTTGGT TTACACTTGT 180

TGGGTCATAG AAGTTGCTTT CCGCCATCAC GCAATAAGTT TGTGTGTAAT CAGAAGGAGT 240

TACCTTATGG TTTCAGTGTC ATTCTTTAGT TAACTTGGGA GCTGTGTAAT TTAGGCTTTG 300

CGTATTATTT CACTTCTGTT CTCCACTTAT GAAGTGATTG TGTGTTCGCG TGTGTGTGCG 360

TGCGCATGTG CTTCCGGCAG TTAACATAAG CAAATACCCA ACATCACACT GCTCGACTT 419

(2) INFORMATION FOR SEQ ID NO:143:

(i) SEQUENCE CHARACTERISTICS:
(A) LENGTH: 402 base pairs
(B) TYPE: nucleic acid

(C) STRANDEDNESS: single
(D) TOPOLOGY: linear

(xi) SEQUENCE DESCRIPTION: SEQ ID NO:143:

TGTAAGTCGA GCAGTGTGAT GTCCACTGCA GTGTGTTGCT GGGAACAGTT AATGAGCAAA 60

TTGTATACAA TGGCTAGTAC ATTGACCGGG ATTTGTTGAA GCTGGTGAGT GTTATGACTT 120

AGCCTGTTAG ACTAGTCTAT GCACATGGCT CTGGTCAACT ACCGCTCTCT CATTTCTCCA 180

GATAAATCCC CCATGCTTTA TATTCTCTTC CAAACATACT ATCCTCATCA CCACATAGTT 240

CCTTTGTTAA TGCTTTGTTC TAGACTTTCC CTTTTCTGTT TTCTTATTCA AACCTATATC 300

TCTTTGCATA GATTGTAAAT TCAAATGCCC TCAGGGTGCA GGCAGTTCAT GTAAGGGAGG 360

GAGGCTAGCC AGTGAGATCT GCATCACACT GCTCGACTTA CA 402

(2) INFORMATION FOR SEQ ID NO:144:

(i) SEQUENCE CHARACTERISTICS:
(A) LENGTH: 224 base pairs
(B) TYPE: nucleic acid
(C) STRANDEDNESS: single
(D) TOPOLOGY: linear

(xi) SEQUENCE DESCRIPTION: SEQ ID NO:144:

TCGGGTGATG CCTCCTCAGG CCAAGAAGAT AAAGCTTCAG ACCCCTAACA CATTTCCAAA 60

AAGGAAGAAA GGAGAAAAAA GGGCATCATC CCCGTTCCGA AGGGTCAGGG AGGAGGAAAT 120

TGAGGTGGAT TCACGAGTTG CGGACAACTC CTTTGATGCC AAGCGAGGTG CAGCCGGAGA 180

CTGGGGAGAG CGAGCCAATC AGGTTTTGAA GTTCCTCTCA GTGC 224

(2) INFORMATION FOR SEQ ID NO:145:

(i) SEQUENCE CHARACTERISTICS:
(A) LENGTH: 111 base pairs
(B) TYPE: nucleic acid
(C) STRANDEDNESS: single
(D) TOPOLOGY: linear

(xi) SEQUENCE DESCRIPTION: SEQ ID NO:145:

AGCCATTTAC CACCCATCCA CAAAAAAAAA AAAAAAAAAG AAAAATATCA AGGAATAAAA 60

ATAGACTTTG AACAAAAAGG AACATTTGCT GGCCTGAGGA GGCATCACCC G 111

(2) INFORMATION FOR SEQ ID NO:146:

(i) SEQUENCE CHARACTERISTICS:
(A) LENGTH: 585 base pairs
(B) TYPE: nucleic acid
(C) STRANDEDNESS: single
(D) TOPOLOGY: linear

(xi) SEQUENCE DESCRIPTION: SEQ ID NO:146:
   TAGCATGTTG AGCCCAGACA CTTGTAGAGA GAGGAGGACA GTTAGAAGAA GAAGAAAAGT 60
   TTTTAAATGC TGAAAGTTAC TATAAGAAAG CTTTGGCTTT GGATGAGACT TTTAAAGATG 120
   CAGAGGATGC TTTGCAGAAA CTTCATAAAT ATATGCAGGT GATTCCTTAT TTCCTCCTAG 180
   AAATTTAGTG ATATTTGAAA TAATGCCCAA ACTTAATTTT CTCCTGAGGA AAACTATTCT 240
   ACATTACTTA AGTAAGGCAT TATGAAAAGT TTCTTTTTAG GTATAGTTTT TCCTAATTGG 300
   GTTTGACATT GCTTCATAGT GCCTCTGTTT TTGTCCATAA TCGAAAGTAA AGATAGCTGT 360
   GAGAAAACTA TTACCTAAAT TTGGTATGTT GTTTTGAGAA ATGTCCTTAT AGGGAGCTCA 420
   CCTGGTGGTT TTTAAATTAT TGTTGCTACT ATAATTGAGC TAATTATAAA AACCTTTTTG 480
   AGACATATTT TAAATTGTCT TTTCCTGTAA TACTGATGAT GATGTTTTCT CATGCATTTT 540
   CTTCTGAATT GGGACCATTG CTGCTGTGTC TGGGCTCACA TGCTA 585

(2) INFORMATION FOR SEQ ID NO:147:

(i) SEQUENCE CHARACTERISTICS:
(A) LENGTH: 579 base pairs
(B) TYPE: nucleic acid
(C) STRANDEDNESS: single
(D) TOPOLOGY: linear

(xi) SEQUENCE DESCRIPTION: SEQ ID NO:147:

TAGCATGTTG AGCCCAGACA CTGGGCAGCG GGGGTGGCCA CGGCAGCTCC TGCCGAGCCC 60

AAGCGTGTTT GTCTGTGAAG GACCCTGACG TCACCTGCCA GGCTAGGGAG GGGTCAATGT 120

GGAGTGAATG TTCACCGACT TTCGCAGGAG TGTGCAGAAG CCAGGTGCAA CTTGGTTTGC 180

TTGTGTTCAT CACCCCTCAA GATATGCACA CTGCTTTCCA AATAAAGCAT CAACTGTCAT 240

CTCCAGATGG GGAAGACTTT TTCTCCAACC AGCAGGCAGG TCCCCATCCA CTCAGACACC 300

AGCACGTCCA CCTTCTCGGG CAGCACCACG TCCTCCACCT TCTGCTGGTA CACGGTGATG 360

ATGTCAGCAA AGCCGTTCTG CANGACCAGC TGCCCCGTGT GCTGTGCCAT CTCACTGGCC 420

TCCACCGCGT ACACCGCTCT AGGCCGCGCA TANTGTGCAC AGAANAAATG ATGATCCAGT 480

CCCACAGCCC ACGTCCAAGA NGACTTTATC CGTCAGGGAT TCTTTATTCT GCAGGATGAC 540

CTGTGGTATT AATTGTTCGT GTCTGGGCTC AACATGCTA 579

(2) INFORMATION FOR SEQ ID NO:148:

(i) SEQUENCE CHARACTERISTICS:
(A) LENGTH: 249 base pairs
(B) TYPE: nucleic acid
(C) STRANDEDNESS: single
(D) TOPOLOGY: linear

(xi) SEQUENCE DESCRIPTION: SEQ ID NO:148:

TGACACCTTG TCCAGCATCT GCAAGCCAGG AAGAGAGTCC TCACCAAGAT CCCCACCCCG 60

TTGGCACCAG GATCTTGGAC TTCCAATCTC CAGAACTGTG AGAAATAAGT ATTTGTCGCT 120

AAATAAATCT TTGTGGTTTC AGATATTTAG CTATAGCAGA TCAGGCTGAC TAAGAGAAAC 180

CCCATAAGAG TTACATACTC ATTAATCTCC GTCTCTATCC CCAGGTCTCA GATGCTGGAC 240

AAGGTGTCA 249

(2) INFORMATION FOR SEQ ID NO:149:

(i) SEQUENCE CHARACTERISTICS:
(A) LENGTH: 255 base pairs
(B) TYPE: nucleic acid
(C) STRANDEDNESS: single
(D) TOPOLOGY: linear

(xi) SEQUENCE DESCRIPTION: SEQ ID NO:149:

TGACACCTTG TCCAGCATCT GCTATTTTGT GACTTTTTAA TAATAGCCAT TCTGACTGGT 60

GTGAGATGGT AACTCATTGT GGGTTTGGTC TGCATTTCTC TAATGATCAG TGATATTAAG 120

CTTTTTTTAA ATATGCTTGT TGACCACATG TATATCATCT TTTGAGAAGT GTCTGTTCAT 180

ATCCTTTGCC CACTTTTTAA TTTTTTTATC TTGTAAATTT GTTTAATTTC CTTACAGATG 240

CTGGACAAGG TGTCA 255

(2) INFORMATION FOR SEQ ID NO:150:

(i) SEQUENCE CHARACTERISTICS:
(A) LENGTH: 318 base pairs
(B) TYPE: nucleic acid
(C) STRANDEDNESS: single
(D) TOPOLOGY: linear

(xi) SEQUENCE DESCRIPTION: SEQ ID NO:150:

TTACGCTGCA ACACTGTGGA GGCCAAGCTG GGATCACTTC TTCATTCTAA CTGGAGAGGA 60

GGGAAGTTCA AGTCCAGCAG AGGGTGGGTG GGTAGACAGT GGCACTCAGA AATGTCAGCT 120

GGACCCCTGT CCCCGCATAG GCAGGACAGC AAGGCTGTGG CTCTCCAGGG CCAGCTGAAG 180

AACAGGACAC TGTCTCCGCT GCCACAAAGC GTCAGAGACT CCCATCTTTG AAGCACGGCC 240

TTCTTGGTCT TCCTGCACTT CCCTGTTCTG TTAGAGACCT GGTTATAGAC AAGGCTTCTC 300

CACAGTGTTG CAGCGTAA 318

(2) INFORMATION FOR SEQ ID NO:151:

(i) SEQUENCE CHARACTERISTICS:
(A) LENGTH: 323 base pairs
(B) TYPE: nucleic acid
(C) STRANDEDNESS: single
(D) TOPOLOGY: linear

(xi) SEQUENCE DESCRIPTION: SEQ ID NO:151:

TNACGCNGCN ACNNTGTAGA GANGGNAAGG CNTTCCCCAC ATTNCCCCTT CATNANAGAA 60

TTATTCNACC AAGNNTGACC NATGCCNTTT ATGACTTACA TGCNNACTNC NTAATCTGTN 120

TCNNGCCTTA AAAGCNNNTC CACTACATGC NTCANCACTG TNTGTGTNAC NTCATNAACT 180

GTCNGNAATA GGGGCNCATA ACTACAGAAA TGCANTTCAT ACTGCTTCCA NTGCCATCNG 240

CGTGTGGCCT TNCCTACTCT TCTTNTATTC CAAGTAGCAT CTCTGGANTG CTTCCCCACT 300

CTCCACATTG TTGCAGCNAT AAT 323

(2) INFORMATION FOR SEQ ID NO:152:

(i) SEQUENCE CHARACTERISTICS:
(A) LENGTH: 311 base pairs
(B) TYPE: nucleic acid
(C) STRANDEDNESS: single
(D) TOPOLOGY: linear

(xi) SEQUENCE DESCRIPTION: SEQ ID NO:152:

TCAAGATTCC ATAGGCTGAC CAGTCCAAGG AGAGTTGAAA TCATGAAGGA GAGTCTATCT 60

GGAGAGAGCT GTAGTTTTGA GGGTTGCAAA GACTTAGGAT GGAGTTGGTG GGTGTGGTTA 120

GTCTCTAAGG TTGATTTTGT TCATAAATTT CATGCCCTGA ATGCCTTGCT TGCCTCACCC 180

TGGTCCAAGC CTTAGTGAAC ACCTAAAAGT CTCTGTCTTC TTGCTCTCCA AACTTCTCCT 240

GAGGATTTCC TCAGATTGTC TACATTCAGA TCGAAGCCAG TTGGCAAACA AGATGCAGTC 300

CAGAGGGTCA G 311

(2) INFORMATION FOR SEQ ID NO:153:

(i) SEQUENCE CHARACTERISTICS:
(A) LENGTH: 332 base pairs
(B) TYPE: nucleic acid
(C) STRANDEDNESS: single
(D) TOPOLOGY: linear

(xi) SEQUENCE DESCRIPTION: SEQ ID NO:153:

CAAGATTCCA TAGGCTGACC AGGAGGCTAT TCAAGATCTC TGGCAGTTGA GGAAGTCTCT 60

TTAAGAAAAT AGTTTAAACA ATTTGTTAAA ATTTTTCTGT CTTACTTCAT TTCTGTAGCA 120

GTTGATATCT GGCTGTCCTT TTTATAATGC AGAGTGGGAA CTTTCCCTAC CATGTTTGAT 180

AAATGTTGTC CAGGCTCCAT TGCCAATAAT GTGTTGTCCA AAATGCCTGT TTAGTTTTTA 240

AAGACGGAAC TCCACCCTTT GCTTGGTCTT AAGTATGTAT GGAATGTTAT GATAGGACAT 300

AGTAGTAGCG GTGGTCAGCC TATGGAATCT TG 332

(2) INFORMATION FOR SEQ ID NO:154:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:154:

TCAACATTCC ATAGGCTGAC CTGGACAGAG ATCTCCTGGG TCTGGCCCAG GACAGCAGGC 60

TCAAGCTCAG TGGAGAAGGT TTCCATGACC CTCAGATTCC CCCAAACCTT GGATTGGGTG 120

ACATTGCATC TCCTCAGAGA GGGAGGAGAT GTANGTCTGG GCTTCCACAG GGACCTGGTA 180

TTTTAGGATC AGGGTACCGC TGGCCTGAGG CTTGGATCAT TCANAGCCTG GGGGTGGAAT 240

GGCTGGCAGC CTGTGGCCCC ATTGAAATAG GCTCTGGGGC ACTCCCTCTG TTCCTANTTG 300

AACTTGGGTA AGGAACAGGA ATGTGGTCAN CCTATGGAAT CTTGA 345

(2) INFORMATION FOR SEQ ID NO:155:

(i) SEQUENCE CHARACTERISTICS:
(A) LENGTH: 295 base pairs
(B) TYPE: nucleic acid
(C) STRANDEDNESS: single
(D) TOPOLOGY: linear

(xi) SEQUENCE DESCRIPTION: SEQ ID NO:155:

GACGCTTGGC CACTTGACAC ATTAAACAGT TTTGCATAAT CACTANCATG TATTTCTAGT 60

TTGCTGTCTG CTGTGATGCC CTGCCCTGAT TCTCTGGCGT TAATGATGGC AAGCATAATC 120

AAACGCTGTT CTGTTAATTC CAAGTTATAA CTGGCATTGA TTAAAGCATT ATCTTTCACA 180

ACTAAACTGT TCTTCATANA ACAGCCCATA TTATTATCAA ATTAAGAGAC AATGTATTCC 240

AATATCCTTT ANGGCCAATA TATTTNATGT CCCTTAATTA AGAGCTACTG TCCGT 295

(2) INFORMATION FOR SEQ ID NO:156:

(i) SEQUENCE CHARACTERISTICS:
(A) LENGTH: 406 base pairs
(B) TYPE: nucleic acid
(C) STRANDEDNESS: single
(D) TOPOLOGY: linear

(xi) SEQUENCE DESCRIPTION: SEQ ID NO:156:

GACGCTTGGC CACTTGACAC TGCAGTGGGA AAACCAGCAT GAGCCGCTGC CCCCAAGGAA 60

CCTCGAAGCC CAGGCAGAGG ACCAGCCATC CCAGCCTGCA GGTAAAGTGT GTCACCTGTC 120

AGGTGGGCTT GGGGTGAGTG GGTGGGGGAA GTGTGTGTGC AAAGGGGGTG TNAATGTNTA 180

TGCGTGTGAG CATGAGTGAT GGCTAGTGTG ACTGCATGTC AGGGAGTGTG AACAAGCGTG 240

CGGGGGTGTG TGTGCAAGTG CGTATGCATA TGAGAATATG TGTCTGTGGA TGAGTGCATT 300

TGAAAGTCTG TGTGTGTGCG TGTGGTCATG ANGGTAANTT ANTGACTGCG CAGGATGTGT 360

GAGTGTGCAT GGAACACTCA NTGTGTGTGT CAAGTGGCCN ANCGTC 406

(2) INFORMATION FOR SEQ ID NO:157:

(i) SEQUENCE CHARACTERISTICS:
(A) LENGTH: 208 base pairs
(B) TYPE: nucleic acid

(C) STRANDEDNESS: single
(D) TOPOLOGY: linear

(xi) SEQUENCE DESCRIPTION: SEQ ID NO:157:

TGACGCTTGG CCACTTGACA CACTAAAGGG TGTTACTCAT CACTTTCTTC TCTCCTCGGT 60

GGCATGTGAG TGCATCTATT CACTTGGCAC TCATTTGTTT GGCAGTGACT GTAANCCANA 120

TCTGATGCAT ACACCAGCTT GTAAATTGAA TAAATGTCTC TAATACTATG TGCTCACAAT 180

ANGGTANGGG TGAGGAGAAG GGGAGAGA 208

(2) INFORMATION FOR SEQ ID NO:158:

(i) SEQUENCE CHARACTERISTICS:
(A) LENGTH: 547 base pairs
(B) TYPE: nucleic acid
(C) STRANDEDNESS: single
(D) TOPOLOGY: linear

(xi) SEQUENCE DESCRIPTION: SEQ ID NO:158:

CTTCAACCTC CTTCAACCTC CTTCAACCTC CTGGATTCAA ACAATCATCC CACCTCAGAC 60

TCCTTAGTAG CTGAGACTAC AGACTCACGC CACTACATCT GGCTAAATTT TTGTAGAGAT 120

AGGGTTTCAT CATGTTGCCC TGGCTGGTCT CAAACTCCTG ACCTCAAGCA ATGTGCCCAC 180

CTCAGCCTCC CAAAGTGCTG GGATTACAGG CATAAGCCAC CATGCCCAGT CCATNTTTAA 240

TCTTTCCTAC CACATTCTTA CCACACTTTC TTTTATGTTT AGATACATAA ATGCTTACCA 300

TTATGATACA ATTGCCCACA GTATTAAGAC AGTAACATGC TGCACAGGTT TGTAGCCTAG 360

GAACAGTAGG CAATACCACA TAGCTTAGGT GTGTGGTAGA CTATACCATC TAGGTTTGTG 420

TAAGTTACAC TTTATGCTGT TTACACAATG ACAAAACCAT CTAATGATGC ATTTCTCAGA 480

ATGTATCCTT GTCAGTAAGC TATGATGTAC AGGGAACACT GCCCAAGGAC ACAGATATTG 540

TACCTGT 547

(2) INFORMATION FOR SEQ ID NO:159:

(i) SEQUENCE CHARACTERISTICS:
(A) LENGTH: 203 base pairs
(B) TYPE: nucleic acid
(C) STRANDEDNESS: single
(D) TOPOLOGY: linear

(xi) SEQUENCE DESCRIPTION: SEQ ID NO:159:

GCTCCTCTTG CCTTACCAAC TCACCCAGTA TGTCAGCAAT TTTATCRGCT TTACCTACGA 60

AACAGCCTGT ATCCAAACAC TTAACACACT CACCTGAAAA GTTCAGGCAA CAATCGCCTT 120

CTCATGGGTC TCTCTGCTCC AGTTCTGAAC CTTTCTCTTT TCCTAGAACA TGCATTTARG 180

TCGATAGAAG TTCCTCTCAG TGC 203

(2) INFORMATION FOR SEQ ID NO:160:

(i) SEQUENCE CHARACTERISTICS:
(A) LENGTH: 402 base pairs
(B) TYPE: nucleic acid
(C) STRANDEDNESS: single
(D) TOPOLOGY: linear

(xi) SEQUENCE DESCRIPTION: SEQ ID NO:160:

TGTAAGTCGA GCAGTGTGAT GGGTGGAACA GGGTTGTAAG CAGTAATTGC AAACTGTATT 60

TAAACAATAA TAATAATATT TAGCATTTAT AGAGCACTTT ATATCTTCAA AGTACTTGCA 120

AACATTAYCT AATTAAATAC CCTCTCTGAT TATAATCTGG ATACAAATGC ACTTAAACTC 180

AGGACAGGGT CATGAGARAA GTATGCATTT GAAAGTTGGT GCTAGCTATG CTTTAAAAAC 240

CTATACAATG ATGGGRAAGT TAGAGTTCAG ATTCTGTTGG ACTGTTTTTG TGCATTTCAG 300

TTCAGCCTGA TGGCAGAATT AGATCATATC TGCACTCGAT GACTYTGCTT GATAACTTAT 360

CACTGAAATC TGAGTGTTGA TCATCACACT GCTCGACTTA CA 402

(2) INFORMATION FOR SEQ ID NO:161:

(i) SEQUENCE CHARACTERISTICS:
(A) LENGTH: 193 base pairs
(B) TYPE: nucleic acid
(C) STRANDEDNESS: single
(D) TOPOLOGY: linear

(xi) SEQUENCE DESCRIPTION: SEQ ID NO:161:

AGCATGTTGA GCCCAGACAC TGACCAGGAG AAAAACCAAC CAATAGAAAC ACGCCCAGAC 60

ACTGACCAGG AGAAAAACCA ACCAATAAAA ACAGGCCCGG ACATAAGACA AATAATAAAA 120

TTAGCGGACA AGGACATGAA AACAGCTATT GTAAGAGCGG ATATAGTGGT GTGTGTCTGG 180

GCTCAACATG CTA 193

(2) INFORMATION FOR SEQ ID NO:162:

(i) SEQUENCE CHARACTERISTICS:
(A) LENGTH: 147 base pairs
(B) TYPE: nucleic acid
(C) STRANDEDNESS: single
(D) TOPOLOGY: linear

(xi) SEQUENCE DESCRIPTION: SEQ ID NO:162:

TGTTGAGCCC AGACACTGAC CAGGAGAAAA ACCAACCAAT AAAAACAGGC CCGGACATAA 60

GACAAATAAT AAAATTAGCG GACAAGGACA TGAAAACAGC TATTGTAAGA GCGGATATAG 120

TGGTGTGTGT CTGGGCTCAA CATGCTA 147

(2) INFORMATION FOR SEQ ID NO:163:

(i) SEQUENCE CHARACTERISTICS:
(A) LENGTH: 294 base pairs
(B) TYPE: nucleic acid
(C) STRANDEDNESS: single
(D) TOPOLOGY: linear

(xi) SEQUENCE DESCRIPTION: SEQ ID NO:163:

TAGCATGTTG AGCCCAGACA CAAATCTTTC CTTAAGCAAT AAATCATTTC TGCATATGTT 60

TTTAAAACCA CAGCTAAGCC ATGATTATTC AAAAGGACTA TTGTATTGGG TATTTTGATT 120

TGGGTTCTTA TCTCCCTCAC ATTATCTTCA TTTCTATCAT TGACCTCTTA TCCCAGAGAC 180

TCTCAAACTT TTATGTTATA CAAATCACAT TCTGTCTCAA AAAATATCTC ACCCACTTCT 240

CTTCTGTTTC TGCGTGTGTA TGTGTGTGTG TGTGTGTCTG GGCTCAACAT GCTA 294

(2) INFORMATION FOR SEQ ID NO:164:

(i) SEQUENCE CHARACTERISTICS:
(A) LENGTH: 412 base pairs
(B) TYPE: nucleic acid
(C) STRANDEDNESS: single
(D) TOPOLOGY: linear

(xi) SEQUENCE DESCRIPTION: SEQ ID NO:164:

CGGGATTGGC TTTGAGCTGC AGATGCTGCC TGTGACCGCA CCCGGCGTGG AACAGAAAGC 60

CACCTGGCTG CAAGTGCGCC AGAGCCGCCC TGACTACGTG CTGCTGTGGG GCTGGGGCGT 120

GATGAACTCC ACCGCCCTGA AGGAAGCCCA GGCCACCGGA TACCCCCGCG ACAAGATGTA 180

CGGCGTGTGG TGGGCCGGTG CGGAGCCCGA TGTGCGTGAC GTGGGCGAAG GCGCCAAGGG 240

CTACAACGCG CTGGCTCTGA ACGGCTACGG CACGCAGTCC AAGGTGATCC ANGACATCCT 300

GAAACACGTG CACGACAAGG GCCAGGGCAC GGGGCCCAAA GACGAAGTGG GCTCGGTGCT 360

GTACACCCGC GGCGTGATCA TCCAGATGCT GGACAAGGTG TCAATCACTA AT 412

(2) INFORMATION FOR SEQ ID NO:165:

(i) SEQUENCE CHARACTERISTICS:
(A) LENGTH: 361 base pairs
(B) TYPE: nucleic acid
(C) STRANDEDNESS: single
(D) TOPOLOGY: linear

(xi) SEQUENCE DESCRIPTION: SEQ ID NO:165:

TTGACACCTT GTCCAGCATC TGCATCTGAT GAGAGCCTCA GATGGCTACC ACTAATGGCA 60

GAAGGCAAAG GAGAACAGGC ATTGTATGGC AAGAAAGGAA GAAAGAGAGA GGGGAGAAAG 120

GTGCTAGGTT CTTTTCAACA ACCAGTTCTT GATGGAACTG AGAGTAAGAG CTCAAGGCCA 180

GGTGTGGTGA CTCCAACCAG TAATCCCAAC ATTTTAGGAG GCTGAGGCAG GCAGATGTCT 240

TGACCCCATG AGTTTGTGAC CAGCCTGAAC AACATCATGA GACTCCATCT CTACAATAAT 300

TACAAAAATT AATCAGGCAT TGTGGTATGC CCTGTAGTCC CAGATGCTGG ACAAGGTGTC 360

A 361

(2) INFORMATION FOR SEQ ID NO:166:

(i) SEQUENCE CHARACTERISTICS:
(A) LENGTH: 427 base pairs
(B) TYPE: nucleic acid
(C) STRANDEDNESS: single
(D) TOPOLOGY: linear

(xi) SEQUENCE DESCRIPTION: SEQ ID NO:166:

TWGACTGACT CATGTCCCCT ACACCCAACT ATCTTCTCCA GGTGGCCAGG CATGATAGAA 60

TCTGATCCTG ACTTAGGGGA ATATTTTCTT TTTACTTCCC ATCTTGATTC CCTGCCGGTG 120

AGTTTCCTGG TTCAGGGTAA GAAAGGAGCT CAGGCCAAAG TAATGAACAA ATCCATCCTC 180

ACAGACGTAC AGAATAAGAG AACWTGGACW TAGCCAGCAG AACMCAAKTG AAAMCAGAAC 240

MCTTAMCTAG GATRACAAMC MCRRARATAR KTGCYCMCMC WTATAATAGA AACCAAACTT 300

GTATCTAATT AAATATTTAT CCACYGTCAG GGCATTAGTG GTTTTGATAA ATACGCTTTG 360

GCTAGGATTC CTGAGGTTAG AATGGAARAA CAATTGCAMC GAGGGTAGGG GACATGAGTC 420

AKTCTAA 427

(2) INFORMATION FOR SEQ ID NO:167:

(i) SEQUENCE CHARACTERISTICS:
(A) LENGTH: 500 base pairs
(B) TYPE: nucleic acid
(C) STRANDEDNESS: single
(D) TOPOLOGY: linear

(xi) SEQUENCE DESCRIPTION: SEQ ID NO:167:

AACGTCGCAT GCTCCCGGCC GCCATGGCCG CGGGATAGAC TGACTCATGT CCCCTAAGAT 60

AGAGGAGACA CCTGCTAGGT GTAAGGAGAA GATGGTTAGG TCTACGGAGG CTCCAGGGTG 120

GGAGTAGTTC CCTGCTAAGG GAGGGTAGAC TGTTCAACCT GTTCCTGCTC CGGCCTCCAC 180

TATAGCAGAT GCGAGCAGGA GTAGGAGAGA GGGAGGTAAG AGTCAGAAGC TTATGTTGTT 240

TATGCGGGGA AACGCCRTAT CGGGGGCAGC CRAGTTATTA GGGGACANTR TAGWYARTCW 300

AGNTAGCATC CAAAGCGNGG GAGTTNTCCC ATATGGTTGG ACCTGCAGGC GGCCGCATTA 360

GTGATTAGCA TGTGAGCCCC AGACACGCAT AGCAACAAGG ACCTAAACTC AGATCCTGTG 420

CTGATTACTT AACATGAATT ATTGTATTTA TTTAACAACT TTGAGTTATG AGGCATATTA 480

TTAGGTCCAT ATTACCTGGA 500

(2) INFORMATION FOR SEQ ID NO:168:

(i) SEQUENCE CHARACTERISTICS:
(A) LENGTH: 358 base pairs
(B) TYPE: nucleic acid
(C) STRANDEDNESS: single
(D) TOPOLOGY: linear

(xi) SEQUENCE DESCRIPTION: SEQ ID NO:168:

TTCATCGCTC GGTGACTCAA GCCTGTAATC CCAGAACTTT GGGAGGCCGA GGGGAGCAGA 60

TCACCTGAGG TTGGGAGTTT GAGACCAGCC TGGCCAACAT GGTGACAACC CGTCTCTGCT 120

AAAAATACAA AAATTAGCCA AGCATGGTGG CATGCACTTG TAATCCCAGC TACTCGGGAG 180

GCTGAGGCAG GAGAATCACT TGAGGCCAGG AGGCAGAGGT TGCAGTGAGG CAGAGGTTGA 240

GATCATGCCA CTGCACTCCA GCCTGGGCAA CAGAGTAAGA CTCCATCTCA AAAAAAAAAA 300

AAAAAAAGAA TGATCAGAGC CACAAATACA GAAAACCTTG AGTCACCGAG CGATGAAA 358

(2) INFORMATION FOR SEQ ID NO:169:

(i) SEQUENCE CHARACTERISTICS:
(A) LENGTH: 1265 base pairs
(B) TYPE: nucleic acid
(C) STRANDEDNESS: single
(D) TOPOLOGY: linear

(xi) SEQUENCE DESCRIPTION: SEQ ID NO:169:

TTCTGTCCAC ACCAATCTTA GAGCTCTGAA AGAATTTGTC TTTAAATATC TTTTAATAGT 60

AACATGTATT TTATGGACCA AATTGACATT TTCGACTATT TTTTCCCAAA AAAAGTCAGG 120

TGAATTTCAG CACACTGAGT TGGGAATTTC TTATCCCAGA AGWCGGCACG AGCAATTTCA 180

TATTTATTTA AGATTGATTC CATACTCCGT TTTCAAGGAG AATCCCTGCA GTCTCCTTAA 240

AGGTAGAACA AATACTTTCT ATTTTTTTTT CACCATTGTG GGATTGGACT TTAAGAGGTG 300

ACTCTAAAAA AACAGAGAAC AAATATGTCT CAGTTGTATT AAGCACGGAC CCATATTATC 360

ATATTCACTT AAAAAAATGA TTTCCTGTGC ACCTTTTGGC AACTTCTCTT TTCAATGTAG 420

GGAAAAACTT AGTCACCCTG AAAACCCACA AAATAAATAA AACTTGTAGA TGTGGGCAGA 480

ARGTTTGGGG GTGGACATTG TATGTGTTTA AATTAAACCC TGTATCACTG AGAAGCTGTT 540

GTATGGGTCA GAGAAAATGA ATGCTTAGAA GCTGTTCACA TCTTCAAGAG CAGAAGCAAA 600

CCACATGTCT CAGCTATATT ATTATTTATT TTTTATGCAT AAAGTGAATC ATTTCTTCTG 660

TATTAATTTC CAAAGGGTTT TACCCTCTAT TTAAATGCTT TGAAAAACAG TGCATTGACA 720

ATGGGTTGAT ATTTTTCTTT AAAAGAAAAA TATAATTATG AAAGCCAAGA TAATCTGAAG 780

CCTGTTTTAT TTTAAAACTT TTTATGTTCT GTGGTTGATG TTGTTTGTTT GTTTGTTTCT 840

ATTTTGTTGG TTTTTTACTT TGTTTTTTGT TTTGTTTTGT TTTGGTTTDG CATACTACAT 900

GCAGTTTCTT TAACCAATGT CTGTTTGGCT AATGTAATTA AAGTTGTTAA TTTATATGAG 960

TGCATTTCAA CTATGTCAAT GGTTTCTTAA TATTTATTGT GTAGAAGTAC TGGTAATTTT 1020

TTTATTTACA ATATGTTTAA AGAGATAACA GTTTGATATG TTTTCATGTG TTTATAGCAG 1080

AAGTTATTTA TTTCTATGGC ATTCCAGCGG ATATTTTGGT GTTTGCGAGG CATGCAGTCA 1140

ATATTTTGTA CAGTTAGTGG ACAGTATTCA GCAACGCCTG ATAGCTTCTT TGGCCTTATG 1200

TTAAATAAAA AGACCTGTTT GGGATGTAAA AAAAAAAAAA AAAAAAAAAA AAAAAAAAAA 1260

AAAAA 1265

(2) INFORMATION FOR SEQ ID NO:170:

(i) SEQUENCE CHARACTERISTICS:
(A) LENGTH: 383 base pairs
(B) TYPE: nucleic acid
(C) STRANDEDNESS: single
(D) TOPOLOGY: linear

(xi) SEQUENCE DESCRIPTION: SEQ ID NO:170:

TGTAAGTCGA GCAGTGTGAT GACGATATTC TTCTTATTAA TGTGGTAATT GAACAAATGA 60

TCTGTGATAC TGATCCTGAG CTAGGAGGCG CTGTTCAGTT AATGGGACTT CTTCGTACTC 120

TAATTGATCC AGAGAACATG CTGGCTACAA CTAATAAAAC CGAAAAAAGT GAATTTCTAA 180

ATTTTTTCTA CAACCATTGT ATGCATGTTC TCACAGCACC ACTTTTGACC AATACTTCAG 240

AAGACAAATG TGAAAAGGAT AATATAGTTG GATCAAACAA AAACAACACA ATTTGTCCCG 300

ATAATTATCA AACAGCACAG CTACTTGCCT TAATTTTAGA GTTACTCACA TTTTGTGTGG 360

AACATCACAC TGCTCGACTT ACA 383

(2) INFORMATION FOR SEQ ID NO:171:

(i) SEQUENCE CHARACTERISTICS:
(A) LENGTH: 383 base pairs
(B) TYPE: nucleic acid
(C) STRANDEDNESS: single
(D) TOPOLOGY: linear

(xi) SEQUENCE DESCRIPTION: SEQ ID NO:171:

TGGGCACCTT CAATATCCCA AGTTAAAAAT AATGTTGAGT TTATTATACT TTTGACCTGT 60

TTAGCTCAAC AGGGTGAAGG CATGTAAAGA ATGTGGACTT CTGAGGAATT TTCTTTTAAA 120

AAGAACATAA TGAAGTAACA TTTTAATTAC TCAAGGACTA CTTTTGGTTG AAGTTTATAA 180

TCTAGATACC TCTACTTTTT GTTTTTGCTG TTCGACAGTT CACAAAGACC TTCAGCAATT 240

TACAGGGTAA AATCGTTGAA GTAGTGGAGG TGAAACTGAA ATTTAAAATT ATTCTGTAAA 300

TACTATAGGG AAAGAGGCTG AGCTTAGAAT CTTTTGGTTG TTCATGTGTT CTGTGCTCTT 360

ATCATCACAC TGCTCGACTT ACA 383

(2) INFORMATION FOR SEQ ID NO:172:

(i) SEQUENCE CHARACTERISTICS:
(A) LENGTH: 699 base pairs
(B) TYPE: nucleic acid
(C) STRANDEDNESS: single
(D) TOPOLOGY: linear

(xi) SEQUENCE DESCRIPTION: SEQ ID NO:172:

TCGGGTGATG CCTCCTCAGG CTTGTCGTTA GTGTACACAG AGCTGCTCAT GAAGCGACAG 60

CGGCTGCCCC TGGCACTTCA GAACCTCTTC CTCTACACTT TTGGTGCGCT TCTGAATCTA 120

GGTCTGCATG CTGGCGGCGG CTCTGGCCCA GGCCTCCTGG AAAGTTTCTC AGGATGGGCA 180

GCACTCGTGG TGCTGAGCCA GGCACTAAAT GGACTGCTCA TGTCTGCTGT CATGGAGCAT 240

GGCAGCAGCA TCACACGCCT CTTTGTGGTG TCCTGCTCGC TGGTGGTCAA CGCCGTGCTC 300

TCAGCAGTCC TGCTACGGCT GCAGCTCACA GCCGCCTTCT TCCTGGCCAC ATTGCTCATT 360

GGCCTGGCCA TGCGCCTGTA CTATGGCAGC CGCTAGTCCC TGACAACTTC CACCCTGATT 420

CCGGACCCTG TAGATTGGGC GCCACCACCA GATCCCCCTC CCAGGCCTTC CTCCCTCTCC 480

CATCAGCGGC CCTGTAACAA GTGCCTTGTG AGAAAAGCTG GAGAAGTGAG GGCAGCCAGG 540

TTATTCTCTG GAGGTTGGTG GATGAAGGGG TACCCCTAGG AGATGTGAAG TGTGGGTTTG 600

GTTAAGGAAA TGCTTACCAT CCCCCACCCC CAACCAAGTT NTTCCAGACT AAAGAATTAA 660

GGTAACATCA ATACCTAGGC CTGAGGAGGC ATCACCCGA 699

(2) INFORMATION FOR SEQ ID NO:173:

(i) SEQUENCE CHARACTERISTICS:
(A) LENGTH: 701 base pairs
(B) TYPE: nucleic acid
(C) STRANDEDNESS: single
(D) TOPOLOGY: linear

(xi) SEQUENCE DESCRIPTION: SEQ ID NO:173:

TCGGGTGATG CCTCCTCAGG CCAGATCAAA CTTGGGGTTG AAAACTGTGC AAAGAAATCA 60

ATGTCGGAGA AAGAATTTTG CAAAAGAAAA ATGCCTAATC AGTACTAATT TAATAGGTCA 120

CATTAGCAGT GGAAGAAGAA ATGTTGATAT TTTATGTCAG CTATTTTATA ATCACCAGAG 180

TGCTTAGCTT CATGTAAGCC ATCTCGTATT CATTAGAAAT AAGAACAATT TTATTCGTCG 240

GAAAGAACTT TTCAATTTAT AGCATCTTAA TTGCTCAGGA TTTTAAATTT TGATAAAGAA 300

AGCTCCACTT TTGGCAGGAG TAGGGGGCAG GGAGAGAGGA GGCTCCATCC ACAAGGACAG 360

AGACACCAGG GCCAGTAGGG TAGCTGGTGG CTGGATCAGT CACAACGGAC TGACTTATGC 420

CATGAGAAGA AACAACCTCC AAATCTCAGT TGCTTAATAC AACACAAGCT CATTTCTTGC 480

TCACGTTACA TGTCCTATGT AGATCAACAG CAGGTGACTC AGGGACCCAG GCTCCATCTC 540

CATATGAGCT TCCATAGTCA CCAGGACACG GGCTCTGAAA GTGTCCTCCA TGCAGGGACA 600

CATGCCTCTT CCTTTCATTG GGCAGAGCAA GTCACTTATG GCCAGAAGTC ACACTGCAGG 660

GCAGTGCCAT CCTGCTGTAT GCCTGAGGAG GCATCACCCG A 701

(2) INFORMATION FOR SEQ ID NO:174:

(i) SEQUENCE CHARACTERISTICS:
(A) LENGTH: 700 base pairs
(B) TYPE: nucleic acid
(C) STRANDEDNESS: single
(D) TOPOLOGY: linear

(xi) SEQUENCE DESCRIPTION: SEQ ID NO:174:

TCGGGTGATG CCTCCTCANG CCCCTAAATC AGAGTCCAGG GTCAGAGCCA CAGGAGACAG 60

GGAAAGACAT AGATTTTAAC CGGCCCCCTT CAGGAGATTC TGAGGCTCAG TTCACTTTGT 120

TGCAGTTTGA ACAGAGGCAG CAAGGCTAGT GGTTAGGGGC ACGGTCTCTA AAGCTGCACT 180

GCCTGGATCT GCCTCCCAGC TCTGCCAGGA ACCAGCTGCG TGGCCTTGAG CTGCTGACAC 240

GCAGAAAGCC CCCTGTGGAC CCAGTCTCCT CGTCTGTAAG ATGAGGACAG GACTCTAGGA 300

ACCCTTTCCC TTGGTTTGGC CTCACTTTCA CAGGCTCCCA TCTTGAACTC TATCTACTCT 360

TTTCCTGAAA CCTTGTAAAA GAAAAAAGTG CTAGCCTGGG CAACATGGCA AAACCCTGTC 420

TCTACAAAAA ATACAAAAAT TAGTTGGGTG TGGTGGCATG TGCCTGTAGT CCCAGCCACT 480

TGGGAGGTGC TGAGGTGGGA GGATCACTTG AGCCCGGGAG GTGGAGGTTG CAGTGAGCCA 540

AGATCATGCC ACTGCACTCC AGCCTGAGTA ATAGAGTAAG ACTCTGTCTC AAAAACAACA 600

ACAACAACAG TGAGTGTGCC TCTGTTTCCG GGTTGGATGG GGCACCACAT TTATGCATCT 660

CTCAGATTTG GACGCTGCAG CCTGAGGAGG CATCACCCGA 700

(2) INFORMATION FOR SEQ ID NO:175:

(i) SEQUENCE CHARACTERISTICS:
(A) LENGTH: 484 base pairs
(B) TYPE: nucleic acid
(C) STRANDEDNESS: single
(D) TOPOLOGY: linear

(xi) SEQUENCE DESCRIPTION: SEQ ID NO:175:

TATAGGGCGA ATTGGGCCCG AGTTGCATGN TCCCGGCCGC CATGGCCGCG GGATTCGGGT 60

GATGCCTCCT CAGGCTTGTC TGCCACAAGC TACTTCTCTG AGCTCAGAAA GTGCCCCTTG 120

ATGAGGGAAA ATGTCCTACT GCACTGCGAA TTTCTCAGTT CCATTTTACC TCCCAGTCCT 180

CCTTCTAAAC CAGTTAATAA ATTCATTCCA CAAGTATTTA CTGATTACCT GCTTGTGCCA 240

GGGACTATTC TCAGGCTGAA GAAGGTGGGA GGGGAGGGCG GAACCTGAGG AGCCACCTGA 300

GCCAGCTTTA TATTTCAACC ATGGCTGGCC CATCTGAGAG CATCTCCCCA CTCTCGCCAA 360

CCTATCGGGG CATAGCCCAG GGATGCCCCC AGGCGGCCCA GGTTAGATGC GTCCCTTTGG 420

CTTGTCAGTG ATGACATACA CCTTAGCTGC TTAGCTGGTG CTGGCCTGAG GAGGCATCAC 480

CCGA 484

(2) INFORMATION FOR SEQ ID NO:176:

(i) SEQUENCE CHARACTERISTICS:
(A) LENGTH: 432 base pairs
(B) TYPE: nucleic acid
(C) STRANDEDNESS: single
(D) TOPOLOGY: linear

(xi) SEQUENCE DESCRIPTION: SEQ ID NO:176:

TCGGGTGATG CCTCCTCAGG GCTCAAGGGA TGAGAAGTGA CTTCTTTCTG GAGGGACCGT 60

TCATGCCACC CAGGATGAAA ATGGATAGGG ACCCACTTGG AGGACTTGCT GATATGTTTG 120

GACAAATGCC AGGTAGCGGA ATTGGTACTG GTCCAGGAGT TATCCAGGAT AGATTTTCAC 180

CCACCATGGG ACGTCATCGT TCAAATCAAC TCTTCAATGG CCATGGGGGA CACATCATGC 240

CTCCCACACA ATCGCAGTTT GGAGAGATGG GAGGCAAGTT TATGAAAAGC CAGGGGCTAA 300

GCCAGCTCTA CCATAACCAG AGTCAGGGAC TCTTATCCCA GCTGCAAGGA CAGTCGAAGG 360

ATATGCCACC TCGGTTTTCT AAGAAAGGAC AGCTTAATGC AGATGAGATT AGCCTGAGGA 420

GGCATCACCC GA 432

(2) INFORMATION FOR SEQ ID NO:177:

(i) SEQUENCE CHARACTERISTICS:
(A) LENGTH: 788 base pairs
(B) TYPE: nucleic acid
(C) STRANDEDNESS: single
(D) TOPOLOGY: linear

(xi) SEQUENCE DESCRIPTION: SEQ ID NO:177:

TAGCATGTTG AGCCCAGACA CAGTAGCATT TGTGCCAATT TCTGGTTGGA ATGGTGACAA 60

CATGCTGGAG CCAAGTGCTA ACATGCCTTG GTTCAAGGGA TGGAAAGTCA CCCGTAAGGA 120

TGGCAATGCC AGTGGAACCA CGCTGCTTGA GGCTCTGGAC TGCATCCTAC CACCAACTCG 180

CCCAACTGAC AAGCCCTTGC GCCTGCCTCT CCAGGATGTC TACAAAATTG GTGGTATTGG 240

TACTGTTCCT GTTGGCCGAG TGGAGACTGG TGTTCTCAAA CCCGGTATGG TGGTCACCTT 300

TGCTCCAGTC AACGTTACAA CGGAAGTAAA ATCTGTCGAA ATGCACCATG AAGCTTTGAG 360

TGAAGCTCTT CCTGGGGACA ATGTGGGCTT CAATGTCAAG AATGTGTCTG TCAAGGATGT 420

TCGTCGTGGC AACGTTGCTG GTGACAGCAA AAATGACCCA CCAATGGAAG CAGCTGGCTT 480

CACTGCTCAG GTGATTATCC TGAACCATCC AGGCCAAATA AGTGCCGGCT ATGCCCCTGT 540

ATTGGATTGC CACACGGCTC ACATTGCATG CAAGTTTGCT GAGCTGAAGG AAAAGATTGA 600

TCGCCGTTCT GGTAAAAAGC TGGAAGATGG CCCTAAATTC TTGAAGTCTG GTGATGCTGC 660

CATTGTTGAT ATGGTTCCTG GCAAGCCCAT GTGTGTTGAG AGCTTCTCAG ACTATCCACC 720

TTTGGGTCGC TTTGCTGTTC GTGATATGAG ACAGACAGTT GCGGTGGGTG TCTGGGCTCA 780

ACATGCTA 788

(2) INFORMATION FOR SEQ ID NO:178:

(i) SEQUENCE CHARACTERISTICS:
(A) LENGTH: 786 base pairs
(B) TYPE: nucleic acid
(C) STRANDEDNESS: single
(D) TOPOLOGY: linear

(xi) SEQUENCE DESCRIPTION: SEQ ID NO:178:

TAGCATGTTG AGCCCAGACA CCTGTGTTTC TGGGAGCTCT GGCAGTGGCG GATTCATAGG 60

CACTTGGGCT GCACTTTGAA TGACACACTT GGCTTTATTA GATTCACTAG TTTTTAAAAA 120

ATTGTTGTTC GTTTCTTTTC ATTAAAGGTT TAATCAGACA GATCAGACAG CATAATTTTG 180

TATTTAATGA CAGAAACGTT GGTACATTTC TTCATGAATG AGCTTGCATT CTGAAGCAAG 240

AGCCTACAAA AGGCACTTGT TATAAATGAA AGTTCTGGCT CTAGAGGCCA GTACTCTGGA 300

GTTTCAGAGC AGCCAGTGAT TGTTCCAGTC AGTGATGCCT AGTTATATAG AGGAGGAGTA 360

CACTGTGCAC TCTTCTAGGT GTAAGGGTAT GCAACTTTGG ATCTTAAAAT TCTGTACACA 420

TACACACTTT ATATATATGT ATGTATGTAT GAAAACATGA AATTAGTTTG TCAAATATGT 480

GTGTGTTTAG TATTTTAGCT TAGTGCAACT ATTTCCACAT TATTTATTAA ATTGATCTAA 540

GACACTTTCT TGTTGACACC TTGAATATTA ATGTTCAAGG GTGCAATGTG TATTCCTTTA 600

GATTGTTAAA GCTTAATTAC TATGATTTGT AGTAAATTAA CTTTTAAAAT GTATTTGAGC 660

CCTTCTGTAG TGTCGTAGGG CTCTTACAGG GTGGGAAAGA TTTTAATTTT CCAGTTGCTA 720

ATTGAACAGT ATGGCCTCAT TATATATTTT GATTTATAGG AGTTTGTGTC TGGGCTCAAC 780

ATGCTA 786

(2) INFORMATION FOR SEQ ID NO:179:

(i) SEQUENCE CHARACTERISTICS:
(A) LENGTH: 796 base pairs
(B) TYPE: nucleic acid
(C) STRANDEDNESS: single
(D) TOPOLOGY: linear

(xi) SEQUENCE DESCRIPTION: SEQ ID NO:179:

TAGCATGTTG AGCCCAGACA CTGGTTACAA GACCAGACCT GCTTCCTCCA TATGTAAACA 60

GCTTTTAAAA AGCCAGTGAA CCTTTTTAAT ACTTTGGCAA CCTTCTTTCA CAGGCAAAGA 120

ACACCCCCAT CCGCCCCTTG TTTGGAGTGC AGAGTTTGGC TTTGGTTCTT TGCCTTGCCT 180

GGAGTATACT TCTAATTCCT GTTGTCCTGC ACAAGCTGAA TACCGAGCTA CCCACCGCCA 240

CCCAGGCCAG GTTTCCACTC ATTTATTACT TTATGTTTCT GTTCCATTGC TGGTCCACAG 300

AAATAAGTTT TCCTTTGGAG GAATGTGATT ATACCCCTTT AATTTCCTCC TTTTGCTTTT 360

TTTTAATATC ATTGGTATGT GTTTGGCCCA GAGGAAACTG AAATTCACCA TCATCTTGAC 420

TGGCAATCCC ATTACCATGC TTTTTTTAAA AAACGTAATT TTTCTTGCCT TACATTGGCA 480

GAGTAGCCCT TCCTGGCTAC TGGCTTAATG TAGTCACTCA GTTTCTAGGT GGCATTAGGC 540

ATGAGACCTG AAGCACAGAC TGTCTTACCA CAAAAGGTGA CAAGATCTCA AACCTTAGCC 600

AAAGGGCTAT GTCAGGTTTC AATGCTATCT GCTTCTGTTC CTGCTCACTG TTCTGGATTT 660

TGTCCTTCTT CATCCCTAGC ACCAGAATTT CCCAGTCTCC CTCCCTACCT TCCCTTGTTT 720

TAATTCTAAT CTATCAGCAA AATAACTTTT CAAATGTTTT AACCGGTATC TCCATGTGTC 780

TGGGCTCAAC ATGCTA 796

(2) INFORMATION FOR SEQ ID NO:180:

(i) SEQUENCE CHARACTERISTICS:
(A) LENGTH: 488 base pairs
(B) TYPE: nucleic acid
(C) STRANDEDNESS: single
(D) TOPOLOGY: linear

(xi) SEQUENCE DESCRIPTION: SEQ ID NO:180:

GGATGTGCTG CAAGGCGATT AAGTTGGGTA ACGCCAGGGT TTTCCCAGTC ACGACGTTGT 60

AAAACGACGG CCAGTGAATT GTAATACGAC TCACTATAGG GCGAATTGGG CCCGACGTCG 120

CATGCTCCCG GCCGCCATGG CCGCGGGATA GCATGTTGAG CCCAGACACC TGCAGGTCAT 180

TTGGAGAGAT TTTTCACGTT ACCAGCTTGA TGGTCTTTTT CAGGAGGAGA GACACTGAGC 240

ACTCCCAAGG TGAGGTTGAA GATTTCCTCT AGATAGCCGG ATAAGAAGAC TAGGAGGGAT 300

GCCTAGAAAA TGATTAGCAT GCAAATTTCT ACCTGCCATT TCAGAACTGT GTGTCAGCCC 360

ACATTCAGCT GCTTCTTGTG AACTGAAAAG AGAGAGGTAT TGAGACTTTT CTGATGGCCG 420

CTCTAACATT GTAACACAGT AATCTGTGTG TGTGTGGGTG TGTGTGTGTG TCTGGGCTCA 480

ACATGCTA 488

(2) INFORMATION FOR SEQ ID NO:181:

(i) SEQUENCE CHARACTERISTICS:
(A) LENGTH: 317 base pairs
(B) TYPE: nucleic acid
(C) STRANDEDNESS: single
(D) TOPOLOGY: linear

(xi) SEQUENCE DESCRIPTION: SEQ ID NO:181:

TAGCATGTTG AGCCCAGACA CGGCGACGGT ACCTGATGAG TGGGGTGATG GCACCTGTGA 60

AAAGGAGGAA CGTCATCCCC CATGATATTG GGGACCCAGA TGATGAACCA TGGCTCCGCG 120

TCAATGCATA TTTAATCCAT GATACTGCTG ATTGGAAGGA CCTGAACCTG AAGTTTGTGC 180

TGCAGGTTTA TCGGGACTAT TACCTCACGG GTGATCAAAA CTTCCTGAAG GACATGTGGC 240

CTGTGTGTCT AGTAAGGGAT GCACATGCAG TGGCCAGTGT GCCAGGGGTA TGGTTGGTGT 300

CTGGGCTCAA CATGCTA 317

(2) INFORMATION FOR SEQ ID NO:182:

(i) SEQUENCE CHARACTERISTICS:
(A) LENGTH: 507 base pairs
(B) TYPE: nucleic acid
(C) STRANDEDNESS: single
(D) TOPOLOGY: linear

(xi) SEQUENCE DESCRIPTION: SEQ ID NO:182:

TAGCATGTTG AGCCCAGACA CTGGCTGTTA GCCAAATCCT CTCTCAGCTG CTCCCTGTGG 60

TTTGGTGACT CAGGATTACA GAGGCATCCT GTTTCAGGGA ACAAAAAGAT TTTAGCTGCC 120

AGCAGAGAGC ACCACATACA TTAGAATGGT AAGGACTGCC ACCTCCTTCA AGAACAGGAG 180

TGAGGGTGGT GGTGAATGGG AATGGAAGCC TGCATTCCCT GATGCATTTG TGCTCTCTCA 240

AATCCTGTCT TAGTCTTAGG AAAGGAAGTA AAGTTTCAAG GACGGTTCCG AACTGCTTTT 300

TGTGTCTGGG CTCAACATGC TATCCCGCGG CCATGGCGGC CGGGAGCATG CGACGTCGGG 360

CCCAATTCGC CCTATAGTGA GTCGTATTAC AATTCACTGG CCGTCGTTTT ACAACGTCGT 420

GACTGGGAAA ACCCTGGCGT TACCCAACTT AATCGCCTTG CAGCACATCC CCCTTTCCCA 480

GCTGGCGTAA TANCGAAAAG GCCCGCA 507

(2) INFORMATION FOR SEQ ID NO:183:

(i) SEQUENCE CHARACTERISTICS:
(A) LENGTH: 227 base pairs
(B) TYPE: nucleic acid
(C) STRANDEDNESS: single
(D) TOPOLOGY: linear

(xi) SEQUENCE DESCRIPTION: SEQ ID NO:183:

GATTTACGCT GCAACACTGT GGAGGTAGCC CTGGAGCAAG GCAGGCATGG ATGCTTCTGC 60

AATCCCCAAA TGGAGCCTGG TATTTCAGCC AGGAATCTGA GCAGAGCCCC CTCTAATTGT 120

AGCAATGATA AGTTATTCTC TTTGTTCTTC AACCTTCCAA TAGCCTTGAG CTTCCAGGGG 180

AGTGTCGTTA ATCATTACAG CCTGGTCTCC ACAGTGTTGC AGCGTAA 227

(2) INFORMATION FOR SEQ ID NO:184:

(i) SEQUENCE CHARACTERISTICS:
(A) LENGTH: 225 base pairs
(B) TYPE: nucleic acid
(C) STRANDEDNESS: single
(D) TOPOLOGY: linear

(xi) SEQUENCE DESCRIPTION: SEQ ID NO:184:

TTACGCTGCA ACACTGTGGA GCAGATTAAC ATCAGACTTT TCTATCAACA TGACTGGGGT 60

TACTAAAAAG ACAACAAATC AATGGCTTCA AAAGTCTAAG GAATAATTTC GATACTTCAA 120

CTTTATAAAA CCTGACAAAA CTATCAATCA AGCATAAAGA CAGATGAAGA ACATTTCCAG 180

ATTTTGGCCA ATCAGATATT TTACCTCCAC AGTGTTGCAG CGTAA 225

(2) INFORMATION FOR SEQ ID NO:185:

(i) SEQUENCE CHARACTERISTICS:
(A) LENGTH: 597 base pairs
(B) TYPE: nucleic acid
(C) STRANDEDNESS: single
(D) TOPOLOGY: linear

(xi) SEQUENCE DESCRIPTION: SEQ ID NO:185:

GGCCCGACGT CGCATGCTCC CGGCCGCCAT GGCCGCGGGA TTCGTTAGGG TCTCTATCCA 60

CTGGGACCCA TAGGCTAGTC AGAGTATTTA GAGTTGAGTT CCTTTCTGCT TCCCAGAATT 120

TGAAAGAAAA GGAGTGAGGT GATAGAGCTG AGAGATCAGA TTTGCCTCTG AAGCCTGTTC 180

AAGATGTATG TGCTCAGACC CCACCACTGG GGCCTGTGGG TGAGGTCCTG GGCATCTATT 240

TGAATGAATT GCTGAAGGGG AGCACTATGC CAAGGAAGGG GAACCCATCC TGGCACTGGC 300

ACAGGGGTCA CCTTATCCAG TGCTCAGTGC TTCTTTGCTG CTACCTGGTT TTCTCTCATA 360

TGTGAGGGGC AGGTAAGAAG AAGTGCCCRG TGTTGTGCGA GTTTTAGAAC ATCTACCAGT 420

AAGTGGGGAA GTTTCACAAA GCAGCAGCTT TGTTTTGTGT ATTTTCACCT TCAGTTAGAA 480

GAGGAAGGCT GTGAGATGAA TGTTAGTTGA GTGGAAAAGA CGGGTAAGCT TAGTGGATAG 540

AGACCCTAAC GAATCACTAG TGCGGCCGCC TTGCAGGTCG ACCATATGGG AGAGCTC 597

(2) INFORMATION FOR SEQ ID NO:186:

(i) SEQUENCE CHARACTERISTICS:
(A) LENGTH: 597 base pairs
(B) TYPE: nucleic acid
(C) STRANDEDNESS: single
(D) TOPOLOGY: linear

(xi) SEQUENCE DESCRIPTION: SEQ ID NO:186:

GGCCCGAAGT TGCATGTTCC CGGCCGCCAT GGCCGCGGGA TTCGTTAGGG TCTCTATCCA 60

CTACCTAAAA AATCCCAAAC ATATAACTGA ACTCCTCACA CCCAATTGGA CCAATCCATC 120

ACCCCAGAGG CCTACAGATC CTCCTTTGAT ACATAAGAAA ATTTCCCCAA ACTACCTAAC 180

TATATCATTT TGCAAGATTT GTTTTACCAA ATTTTGATGG CCTTTCTGAG CTTGTCAGTG 240

TGAACCACTA TTACGAACGA TCGGATATTA ACTGCCCCTC ACCGTCCAGG TGTAGCTGGC 300

AACATCAAGT GCAGTAAATA TTCATTAAGT TTTCACCTAC TAAGGTGCTT AAACACCCTA 360

GGGTGCCATG TCGGTAGCAG ATCTTTTGAT TTGTTTTTAT TTCCCATAAG GGTCCTGTTC 420

AAGGTCAATC ATACATGTAG TGTGAGCAGC TAGTCACTAT CGCATGACTT GGAGGGTGAT 480

AATAGAGGCC TCCTTTGCTG TTAAAGAACT CTTGTCCCAG CCTGTCAAAG TGGATAGAGA 540

CCCTAACGAA TCACTAGTGC GGCCGCCTGC AGGTCGACCA TATGGGAGAG CTCCCAA 597

(2) INFORMATION FOR SEQ ID NO:187:

(i) SEQUENCE CHARACTERISTICS:
(A) LENGTH: 324 base pairs
(B) TYPE: nucleic acid
(C) STRANDEDNESS: single
(D) TOPOLOGY: linear

(xi) SEQUENCE DESCRIPTION: SEQ ID NO:187:

TCGTTAGGGT CTCTATCCAC TTGCAGGTAA AATCCAATCC TGTGTATATC TTATAGTCTT 60

CCATATGTAG TGGTTCAAGA GACTGCAGTT CCAGAAAGAC TAGCCGAGCC CATCCATGTC 120

TTCCACTTAA CCCTGCTTTG GGTTACACAT CTTAACTTTT CTGTTCAAGT TTCTCTGTGT 180

AGTTTATAGC ATGAGTATTG GGAWAATGCC CTGAAACCTG ACATGAGATC TGGGAAACAC 240

AAACTTACTC AATAAGAATT TCTCCCATAT TTTTATGATG GAAAAATTTC ACATGCACAG 300

AGGAGTGGAT AGAGACCCTA ACGA 324

(2) INFORMATION FOR SEQ ID NO:188:

(i) SEQUENCE CHARACTERISTICS:
(A) LENGTH: 178 base pairs
(B) TYPE: nucleic acid
(C) STRANDEDNESS: single
(D) TOPOLOGY: linear

(xi) SEQUENCE DESCRIPTION: SEQ ID NO:188:

GCGCGGGGAT TCGGGGTGAT ACCTCCTCAT GCCAAAATAC AACGTNTAAT TTCACAACTT 60

GCCTTCCAAT TTACGCATTT TCAATTTGCT CTCCCCATTT GTTGAGTCAC AACAAACACC 120

ATTGCCCAGA AACATGTATT ACCTAACATG CACATACTCT TAAAACTACT CATCCCTT 178

(2) INFORMATION FOR SEQ ID NO:189:

(i) SEQUENCE CHARACTERISTICS:
(A) LENGTH: 367 base pairs
(B) TYPE: nucleic acid
(C) STRANDEDNESS: single
(D) TOPOLOGY: linear

(xi) SEQUENCE DESCRIPTION: SEQ ID NO:189:

TGACACCTTG TCCAGCATCT GACACAGTCT TGGCTCTTGG AAAATATTGG ATAAATGAAA 60

ATGAATTTCT TTAGCAAGTG GTATAAGCTG AGAATATACG TATCACATAT CCTCATTCTA 120

AGACACATTC AGTGTCCCTG AAATTAGAAT AGGACTTACA ATAAGTGTGT TCACTTTCTC 180

AATAGCTGTT ATTCAATTGA TGGTAGGCCT TAAAAGTCAA AGAAATGAGA GGGCATGTGA 240

AAAAAAGCTC AACATCACTG ATCATTAGAA AACTTCCATT CAAACCCCCA ATGAGATACC 300

ATCTCATACC AGTCAGAATG GCTATTATTA AAAAGTCAAA AAATAACAGA TGCTGGACAA 360

GGTGTCA 367

(2) INFORMATION FOR SEQ ID NO:190:

(i) SEQUENCE CHARACTERISTICS:
(A) LENGTH: 369 base pairs
(B) TYPE: nucleic acid
(C) STRANDEDNESS: single
(D) TOPOLOGY: linear

(xi) SEQUENCE DESCRIPTION: SEQ ID NO:190:

GACACCTTGT CCAGCATCTG ACAACGCTAA CAGCCTGAGG AGATCTTTAT TTATTTATTT 60

AGTTTTTACT CTGGCTAGGC AGATGGTGGC TAAAACATTC ATTTACCCAT TTATTCATTT 120

AATTGTTCCT GCAAGGCCTA TGGATAGAGT ATTGTCCAGC ACTGCTCTGG AAGCTAGGAG 180

CATGGGGATG AACAAGATAG GCTACATCCT GTTCCCACAG AACTTCCACT TTAGTCTGGG 240

AAACAGATGA TATATACAAA TATATAAATG AATTCAGGTA GTTTTAAGTA CGAAAAGAAT 300

AAGAAAGCAG AGTCATGATT TANAATGCTG GAAACAGGGG CTATTGCTTG AGATATTGAA 360

GGTGCCCAA 369

(2) INFORMATION FOR SEQ ID NO:191:

(i) SEQUENCE CHARACTERISTICS:
(A) LENGTH: 369 base pairs

(B) TYPE: nucleic acid
(C) STRANDEDNESS: single
(D) TOPOLOGY: linear

(xi) SEQUENCE DESCRIPTION: SEQ ID NO:191:

TGACACCTTG TCCAGCATCT GCACAGGGAA AAGAAACTAT TATCAGAGTG AACAGGCAAC 60

CTACAGAATG GGAGAAAATT TTTGCAATCT ATCCATCTGA CAAAGGGCTA ATATCCAGAA 120

TCTACAAAGA ACTTATACAA ATTTACAAGA AACAAACAAA CAAACAACTC CTCAAAAAGT 180

GGGTGAAGGA TGTGAACAGA CACTTCTCAA AAGAAGACAT TTATGGGGCC AACAAACATA 240

TGAAAAAAAG CTCATCATCA CTGGTCACTA GATAAATGCA AATCAAAACC ACAATGAGAT 300

ACCATCTCAT TCCAGTTAGA ATGGCAATCA TTAAAAAGTC AGGAAACAAC AGATGCTGGA 360

CAAGGTGTC 369

(2) INFORMATION FOR SEQ ID NO:192:

(i) SEQUENCE CHARACTERISTICS:
(A) LENGTH: 449 base pairs
(B) TYPE: nucleic acid
(C) STRANDEDNESS: single
(D) TOPOLOGY: linear

(xi) SEQUENCE DESCRIPTION: SEQ ID NO:192:

TGACGCTTGG CCACTTGACA CTTCATCTTT GCACAGAAAA ACTTCTTTAC AGATTTAATT 60

CAAGACTGGT CTAGTGACAG TCCTCCAGAC ATTTTTTCAT TTGTTCCATA TACGTGGAAT 120

TTTAAAATCA TGTTTCATCA GTTTGAAATG ATTTGGGCTG CTAATCAACA CAATTGGATC 180

GACTGTTCTA CTAAACAACA GGAAAATGTG TATCTGGCAG CCTGTGGAGA AACACTAAAC 240

ATTGATTTTT CTTTGCCTTT TACGGACTTT GTTCCAGCTA CATGTAATAC CAAGTTCTCT 300

TTAAGAGGAG AAGATGTTGA TCTTCATTTG TTTCTACCAG ACTGCCACCC TAGTAAATAT 360

TCTTTATTTA TGCTGGTAAA AAATTGCCAT CCAAATAAGA TGATTCATGA TACTGGTATT 420

CCTGCTGAGT GTCAAGTGGC CAAGCGTCA 449

(2) INFORMATION FOR SEQ ID NO:193:

(i) SEQUENCE CHARACTERISTICS:
(A) LENGTH: 372 base pairs
(B) TYPE: nucleic acid
(C) STRANDEDNESS: single
(D) TOPOLOGY: linear

(xi) SEQUENCE DESCRIPTION: SEQ ID NO:193:

TGACGCTTGG CCACTTGACA CCAGGGATGT AKCAGTTGAA TATAATCCTG CAATTGTACA 60

TATTGGCAAT TTCCCATCAA ACATTCTAGA AAGAGACAAC CAGGATTGCT AGGCCATAAA 120

AGCTGCAATA AATAACTGGT AATTGCAGTA ATCATTTCAG GCCAATTCAA TCCAGTTTGG 180

CTCAGAGGTG CCTTTGGCTG AGAGAAGAGG TGAGATATAA TGTGTTTTCT TGCAACTTCT 240

TGGAAGAATA ACTCCACAAT AGTCTGAGGA CTAGATACAA ACCTATTTGC CATTAAAGCA 300

CCAGAGTCTG TTAATTCCAG TACTGATAAG TGTTGGAGAT TAGACTCCAG TGTGTCAAGT 360

GGCCAAGCGT CA 372

(2) INFORMATION FOR SEQ ID NO:194:

(i) SEQUENCE CHARACTERISTICS:
(A) LENGTH: 309 base pairs
(B) TYPE: nucleic acid
(C) STRANDEDNESS: single
(D) TOPOLOGY: linear

(xi) SEQUENCE DESCRIPTION: SEQ ID NO:194:

TGACGCTTGG CCACTTGACA CTTATGTAGA ATCCATCGTG GGCTGATGCA AGCCCTTTAT 60

TTAGGCTTAG TGTTGTGGGC ACCTTCAATA TCACACTAGA GACAAACGCC ACAAGATCTG 120

CAGAAACATT CAGTTCTGAN CACTCGAATG GCAGGATAAC TTTTTGTGTT GTAATCCTTC 180

ACATATACAA AAACAAACTC TGCANTCTCA CGTTACAAAA AAACGTACTG CTGTAAAATA 240

TTAAGAAGGG GTAAAGGATA CCATCTATAA CAAAGTAACT TACAACTAGT GTCAAGTGGC 300

CAAGCGTCA 309

(2) INFORMATION FOR SEQ ID NO:195:

(i) SEQUENCE CHARACTERISTICS:
(A) LENGTH: 312 base pairs
(B) TYPE: nucleic acid
(C) STRANDEDNESS: single
(D) TOPOLOGY: linear

(xi) SEQUENCE DESCRIPTION: SEQ ID NO:195:

TGACGCTTGG CCACTTGACA CCCAATCTCG CACTTCATCC TCCCAGCACC TGATGAAGTA 60

GGACTGCAAC TATCCCCACT TCCCAGATGA GGGGACCAAN GTACACATTA GGACCCGGAT 120

GGGAGCACAG ATTTGTCCGA TCCCAGACTC CAAGCACTCA GCGTCACTCC AGGACAGCGG 180

CTTTCAGATA AGGTCACAAA CATGAATGGC TCCGACAACC GGAGTCAGTC CGTGCTGAGT 240

TAAGGCAATG GTGACACGGA TGCACGTGTN ACCTGTAATG GTTCATCGTA AGTGTCAAGT 300

GGCCAAGCGT CA 312

(2) INFORMATION FOR SEQ ID NO:196:

(i) SEQUENCE CHARACTERISTICS:
(A) LENGTH: 288 base pairs
(B) TYPE: nucleic acid
(C) STRANDEDNESS: single
(D) TOPOLOGY: linear

(xi) SEQUENCE DESCRIPTION: SEQ ID NO:196:

TGTATCGACG TAGTGGTCTC CTCAGCCATG CAGAACTGTG ACTCAATTAA ACCTCTTTCC 60

TTTATGAATT ACCCAATCTC GGGTAGTGTC TTTATAGTAG TGTGAGAATG GACTAATACA 120

AGTACATTTT ACTTAGTAAT AATAATAAAC AAATATATTA CATTTTTGTG TATTTACTAC 180

ACCATATTTT TTATTGTTAT TGTAGTGTAC ACCTTCTACT TATTAAAAGA AATAGGCCCG 240

AGGCGGGCAG ATCACGAGGT CAGGAGATGG AGACCACTAC GTCGATAC 288

(2) INFORMATION FOR SEQ ID NO:197:

(i) SEQUENCE CHARACTERISTICS:
(A) LENGTH: 289 base pairs

(B) TYPE: nucleic acid
(C) STRANDEDNESS: single
(D) TOPOLOGY: linear

(xi) SEQUENCE DESCRIPTION: SEQ ID NO:197:

TTGGGCACCT TCAATATCAT GACAGGTGAT GTGATAACCA AGAAGGCTAC TAAGTGATTA 60

ATGGGTGGGT AATGTATACA GAGTAGGTAC ACTGGACAGA GGGGTAATTC ATAGCCAAGG 120

CAGGAGAAGC AGAATGGCAA AACATTTCAT CACACTACTC AGGATAGCAT GCAGTTTAAA 180

ACCTATAAGT AGTTTATTTT TGGAATTTTC CACTTAATAT TTTCAGACTG CAGGTAACTA 240

AACTGTGGAA CACAAGAACA TAGATAAGGG GAGACCACTA CGTCGATAC 289

(2) INFORMATION FOR SEQ ID NO:198:

(i) SEQUENCE CHARACTERISTICS:
(A) LENGTH: 288 base pairs
(B) TYPE: nucleic acid
(C) STRANDEDNESS: single
(D) TOPOLOGY: linear

(xi) SEQUENCE DESCRIPTION: SEQ ID NO:198:

GTATCGACGT AGTGGTCTCC CAAGCAGTGG GAAGAAAACG TGAACCAATT AAAATGTATC 60

AGATACCCCA AAGAAAGGCG CTTGAGTAAA GATTCCAAGT GGGTCACAAT CTCAGATCTT 120

AAAATTCAGG CTGTCAAAGA GATTTGCTAT GAGGTTGCTC TCAATGACTT CAGGCACAGT 180

CGGCAGGAGA TTGAAGCCCT GGCCATTGTC AAGATGAAGG AGCTTTGTGC CATGTATGGC 240

AAGAAAGACC CCAATGAGCG GGACTCCTGG AGACCACTAC GTCGATAC 288

(2) INFORMATION FOR SEQ ID NO:199:

(i) SEQUENCE CHARACTERISTICS:
(A) LENGTH: 1027 base pairs
(B) TYPE: nucleic acid
(C) STRANDEDNESS: single
(D) TOPOLOGY: linear

(xi) SEQUENCE DESCRIPTION: SEQ ID NO:199:
   GCTTTTTGGG AAAAACNCAA NTGGGGGAAA GGGGGNTTNN TNGCAAGGGG ATAAAGGGGG 60
   AANCCCAGGG TTTCCCCATT CAGGGAGGTG TAAAAAGNCG GCCAGGGGAT TGTAANAGGA 120
   TTCAATAATA GGGGGAATGG GCCCNGAAGT TGCAAGGTTC CNGCCCGCCA TGNCCGCGGG 180
   ATTTAGTGAC ATTACGACGS TGGTAATAAA GTGGGSCCAA WAAATATTTG TGATGTGATT 240
   TTTSGACCAG TGAACCCATT GWACAGGACC TCATTTCCTY TGAGATGRTA GCCATAATCA 300
   GATAAAAGRT TAGAAGTYTT TCTGCACGTT AACAGCATCA TTAAATGGAG TGGCATCACC 360
   AATTTCACCC TTTGTTAGCC GATACCTTCC CCTTGAAGGC ATTCAATTAA GTGACCAATC 420
   GTCATACGAG AGGGGATGGC ATGGGGATTG ATGATGATAT CAGGGGTGAT ACCTTCACAG 480
   GTGAAAGGCA TATCCTCTTG TCTATACTGA ATACCACAAG TACCCTTTTG ACCATGTCGA 540
   CTAGCAAATT TGTCTCCAAT CTGTGTWATC CCTAACAGAG CGTACCCTTA TTTTACAAAA 600
   TTTATATCCT TCCTGATTGA GAGTTACCAT AACCTGATCC ACAATGCCCG TCTCGCTWGT 660
   TCTGAGAAAA GTGCTACAGT CTCTCTTGGT ATAGCGTCTA TTGGTGCTCT CCAATTCATC 720
   TTCATTTTTC AGGCAAGGTG AACTGTTTTG CCTATAATAA CMTCATCTCC TGATACMCGA 780
   AACCCCKGGA RCTATCAAAC CATCATCATC CAGCGTTCKT WATGTYMCTA AATCCCTATT 840
   GCGGCCGCCT GCAGGTCAAC ATATNGGAAA ACCCCCCACC CCTTNGGAGC NTACCTTGAA 900
   TTTTCCATAT GTCCCNTAAA TTANCTNGNC TTANCCTGGC CNTAACCTNT TCCGGTTTAA 960
   ATTGTTTCCG CCCCCNTTCC CCNCCTTNNA ACCGGAAACC TTAATTTTNA ACCNGGGGTT 1020
   CCTATCC 1027

(2) INFORMATION FOR SEQ ID NO:200:

(i) SEQUENCE CHARACTERISTICS:
(A) LENGTH: 207 base pairs
(B) TYPE: nucleic acid
(C) STRANDEDNESS: single
(D) TOPOLOGY: linear

(xi) SEQUENCE DESCRIPTION: SEQ ID NO:200:

AGTGACATTA CGACGCTGGC CATCTTGAAT CCTAGGGCAT GAAGTTGCCC CAAAGTTCAG 60

CACTTGGTTA AGCCTGATCC CTCTGGTTTA TCACAAAGAA TAGGATGGGA TAAAGAAAGT 120

GGACACTTAA ATAAGCTATA AATTATATGG TCCTTGTCTA GCAGGAGACA ACTGCACAGG 180

TATACTACCA GCGTCGTAAT GTCACTA 207

(2) INFORMATION FOR SEQ ID NO:201:

(i) SEQUENCE CHARACTERISTICS:
(A) LENGTH: 209 base pairs

(B) TYPE: nucleic acid
(C) STRANDEDNESS: single
(D) TOPOLOGY: linear

(xi) SEQUENCE DESCRIPTION: SEQ ID NO:201:

TGGGCACCTT CAATATCTAT TAAAAGCACA AATACTGAAG AACACACCAA GACTATCAAT 60

GAGGTTACAT CTGGAGTCCT CGATATATCA GGAAAAAATG AAGTGAACAT TCACAGAGTT 120

TTACTTCTTT GGGAACTCAA ATGCTAGAAA AGAAAAGGGT GCCCTCTTTC TCTGGCTTCC 180

TGGTCCTATC CAGCGTCGTA ATGTCACTA 209

(2) INFORMATION FOR SEQ ID NO:202:

(i) SEQUENCE CHARACTERISTICS:
(A) LENGTH: 349 base pairs
(B) TYPE: nucleic acid
(C) STRANDEDNESS: single
(D) TOPOLOGY: linear

(xi) SEQUENCE DESCRIPTION: SEQ ID NO:202:

NTACGCTGCA ACACTGTGGA GCCACTGGTT TTTATTCCCG GCAGGTTATC CAGCAAACAG 60

TCACTGAACA CACCGAAGAC CGTGGTATGG TAACCGTTCA CAGTAATCGT TCCAGTCGTC 120

TGCGGGACCC CGACGAGCGT CACTGGGTAC AGACCAGATT CAGCCGGAAG AGAAAGCGCC 180

GCAGGGAGAG ACTCGAACTC CACTCCGCTG GTGAGCAGCC CCATGTTTTC AACTCGAAGT 240

TCAAACGGCA TTGGGTTATA TACCATCAGC TGAACTTCAC ACACATCTCC TTGAACCCAC 300

TGGAAATCTA TTTTCTTGTT CCGCTCTTCT CCACAGTGTT GCAGCGTAA 349

(2) INFORMATION FOR SEQ ID NO:203:

(i) SEQUENCE CHARACTERISTICS:
(A) LENGTH: 241 base pairs
(B) TYPE: nucleic acid
(C) STRANDEDNESS: single
(D) TOPOLOGY: linear

(xi) SEQUENCE DESCRIPTION: SEQ ID NO:203:

TGCTCCTCTT GCCTTACCAA CCCAAAGCCC ACTGTGAAAT ATGAAGTGAA TGACAAAATT 60

CAGTTTTCAA CGCAATATAG TATAGTTTAT CTGATTCTTT TGATCTCCAG GACACTTTAA 120

ACAACTGCTA CCACCACCAC CAACCTAGGG ATTTAGGATT CTCCACAGAC CAGAAATTAT 180

TTCTCCTTTG AGTTTCAGGC TCCTCTGGGA CTCCTGTTCA TCAATGGGTG GTAAATGGCT 240

A 241

(2) INFORMATION FOR SEQ ID NO:204:

(i) SEQUENCE CHARACTERISTICS:
(A) LENGTH: 248 base pairs
(B) TYPE: nucleic acid
(C) STRANDEDNESS: single
(D) TOPOLOGY: linear

(xi) SEQUENCE DESCRIPTION: SEQ ID NO:204:

TAGCCATTTA CCACCCATCT GCAAACCSWG ACMWWCARGR CYWGWACKYA GGCGATTTGA 60

AGTACTGGTA ATGCTCTGAT CATGTTAGTT ACATAAGTGT GGTCAGTTTA CAAAAATTCA 120

CAGAACTAAA TACTCAATGC TATGTGTTCA TGTCTGTGTT TATGTGTGTG TAATGTTTCA 180

ATTAAGTTTT TTTAAAAAAA AGAGATGATT TCCAAATAAG AAAGCCGTGT TGGTAAGGCA 240

AGAGGAGC 248

(2) INFORMATION FOR SEQ ID NO:205:

(i) SEQUENCE CHARACTERISTICS:
(A) LENGTH: 505 base pairs
(B) TYPE: nucleic acid
(C) STRANDEDNESS: single
(D) TOPOLOGY: linear

(xi) SEQUENCE DESCRIPTION: SEQ ID NO:205:

TACGCTGCAA CACTGTGGAG CCATTCATAC AGGTCCCTAA TTAAGGAACA AGTGATTATG 60

CTACCTTTGC ACGGTTAGGG TACCGCGGCC GTTAAACATG TGTCACTGGG CAGGCGGTGC 120

CTCTAATACT GGTGATGCTA GAGGTGATGT TTTTGGTAAA CAGGCGGGGT AAGATTTGCC 180

GAGTTCCTTT TACTTTTTTT AACCTTTCCT TATGAGCATG CCTGTGTTGG GTTGACAGTG 240

GGGGTAATAA TGACTTGTTG GTTGATTGTA GATATTGGGC TGTTAATTGT CAGTTCAGTG 300

TTTTAATCTG ACGCAGGCTT ATGCGGAGGA GAATGTTTTC ATGTTACTTA TACTAACATT 360

AGTTCTTCTA TAGGGTGATA GATTGGTCCA ATTGGGTGTG AGGAGTTCAG TTATATGTTT 420

GGGATTTTTT AGGTAGTGGG TGTTGANCTT GAACGCTTTC TTAATTGGTG GCTGCTTTTA 480

RGCCTACTAT GGGTGGTAAA TGGCT 505

(2) INFORMATION FOR SEQ ID NO:206:

(i) SEQUENCE CHARACTERISTICS:
(A) LENGTH: 179 base pairs
(B) TYPE: nucleic acid
(C) STRANDEDNESS: single
(D) TOPOLOGY: linear

(xi) SEQUENCE DESCRIPTION: SEQ ID NO:206:

TAGACTGACT CATGTCCCCT ACCAAAGCCC ATGTAAGGAG CTGAGTTCTT AAAGACTGAA 60

GACAGACTAT TCTCTGGAGA AAAATAAAAT GGAAATTGTA CTTTAAAAAA AAAAAAAATC 120

GGCCGGGCAT GGTAGCACAC ACCTGTAATC CCAGCTACTA GGGGACATGA GTCAGTCTA 179

(2) INFORMATION FOR SEQ ID NO:207:

(i) SEQUENCE CHARACTERISTICS:
(A) LENGTH: 176 base pairs
(B) TYPE: nucleic acid
(C) STRANDEDNESS: single
(D) TOPOLOGY: linear

(xi) SEQUENCE DESCRIPTION: SEQ ID NO:207:

AGACTGACTC ATGTCCCCTA CCCCACCTTC TGCTGTGCTG CCGTGTTCCT AACAGGTCAC 60

AGACTGGTAC TGGTCAGTGG CCTGGGGGTT GGGGACCTCT ATTATATGGG ATACAAATTT 120

AGGAGTTGGA ATTGACACGA TTTAGTGACT GATGGGATAT GGGTGGTAAA TGGCTA 176

(2) INFORMATION FOR SEQ ID NO:208:

(i) SEQUENCE CHARACTERISTICS:
(A) LENGTH: 196 base pairs
(B) TYPE: nucleic acid
(C) STRANDEDNESS: single
(D) TOPOLOGY: linear

(xi) SEQUENCE DESCRIPTION: SEQ ID NO:208:

AGACTGACTC ATGTCCCCTA TTTAACAGGG TCTCTAGTGC TGTGAAAAAA AAAAATGCTG 60

AACATTGCAT ATAACTTATA TTGTAAGAAA TACTGTACAA TGACTTTATT GCATCTGGGT 120

AGCTGTAAGG CATGAAGGAT GCCAAGAAGT TTAAGGAATA TGGGTGGTAA ATGGCTAGGG 180

GACATGAGTC AGTCTA 196

(2) INFORMATION FOR SEQ ID NO:209:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:209:

GACGCTTGGC CACTTGACAC CTTTTATTTT TTAAGGATTC TTAAGTCATT TANGTNACTT 60

TGTAAGTTTT TCCTGTGCCC CCATAAGAAT GATAGCTTTA AAAATTATGC TGGGGTAGCA 120

AAGAAGATAC TTCTAGCTTT AGAATGTGTA GGTATAGCCA GGATTCTTGT GAGGAGGGGT 180

GATTTAGAGC AAATTTCTTA TTCTCCTTGC CTCATCTGTA ACATGGGGAT AATAATAGAA 240

CTGGCTTGAC AAGGTTGGAA TTAGTATTAC ATGGTAAATA CATGTAAAAT GTTTAGAATG 300

GTGCCAAGTA TCTAGGAAGT ACTTGGGCAT GGGTGGTAAA TGGCT 345

(2) INFORMATION FOR SEQ ID NO:210:

(i) SEQUENCE CHARACTERISTICS:
(A) LENGTH: 178 base pairs
(B) TYPE: nucleic acid
(C) STRANDEDNESS: single
(D) TOPOLOGY: linear

(xi) SEQUENCE DESCRIPTION: SEQ ID NO:210:

GACGCTTGGC CACTTGACAC TAGAGTAGGG TTTGGCCAAC TTTTTCTATA AAGGACCAGA 60

GAGTAAATAT TTCAGGCTTT GTGGGTTGTG CAGTCTCTCT TGCAACTACT CAGCTCTGCC 120

ATTGTAGCAT AGAAATCAGC CATAGACAGG ACAGAAATGA ATGGGTGGTA AATGGCTA 178

(2) INFORMATION FOR SEQ ID NO:211:

(i) SEQUENCE CHARACTERISTICS:
(A) LENGTH: 454 base pairs
(B) TYPE: nucleic acid
(C) STRANDEDNESS: single
(D) TOPOLOGY: linear

(xi) SEQUENCE DESCRIPTION: SEQ ID NO:211:

TGGGCACCTT CAATATCTAT CCAGCGCATC TAAATTCGCT TTTTTCTTGA TTAAAAATTT 60

CACCACTTGC TGTTTTTGCT CATGTATACC AAGTAGCAGT GGTGTGAGGC CATGCTTGTT 120

TTTTGATTCG ATATCAGCAC CGTATAAGAG CAGTGCTTTG GCCATTAATT TATCTTCATT 180

GTAGACAGCA TAGTGTAGAG TGGTATCTCC ATACTCATCT GGAATATTTG GATCAGTGCC 240

ATGTTCCAGC AACATTAACG CACATTCATC TTCCTGGCAT TGTACGGCCT TTGTCAGAGC 300

TGTCCTCTTT TTGTTGTCAA GGACATTAAG TTGACATCGT CTGTCCAGCA CGAGTTTTAC 360

TACTTCTGAA TTCCCATTGG CAGAGGCCAG ATGTAGAGCA GTCCTCTTTT GCTTGTCCCT 420

CTTGTTCACA TCAGTGTCCC TGAGCATAAC GGAA 454

(2) INFORMATION FOR SEQ ID NO:212:

(i) SEQUENCE CHARACTERISTICS:
(A) LENGTH: 337 base pairs
(B) TYPE: nucleic acid
(C) STRANDEDNESS: single
(D) TOPOLOGY: linear

(xi) SEQUENCE DESCRIPTION: SEQ ID NO:212:

TCCGTTATGC CACCCAGAAA ACCTACTGGA GTTACTTATT AACATCAAGG CTGGAACCTA 60

TTTGCCTCAG TCCTATCTGA TTCATGAGCA CATGGTTATT ACTGATCGCA TTGAAAACAT 120

TGATCACCTG GGTTTCTTTA TTTATCGACT GTGTCATGAC AAGGAAACTT ACAAACTGCA 180

ACGCAGAGAA ACTATTAAAG GTATTCAGAA ACGTGAAGCC AGCAATTGTT TCGCAATTCG 240

GCATTTTGAA AACAAATTTG CCGTGGAAAC TTTAATTTGT TCTTGAACAG TCAAGAAAAA 300

CATTATTGAG GAAAATTAAT ATCACAGCAT AACGGAA 337

(2) INFORMATION FOR SEQ ID NO:213:

(i) SEQUENCE CHARACTERISTICS:
(A) LENGTH: 715 base pairs
(B) TYPE: nucleic acid
(C) STRANDEDNESS: single
(D) TOPOLOGY: linear

(xi) SEQUENCE DESCRIPTION: SEQ ID NO:213:

TCGGGTGATG CCTCCTCAGG CATCTTCCAT CCATCTCTTC AAGATTAGCT GTCCCAAATG 60

TTTTTCCTTC TCTTCTTTAC TGATAAATTT GGACTCCTTC TTGACACTGA TGACAGCTTT 120

AGTATCCTTC TTGTCACCTT GCAGACTTTA AACATAAAAA TACTCATTGG TTTTAAAAGG 180

AAAAAAGTAT ACATTAGCAC TATTAAGCTT GGCCTTGAAA CATTTTCTAT CTTTTATTAA 240

ATGTCGGTTA GCTGAACAGA ATTCATTTTA CAATGCAGAG TGAGAAAAGA AGGGAGCTAT 300

ATGCATTTGA GAATGCAAGC ATTGTCAAAT AAACATTTTA AATGCTTTCT TAAAGTGAGC 360

ACATACAGAA ATACATTAAG ATATTAGAAA GTGTTTTTGC TTGTGTACTA CTAATTAGGG 420

AAGCACCTTG TATAGTTCCT CTTCTAAAAT TGAAGTAGAT TTTAAAAACC CATGTAATTT 480

AATTGAGCTC TCAGTTCAGA TTTTAGGAGA ATTTTAACAG GGATTTGGTT TTGTCTAAAT 540

TTTGTCAATT TNTTTAGTTA ATCTGTATAA TTTTATAAAT GTCAAACTGT ATTTAGTCCG 600

TTTTCATGCT GCTATGAAAG AAATACCCAN GACAGGGTTA TTTATAAANG GAAAGANGTT 660

AATTTGACTC CCAGTTCACA GGCCTGAGGA NGNATCNCCC GAAATCCTTA TTGCG 715

(2) INFORMATION FOR SEQ ID NO:214:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:214:

GGTAANGNGC ATACNTCGGT GCTCCGGCCG CCGGAGTCGG GGGATTCGGG TGATGCCTCC 60

TCAGGCCCAC TTGGGCCTGC TTTTCCCAAA TGGCAGCTCC TCTGGACATG CCATTCCTTC 120

TCCCACCTGC CTGATTCTTC ATATGTTGGG TGTCCCTGTT TTTCTGGTGC TATTTCCTGA 180

CTGCTGTTCA GCTGCCACTG TCCTGCAAAG CCTGCCTTTT TAAATGCCTC ACCATTCCTT 240

CATTTGTTTC TTAAATATGG GAAGTGAAAG TGCCACCTGA GGCCGGGCAC AGTGGCTCAC 300

GCCTGTAATC CCAGCACTTT GGGAGCCTGA GGAGGCATCA CCCGA 345

(2) INFORMATION FOR SEQ ID NO:215:

(i) SEQUENCE CHARACTERISTICS:
(A) LENGTH: 429 base pairs
(B) TYPE: nucleic acid
(C) STRANDEDNESS: single
(D) TOPOLOGY: linear

(xi) SEQUENCE DESCRIPTION: SEQ ID NO:215:

GGTGATGCCT CCTCAGGCGA AGCTCAGGGA GGACAGAAAC CTCCCGTGGA GCAGAAGGGC 60

AAAAGCTCGC TTGATCTTGA TTTTCAGTAC GAATACAGAC CGTGAAAGCG GGGCCTCACG 120

ATCCTTCTGA CCTTTTGGGT TTTAAGCAGG AGGTGTCAGA AAAGTTACCA CAGGGATAAC 180

TGGCTTGTGG CGGCCAAGCG TTCATAGCGA CGTCGCTTTT TGATCCTTCG ATGTCGGCTC 240

TTCCTATCAT TGTGAAGCAG AATTCACCAA GCGTTGGATT GTTCACCCAC TAATAGGGAA 300

CGTGAGCTGG GTTTAGACCG TCGTGAGACA GGTTAGTTTT ACCCTACTGA TGATGTGTKG 360

TTGCCATGGT AATCCTGCTC AGTACGAGAG GAACCGCAGG TTCASACATT TGGTGTATGT 420

GCTTGCCTT 429

(2) INFORMATION FOR SEQ ID NO:216:

(i) SEQUENCE CHARACTERISTICS:
(A) LENGTH: 593 base pairs
(B) TYPE: nucleic acid
(C) STRANDEDNESS: single
(D) TOPOLOGY: linear

(xi) SEQUENCE DESCRIPTION: SEQ ID NO:216:

TGACACCTAT GTCCNGCATC TGTTCACAGT TTCCACAAAT AGCCAGCCTT TGGCCACCTC 60

TCTGTCCTGA GGTATACAAG TATATCAGGA GGTGTATACC TTCTCTTCTC TTCCCCACCA 120

AAGAGAACAT GCAGGCTCTG GAAGCTGTCT TAGGAGCCTT TGGGCTCAGA ATTTCAGAGT 180

CTTGGGTACC TTGGATGTGG TCTGGAAGGA GAAACATTGG CTCTGGATAA GGAGTACAGC 240

CGGAGGAGGG TCACAGAGCC CTCAGCTCAA GCCCCTGTGC CTTAGTCTAA AAGCAGCTTT 300

GGATGAGGAA GCAGGTTAAG TAACATACGT AAGCGTACAC AGGTAGAAAG TGCTGGGAGT 360

CAGAATTGCA CAGTGTGTAG GAGTAGTACC TCAATCAATG AGGGCAAATC AACTGAAAGA 420

AGAAGACCNA TTAATGAATT GCTTANGGGG AAGGATCAAG GCTATCATGG AGATCTTTCT 480

AGGAAGATTA TTGTTTANAA TTATGAAAGG ANTAGGGCAG GGACAGGGCC AGAAGTANAA 540

GANAACATTG CCTATANCCC TTGTCTTGCA CCCAGATGCT GGACAAGGTG TCA 593

(2) INFORMATION FOR SEQ ID NO:217:

(i) SEQUENCE CHARACTERISTICS:
(A) LENGTH: 335 base pairs
(B) TYPE: nucleic acid
(C) STRANDEDNESS: single
(D) TOPOLOGY: linear

(xi) SEQUENCE DESCRIPTION: SEQ ID NO:217:

TGACACCTTG TCCAGCATCT GACGTGAAGA TGAGCAGCTC AGAGGAGGTG TCCTGGATTT 60

CCTGGTTCTG TGGGCTCCGT GGCAATGAAT TCTTCTGTGA AGTGGATGAA GACTACATCC 120

AGGACAAATT TAATCTTACT GGACTCAATG AGCAGGTCCC TCACTATCGA CAAGCTCTAG 180

ACATGATCTT GGACCTGGAG CCTGATGAAG AACTGGAAGA CAACCCCAAC CAGAGTGACC 240

TGATTGAGCA GGCAGCCGAG ATGCTTTATG GATTGATCCA CGCCCGCTAC ATCCTTACCA 300

ACCGTGGCAT CGCCCAGATG CTGGACAAGG TGTCA 335

(2) INFORMATION FOR SEQ ID NO:218:

(i) SEQUENCE CHARACTERISTICS:
(A) LENGTH: 248 base pairs
(B) TYPE: nucleic acid

(C) STRANDEDNESS: single
(D) TOPOLOGY: linear

(xi) SEQUENCE DESCRIPTION: SEQ ID NO:218:

TACGTACTGG TCTTGAAGGT CTTAGGTAGA GAAAAAATGT GAATATTTAA TCAAAGACTA 60

TGTATGAAAT GGGACTGTAA GTACAGAGGG AAGGGTGGCC CTTATCGCCA GAAGTTGGTA 120

GATGCGTCCC CGTCATGAAA TGTTGTGTCA CTGCCCGACA TTTGCCGAAT TACTGAAATT 180

CCGTAGAATT AGTGCAAATT CTAACGTTGT TCATCTAAGA TTATGGTTCC ATGTTTCTAG 240

TACTTTTA 248

(2) INFORMATION FOR SEQ ID NO:219:

(i) SEQUENCE CHARACTERISTICS:
(A) LENGTH: 530 base pairs
(B) TYPE: nucleic acid
(C) STRANDEDNESS: single
(D) TOPOLOGY: linear

(xi) SEQUENCE DESCRIPTION: SEQ ID NO:219:

TGACGCTTGG CCACTTGACA CAAGTAGGGG ATAAGGACAA AGACCCATNA GGTGGCCTGT 60

CAGCCTTTTG TTACTGTTGC TTCCCTGTCA CCACGGCCCC CTCTGTAGGG GTGTGCTGTG 120

CTCTGTGGAC ATTGGTGCAT TTTCACACAT ACCATTCTCT TTCTGCTTCA CAGCAGTCCT 180

GAGGCGGGAG CACACAGGAC TACCTTGTCA GATGANGATA ATGATGTCTG GCCAACTCAC 240

CCCCCAACCT TCTCACTAGT TATANGAAGA GCCANGCCTA NAACCTTCTA TCCTGNCCCC 300

TTGCCCTATG ACCTCATCCC TGTTCCATGC CCTATTCTGA TTTCTGGTGA ACTTTGGAGC 360

AGCCTGGTTT NTCCTCCTCA CTCCAGCCTC TCTCCATACC ATGGTANGGG GGTGCTGTTC 420

CACNCAAANG GTCAGGTGTG TCTGGGGAAT CCTNANANCT GCCNGGAGTT TCCNANGCAT 480

TCTTAAAAAC CTTCTTGCCT AATCANATNG TGTCCAGTGG CCAACCNTCN 530

(2) INFORMATION FOR SEQ ID NO:220:

(i) SEQUENCE CHARACTERISTICS:
(A) LENGTH: 531 base pairs
(B) TYPE: nucleic acid
(C) STRANDEDNESS: single
(D) TOPOLOGY: linear

(xi) SEQUENCE DESCRIPTION: SEQ ID NO:220:

TGACGCTTGG CCACTTGACA CTAAATAGCA TCTTCTAAAG GCCTGATTCA GAGTTGTGGA 60

AAATTCTCCC AGTGTCAGGG ATTGTCAGGA ACAGGGCTGC TCCTGTGCTC ACTTTACCTG 120

CTGTGTTTCT GCTGGAAAAG GAGGGAAGAG GAATGGCTGA TTTTTACCTA ATGTCTCCCA 180

GTTTTTCATA TTCTTCTTGG ATCCTCTTCT CTGACAACTG TTCCCTTTTG GTCTTCTTCT 240

TCTTGCTCAG AGAGCAGGTC TCTTTAAAAC TGAGAAGGGA GAATGAGCAA ATGATTAAAG 300

AAAACACACT TCTGAGGCCC AGAGATCAAA TATTAGGTAA ATACTAAACC GCTTGCCTGC 360

TGTGGTCACT TTTCTCCTCT TTCACATGCT CTATCCCTCT ATCCCCCACC TATTCATATG 420

GCTTTTATCT GCCAAGTTAT CCGGCCTCTC ATCAACCTTC TCCCCTAGCC TACTGGGGGA 480

TATCCATCTG GGTCTGTCTC TGGTGTATTG GTGTCAAGTG GCCAAGCGTC A 531

(2) INFORMATION FOR SEQ ID NO:221:

(i) SEQUENCE CHARACTERISTICS:
(A) LENGTH: 530 base pairs
(B) TYPE: nucleic acid
(C) STRANDEDNESS: single
(D) TOPOLOGY: linear

(xi) SEQUENCE DESCRIPTION: SEQ ID NO:221:

ATTGACGCTT GGCCACTTGA CACCCGCCTG CCTGCAATAC TGGGGCAAGG GCCTTCACTG 60

CTTTCCTGCC ACCAGCTGCC ACTGCACACA GAGATCAGAA ATGCTACCAA CCAAGACTGT 120

TGGTCCTCAG CCTCTCTGAG GAGAAAGAGC AGAAGCCTGG AAGTCAGAAG AGAAGCTAGA 180

TCGGCTACGG CCTTGGCAGC CAGCTTCCCC ACCTGTGGCA ATAAAGTCGT GCATGGCTTA 240

ACAATGGGGG CACCTCCTGA GAAACACATT GTTAGGCAAT TCGGCGTGTG TTCATCAGAG 300

CATATTTACA CAAACCTCGA TAGTGCAGCC TACTATCCAC TATTGCTCCT ACGCTGCAAA 360

CCTGAACAGC ATGGGACTGT ACTGAATACT GGAAGCAGCT GGTGATGGTA CTTATTTGTG 420

TATCTAAACA CAGAGAAGGT ACAGTAAGAA TATGGTATCA TAAACTTACA GGGACCGCCA 480

TCCTATATGC AGTCTGTTGT GACCAAAATG TGTCAAGTGG CCAAGCGTCA 530

(2) INFORMATION FOR SEQ ID NO:222:

(i) SEQUENCE CHARACTERISTICS:
(A) LENGTH: 578 base pairs
(B) TYPE: nucleic acid

(C) STRANDEDNESS: single
(D) TOPOLOGY: linear

(xi) SEQUENCE DESCRIPTION: SEQ ID NO:222:

TGTATCGACG TAGTGGTCTC CGGGCTACTA GGCCGTTGTG TGCTGGTAGT ACCTGGTTCA 60

CTGAAAGGCG CATCTCCCTC CCCGCGTCGC CCTGAAGCAG GGGGAGGACT TCGCCCAGCC 120

AAGGCAGTTG TATGAGTTTT AGCTGCGGCA CTTCGAGACC TCTGAGCCCA CCTCCTTCAG 180

GAGCCTTCCC CGATTAAGGA AGCCAGGGTA AGGATTCCTT CCTCCCCCAG ACACCACGAA 240

CAAACCACCA CCCCCCCTAT TCTGGCAGCC CATATACATC AGAACGAAAC AAAAATAACA 300

AATAAACNAA AACCAAAAAA AAAAGAGAAG GGGAAATGTA TATGTCTGTC CATCCTGTTG 360

CTTTAGCCTG TCAGCTCCTA NAGGGCAGGG ACCGTGTCTT CCGAATGGTC TGTGCAGCGC 420

CGACTGCGGG AAGTATCGGA GGAGGAAGCA GAGTCAGCAG AAGTTGAACG GTGGGCCCGG 480

CGGCTCTTGG GGGCTGGTGT TGTACTTCGA GACCGCTTTC GCTTTTTGTC TTAGATTTAC 540

GTTTGCTCTT TGGAGTGGGA NACCACTACN TCNATACA 578

(2) INFORMATION FOR SEQ ID NO:223:

(i) SEQUENCE CHARACTERISTICS:
(A) LENGTH: 578 base pairs
(B) TYPE: nucleic acid
(C) STRANDEDNESS: single
(D) TOPOLOGY: linear

(xi) SEQUENCE DESCRIPTION: SEQ ID NO:223:

TGTATCGACG TAGTGGTCTC CTCTTGCAAA GGACTGGCTG GTGAATGGTT TCCCTGAATT 60

ATGGACTTAC CCTAAACATA TCTTATCATC ATTACCAGTT GCAAAATATT AGAATGTGTT 120

GTCACTGTTT CATTTGATTC CTAGAAGGTT AGTCTTAGAT ATGTTACTTT AACCTGTATG 180

CTGTAGTGCT TTGAATGCAT TTTTTGTTTG CATTTTTGTT TGCCCAACCT GTCAATTATA 240

GCTGCTTAGG TCTGGACTGT CCTGGATAAA GCTGTTAAAA TATTCACCAG TCCAGCCATC 300

TTACAAGCTA ATTAAGTCAA CTAAATGCTT CCTTGTTTTG CCAGACTTGT TATGTCAATC 360

CTCAATTTCT GGGTTCATTT TGGGTGCCCT AAATCTTAGG GTGTGACTTT CTTAGCATCC 420

TGTAACATCC ATTCCCAAGC AAGCACAACT TCACATAATA CTTTCCAGAA GTTCATTGCT 480

GAAGCCTTTC CTTCACCCAG CGGAGCAACT TGATTTTCTA CAACTTCCCT CATCAGAGCC 540

ACAAGAGTAT GGGATATGGA GACCACTACG TCGATACA 578

(2) INFORMATION FOR SEQ ID NO:224:

(i) SEQUENCE CHARACTERISTICS:
(A) LENGTH: 345 base pairs
(B) TYPE: nucleic acid
(C) STRANDEDNESS: single
(D) TOPOLOGY: linear

(xi) SEQUENCE DESCRIPTION: SEQ ID NO:224:

TGTATCGACG TANTGGTCTC CCAAGGTGCT GGGATTGCAG GCATGAGCCA CCACTCCCAG 60

GTGGATCTTT TTCTTTATAC TTACTTCATT AGGTTTCTGT TATTCAAGAA GTGTAGTGGT 120

AAAAGTCTTT TCAATCTACA TGGTTAAATA ATGATAGCCT GGGAAATAAA TAGAAATTTT 180

TTCTTTCATC TTTAGGTTGA ATAAAGAAAC AGAAAAAATA GAACATACTG AAAATAATCT 240

AAGTTCCAAC CATAGAAGAA CTGCAGAAGA AATGAAGAAA GTGATGATGA TTTAGATTTT 300

GATATTGATT TAGAAGACAC AGGAGGAGAC CACTACGTCG ATACA 345

(2) INFORMATION FOR SEQ ID NO:225:

(i) SEQUENCE CHARACTERISTICS:
(A) LENGTH: 347 base pairs
(B) TYPE: nucleic acid
(C) STRANDEDNESS: single
(D) TOPOLOGY: linear

(xi) SEQUENCE DESCRIPTION: SEQ ID NO:225:

TGTATCGACG TAGTGGTCTC CAAACTGAGG TATGTGTGCC ACTAGCACAC AAAGCCTTCC 60

AACAGGGACG CAGGCACAGG CAGTTTAAAG GGAATCTGTT TCTAAATTAA TTTCCACCTT 120

CTCTAAGTAT TCTTTCCTAA AACTGATCAA GGTGTGAAGC CTGTGCTCTT TCCCAACTCC 180

CCTTTGACAA CAGCCTTCAA CTAACACAAG AAAAGGCATG TCTGACACTC TTCCTGAGTC 240

TGACTCTGAT ACGTTGTTCT GATGTCTAAA GAGCTCCAGA ACACCAAAGG GACAATTCAG 300

AATGCTGGTG TATAACAGAC TCCAATGGAG ACCACTACGT CGATACA 347

(2) INFORMATION FOR SEQ ID NO:226:

(i) SEQUENCE CHARACTERISTICS:
(A) LENGTH: 281 base pairs
(B) TYPE: nucleic acid
(C) STRANDEDNESS: single
(D) TOPOLOGY: linear

(xi) SEQUENCE DESCRIPTION: SEQ ID NO:226:

AGGNGNGGGA NTGTATCGAC GTAGTGGTCT CCCAACAGTC TGTCATTCAG TCTGCAGGTG 60

TCAGTGTTTT GGACAATGAG GCACCATTGT CACTTATTGA CTCCTCAGCT CTAAATGCTG 120

AAATTAAATC TTGTCATGAC AAGTCTGGAA TTCCTGATGA GGTTTTACAA AGTATTTTGG 180

ATCAATACTC CAACAAATCA GAAAGCCAGA AAGAGGATCC TTTCAATATT GCAGAACCAC 240

GAGTGGATTT ACACACCTCA GGAGACCACT ACGTCGATAC A 281

(2) INFORMATION FOR SEQ ID NO:227:

(i) SEQUENCE CHARACTERISTICS:

(A) LENGTH: 3646 base pairs

(B) TYPE: nucleic acid

(C) STRANDEDNESS: single

(D) TOPOLOGY: linear

(xi) SEQUENCE DESCRIPTION: SEQ ID NO:227:

GGGAAACACT TCCTCCCAGC CTTGTAAGGG TTGGAGCCCT CTCCAGTATA TGCTGCAGAA 60

TTTTTCTCTC GGTTTCTCAG AGGATTATGG AGTCCGCCTT AAAAAAGGCA AGCTCTGGAC 120

ACTCTGCAAA GTAGAATGGC CAAAGTTTGG AGTTGAGTGG CCCCTTGAAG GGTCACTGAA 180

CCTCACAATT GTTCAAGCTG TGTGGCGGGT TGTTACTGAA ACTCCCGGCC TCCCTGATCA 240

GTTTCCCTAC ATTGATCAAT GGCTGAGTTT GGTCAGGAGC ACCCCTTCCG TGGCTCCACT 300

CATGCACCAT TCATAATTTT ACCTCCAAGG TCCTCCTGAG CCAGACCGTG TTTTCGCCTC 360

GACCCTCAGC CGGTTCGGCT CGCCCTGTAC TGCCTCTCTC TGAAGAAGAG GAGAGTCTCC 420

CTCACCCAGT CCCACCGCCT TAAAACCAGC CTACTCCCTT AGGGTCATCC CATGTCTCCT 480

CGGCTATGTC CCCTGTAGGC TCATCACCCA TTGCCTCTTG GTTGCAACCG TGGTGGGAGG 540

AAGTAGCCCC TCTACTACCA CTGAGAGAGG CACAAGTCCC TCTGGGTGAT GAGTGCTCCA 600

CCCCCTTCCT GGTTTATGTC CCTTCTTTCT ACTTCTGACT TGTATAATTG GAAAACCCAT 660

AATCCTCCCT TCTCTGAAAA GCCCCAGGCT TTGACCTCAC TGATGGAGTC TGTACTCTGG 720

ACACATTGGC CCACCTGGGA TGACTGTCAA CAGCTCCTTT TGACCCTTTT CACCTCTGAA 780

GAGAGGGAAA GTATCCAAAG AGAGGCCAAA AAGTACAACC TCACATCAAC CAATAGGCCG 840

GAGGAGGAAG CTAGAGGAAT AGTGATTAGA GACCCAATTG GGACCTAATT GGGACCCAAA 900

TTTCTCAAGT GGAGGGAGAA CTTTTGACGA TTTCCACCGG TATCTCCTCG TGGGTATTCA 960

GGGAGCTGCT CAGAAACCTA TAAACTTGTC TAAGGCGACT GAAGTCGTCC AGGGGCATGA 1020

TGAGTCACCA GGAGTGTTTT TAGAGCACCT CCAGGAGGCT TATCAGATTT ACACCCCTTT 1080

TGACCTGGCA GCCCCCGAAA ATAGCCATGC TCTTAATTTG GCATTTGTGG CTCAGGCAGC 1140

CCCAGATAGT AAAAGGAAAC TCCAAAAACT AGAGGGATTT TGCTGGAATG AATACCAGTC 1200

AGCTTTTAGA GATAGCCTAA AAGGTTTTTG ACAGTCAAGA GGTTGAAAAA CAAAAACAAG 1260

CAGCTCAGGC AGCTGAAAAA AGCCACTGAT AAAGCATCCT GGAGTATCAG AGTTTACTGT 1320

TAGATCAGCC TCATTTGACT TCCCCTCCCA CATGGTGTTT AAATCCAGCT ACACTACTTC 1380

CTGACTCAAA CTCCACTATT CCTGTTCATG ACTGTCAGGA ACTGTTGGAA ACTACTGAAA 1440

CTGGCCGACC TGATCTTCAA AATGTGCCCC TAGGAAAGGT GGATGCCACC ATGTTCACAG 1500

ACAGTAGCAG CTTCCTCGAG AAGGGACTAC GAAAGGCCGG TGCAGCTGTT ACCATGGAGA 1560

CAGATGTGTT GTGGGCTCAG GCTTTACCAG CAAACACCTC AGCACAAAAG GCTGAATTGA 1620

TCGCCCTCAC TCAGGCTCTC CGATGGGGTA AGGATATTAA CGTTAACACT GACAGCAGGT 1680

ACGCCTTTGC TACTGTGCAT GTACGTGGAG CCATCTACCA GGAGCGTGGG CTACTCACCT 1740

CAGCAGGTGG CTGTAATCCA CTGTAAAGGA CATCAAAAGG AAAACACGGC TGTTGCCCGT 1800

GGTAACCAGA AAGCTGATTC AGCAGCTCAA GATGCAGTGT GACTTTCAGT CACGCCTCTA 1860

AACTTGCTGC CCACAGTCTC CTTTCCACAG CCAGATCTGC CTGACAATCC CGCATACTCA 1920

ACAGAAGAAG AAAACTGGCC TCAGAACTCA GAGCCAATAA AAATCAGGAA GGTTGGTGGA 1980

TTCTTCCTGA CTCTAGAATC TTCATACCCC GAACTCTTGG GAAAACTTTA ATCAGTCACC 2040

TACAGTCTAC CACCCATTTA GGAGGAGCAA AGCTACCTCA GCTCCTCCGG AGCCGTTTTA 2100

AGATCCCCCA TCTTCAAAGC CTAACAGATC AAGCAGCTCT CCGGTGCACA ACCTGCGCCC 2160

AGGTAAATGC CAAAAAAGGT CCTAAACCCA GCCCAGGCCA CCGTCTCCAA GAAAACTCAC 2220

CAGGAGAAAA GTGGGAAATT GACTTTACAG AAGTAAAACC ACACCGGGCT GGGTACAAAT 2280

ACCTTCTAGT ACTGGTAGAC ACCTTCTCTG GATGGACTGA AGCATTTGCT ACCAAAAACG 2340

AAACTGTCAA TATGGTAGTT AAGTTTTTAC TCAATGAAAT CATCCCTCGA CATGGGCTGC 2400

CTGTTTGCCA TAGGGTCTGA TAATGGACCG GCCTTCGCCT TGTCTATAGT TTAGTCAGTC 2460

AGTAAGGCGT TAAACATTCA ATGGAAGCTC CATTGTGCCT ATCGACCCCA GAGCTCTGGG 2520

CAAGTAGAAC GCATGAACTG CACCCTAAAA AACACTCTTA CAAAATTAAT CTTAGAAACC 2580

GGTGTAAATT GTGTAAGTCT CCTTCCTTTA GCCCTACTTA GAGTAAGGTG CACCCCTTAC 2640

TGGGCTGGGT TCTTACCTTT TGAAATCATG TATGGGAGGG TGCTGCCTAT CTTGCCTAAG 2700

CTAAGAGATG CCCAATTGGC AAAAATATCA CAAACTAATT TATTACAGTA CCTACAGTCT 2760

CCCCAACAGG TACAAGATAT CATCCTGCCA CTTGTTCGAG GAACCCATCC CAATCCAATT 2820

CCTGAACAGA CAGGGCCCTG CCATTCATTC CCGCCAGGTG ACCTGTTGTT TGTTAAAAAG 2880

TTCCAGAGAG AAGGACTCCC TCCTGCTTGG AAGAGACCTC ACACCGTCAT CACGATGCCA 2940

ACGGCTCTGA AGGTGGATGG CATTCCTGCG TGGATTCATC ACTCCCGCAT CAAAAAGGCC 3000

AACAGAGCCC AACTAGAAAC ATGGGTCCCC AGGGCTGGGT CAGGCCCCTT AAAACTGCAC 3060

CTAAGTTGGG TGAAGCCATT AGATTAATTC TTTTTCTTAA TTTTGTAAAA CAATGCATAG 3120

CTTCTGTCAA ACTTATGTAT CTTAAGACTC AATATAACCC CCTTGTTATA ACTGAGGAAT 3180

CAATGATTTG ATTCCCCCAA AAACACAAGT GGGGAATGTA GTGTCCAACC TGGTTTTTAC 3240

TAACCCTGTT TTTAGACTCT CCCTTTCCTT TAATCACTCA GCTTGTTTCC ACCTGAATTG 3300

ACTCTCCCTT AGCTAAGAGC GCCAGATGGA CTCCATCTTG GCTCTTTCAC TGGCAGCCGC 3360

TTCCTCAAGG ACTTAACTTG TGCAAGCTGA CTCCCAGCAC ATCCAAGAAT GCAATTAACT 3420

GATAAGATAC TGTGGCAAGC TATATCCGCA GTTCCCAGGA ATTCGTCCAA TTGATCACAG 3480

CCCCTCTACC CTTCAGCAAC CACCACCCTG ATCAGTCAGC AGCCATCAGC ACCGAGGCAA 3540

GGCCCTCCAC CAGCAAAAAG ATTCTGACTC ACTGAAGACT TGGATGATCA TTAGTATTTT 3600

TAGCAGTAAA GTTTTTTTTT CTTTTTCTTT CTTTTTTTCT CGTGCC 3646